# EUROPEAN PATENT APPLICATION

(11) **EP 3 786 180 A1**
(43) Date of publication of application: **03.03.2021**
(21) Application number: 19306033.2
(22) Date of filing: 27.08.2019
(51) Int. Cl.: C07K 16/10, G01N 33/53, A61P 31/18

(54) **ANTIBODIES AND THE USES THEREOF**

(71) Applicant: Diaccurate, 75008 Paris (FR)
(72) Inventor: Pothlichet, Julien, 92310 Sevres (FR); Theze, Jacques, 75007 Paris (FR)
(74) Representative: Cabinet Becker et Associés

(57) **Abstract**

The present invention relates to antibodies and their use. The invention particularly relates to antibodies against a particular domain of gp41, pharmaceutical compositions or kits or any device comprising the same, as well as the uses thereof. The invention is particularly useful for treating (e.g., curing or preventing), detecting or monitoring AIDS in a subject, either, alone or in combination with other treatments/agents.

## Description

The present invention relates to antibodies and their use. The invention particularly relates to antibodies against a particular domain of gp41, pharmaceutical compositions or kits or any device comprising the same, as well as the uses thereof. The invention is particularly useful for treating (e.g., curing or preventing), detecting or monitoring AIDS in a subject, either, alone or in combination with other treatments/agents.

### Introduction and background

It has been documented by the inventors that sPLA2-GIB is involved in the inactivation of CD4 T cells in HIV infected patients (see WO2015/097140). It was proposed and documented by the inventor that sPLA2-GIB modulators are effective for treating diseases in mammal, e.g., disorders associated with an immune deficiency.
Continuing their research, applicant found that the effect of sPLA2-GIB can be mediated and/or amplified by cofactors present in diseased subjects, and that such cofactors act through a gClq receptor at the surface of T cells (see PCT/EP2019/054686). It was proposed and documented by the inventors that modulators of such cofactors are effective for treating diseases in mammal, e.g., disorders associated with an immune deficiency. In relation to HIV viremic patients, it was more specifically disclosed that gp41 acts as a cofactor that increases sPLA2-GIB inhibitory effect on human CD4 T cells. It was also shown that gp41-derived PEP3 peptide alone also increases sPLA2-GIB activity, suggesting that PEP3 gp41 sequence is a key domain for gp41 cofactor activity.

### Summary of the invention

The present invention now discloses novel anti-gp41 antibodies that are specific of the PEP3 sequence. The present invention relates to such antibodies, variants thereof, their manufacture, compositions or devices containing the same, and the uses thereof e.g., for detecting, monitoring or treating HIV disorders.

More specifically, the invention provides antibodies and variants thereof selected for their binding on gp41 protein, more specifically on the PEP3 domain of gp41. The invention discloses the sequence of said antibodies and their functional properties, such as e.g., their specificity for gp41 relatively to gp120, their ability to reduce the inhibitory activity of HIV viremic patient plasma on CD4 T cells, and/or their ability to quantify gp41 products in the plasma of HIV viremic patients.

Altogether these antibodies can thus be used to follow gp41 concentration in the plasma of HIV patients or any other sample and thus to predict the severity of HIV disease or the outcome of HIV viremic patients.

### Legend to the figures

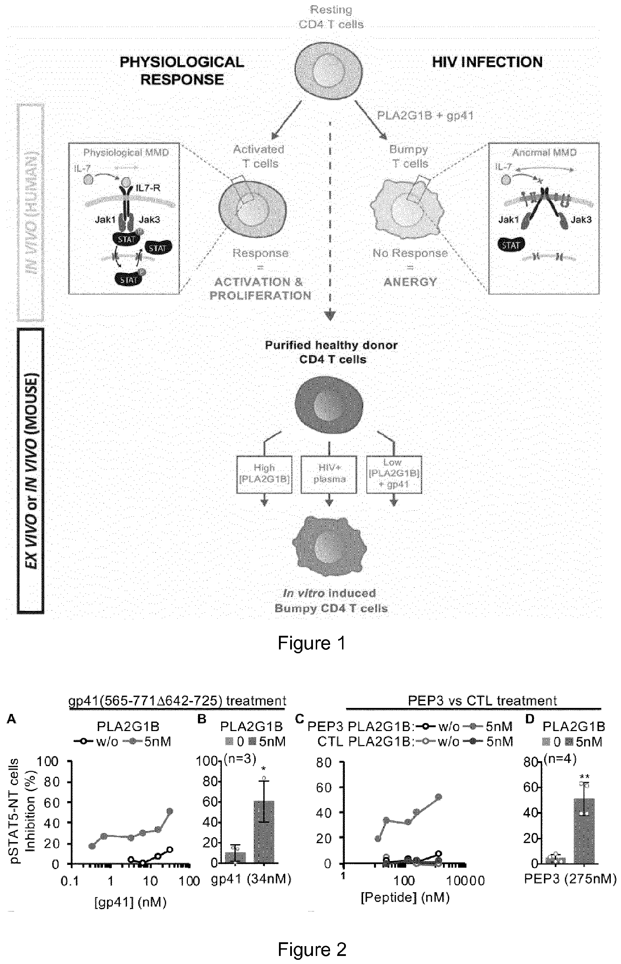

Figure 1. Model of regulation by gp41 of sPLA2-GIB activity on CD4 T cells. This model summarizes the mode of action of PLA2-GIB on CD4 T cells. In vivo in human, physiological membrane microdomains (pMMDs) of small size (average diameter 100nm) are induced by gamma c cytokines (e.g. IL-7, IL-2 and IL-4) and T-cell receptor (TCR) activation on CD4 T cells and are necessary for their signaling pathways. In the plasma of HIV viremic patients, PLA2-GIB synergizes with gp41 to induce abnormal membrane microdomains (aMMDs) of big size (diameter >200nm) trapping and inactivating physiological receptors. In vivo in mice or in vitro on mouse and human purified CD4 T cells, high concentration of PLA2-GIB alone can inhibit CD4 T cells response to gamma c cytokines and TCR stimulation. The HIV viremic plasma alone and low concentrations of PLA2-GIB with gp41, as present in viremic plasma, reduce the efficiency of gamma c cytokines signaling pathways. This model explain how PLA2-GIB participates to the anergy and lymphopenia observed in HIV-infected patients.
**Figure 2****. Synergy between PLA2GIB activity and gp41. (A)** Recombinant gp41 protein causes sensitivity of CD4 T cells to PLA2-GIB inhibitory activity on phosphoSTAT5 nuclear translocation (pSTAT5 NT) in response to IL-7. A doseeffect of recombinant gp41 protein on PLA2-GIB activity on pSTAT5 NT response to IL-7 is shown. Purified CD4 T cells from healthy donor were pretreated for 15 min with several amounts of gp41 or buffer (PBS/1%BSA), incubated for 30 min with 5 nM of PLA2-GIB or not (w/o) and stimulated with IL-7 for 15 min. pSTAT5 NT was analyzed by confocal microscopy. One representative experiment among three is presented. **(B)** Summary of experiments on 3 independent healthy donors of CD4 T cells treated with 34 nM of gp41 for 15 min, 30 min with 5 nM of PLA2-GIB or not (w/o) and stimulated with IL-7 for 15 min. **A** and **B**, results presented the percentage of inhibition of pSTAT5 NT normalized with the pSTAT5 NT in response to IL-7 in buffer. **(B)** Results are shown as the mean ± SD of the percentage of pSTAT5 NT cells inhibition on CD4 T cells from 3 donors. Statistical analysis of the difference of inhibition with gp41 and 5 nM of PLA2-GIB relatively to gp41 alone without PLA2-GIB by two tailed unpaired t-test, * means *p*<0.05. **(C)** Doseeffect of PEP3 and CTL peptides on PLA2-GIB activity on the percentage of inhibition of pSTAT5 NT response to IL-7. Purified CD4 T cells from healthy donor were pretreated for 15 min with several amounts of PEP3 or CTL peptides or buffer (PBS/1%BSA), incubated for 30 min with 5 nM of PLA2-GIB or not (w/o) and stimulated with IL-7 for 15 min. pSTAT5 NT was analyzed by confocal microscopy. One representative experiment among two is presented. **(D)** Summary of experiments on 4 independent healthy donors of CD4 T cells treated with 275 nM of PEP3 for 45 min with 5 nM of PLA2-GIB or not (w/o) and stimulated with IL-7 for 15 min. **C** and **D,** results are presented as the percentage of inhibition of pSTAT5 NT normalized with the pSTAT5 NT in response to IL-7 in buffer. **(D)** Results are shown as the mean ± SD of the percentage of pSTAT5 NT cells inhibition on CD4 T cells from 4 healthy donors. Statistical analysis of the difference of inhibition with PEP3 and 5 nM of PLA2-GIB relatively to PEP3 alone without PLA2-GIB with two tailed unpaired t-test, ** means *p*<0.01.

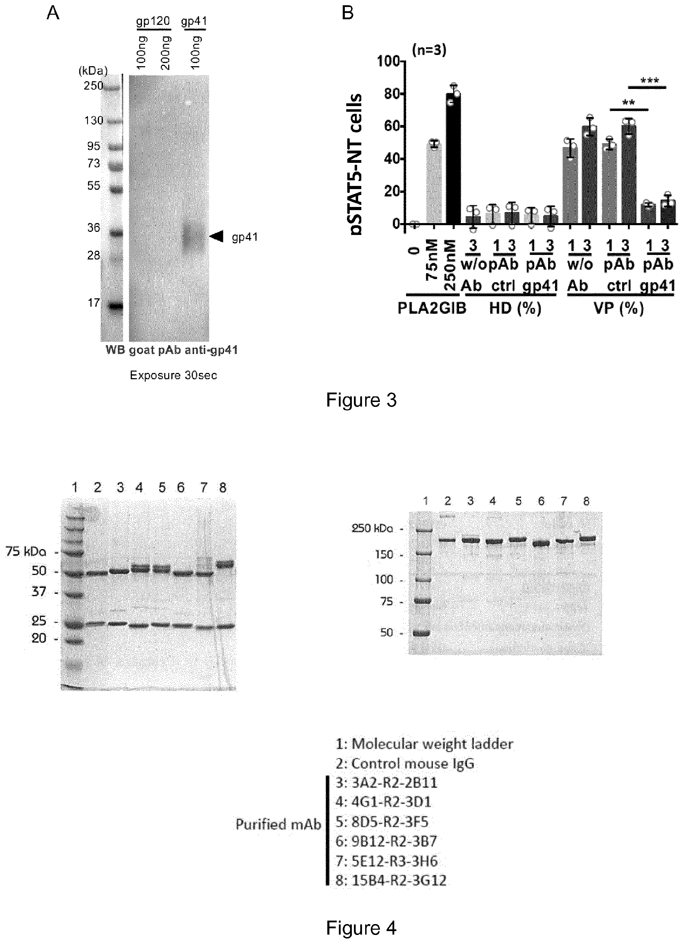

**Figure 3****. Immunodepletion of viremic patient plasma with anti-gp41 antibody abrogates the inhibitory activity of PLA2-GIB on pSTAT5 NT in CD4 T cells (i.e., restores resistance of CD4 T cells to inactivation by PLA2-GIB). (A)** The goat anti-gp41 pAb binds to gp41 but not gp120. Immunoblot with anti-gp41 pAb: 100 and 200 ng of D117 III gp120 or 100 ng of MN gp41 recombinant proteins were separated on 4%-20% Tris-Bis SDS-PAGE in reducing conditions and transferred on PVDF membranes. Binding of anti-gp41 pAb (5 µg/ml) was revealed with donkey anti-goat IgG-HRP (1:10000). **(B)** Purified CD4 T cells from 3 independent healthy donors were treated in 3 independent experiments for 30 min with PLA2-GIB alone, as positive control of sensitivity to PLA2-GIB, healthy donor (HD) plasma or viremic patient (VP) plasma, previously depleted with anti-gp41 polyclonal (pAb anti-gp41), control polyclonal antibody (pAb ctrl) or treated without antibody (only) and stimulated with IL-7 for 15 min. Results are presented as the percentage of inhibition of pSTAT5 NT normalized with the pSTAT5 NT in response to IL-7 in buffer. ** means *p*<0.01 and *** means that *p*<0.001 with two tailed unpaired t-test for the difference of pSTAT5 NT inhibition with pAb ctrl relatively to pAb anti-gp41 treated viremic plasma.
**Figure 4****. Characterization of anti-PEP3 mAb on SDS-PAGE polyacrylamide gel stained by Coomassie blue.** The 6 anti-PEP3 mAbs, 3A2 (lane 3), 4G1 (lane 4), 8D5 (lane 5), 9B12 (lane 6), 5E12 (lane 7) and 15B4 (lane 8) have an expected pattern on SDS-PAGE polyacrylamide gel after Coomassie blue staining. Molecular weight protein ladder (lane 1), control mouse IgG (lane 2) and the 6 purified anti-PEP3 mAb selected were separated on SDS-PAGE polyacrylamide gel in **(A)** reducing (13.5% polyacrylamide) and **(B)** non-reducing (7.5% polyacrylamide) conditions.

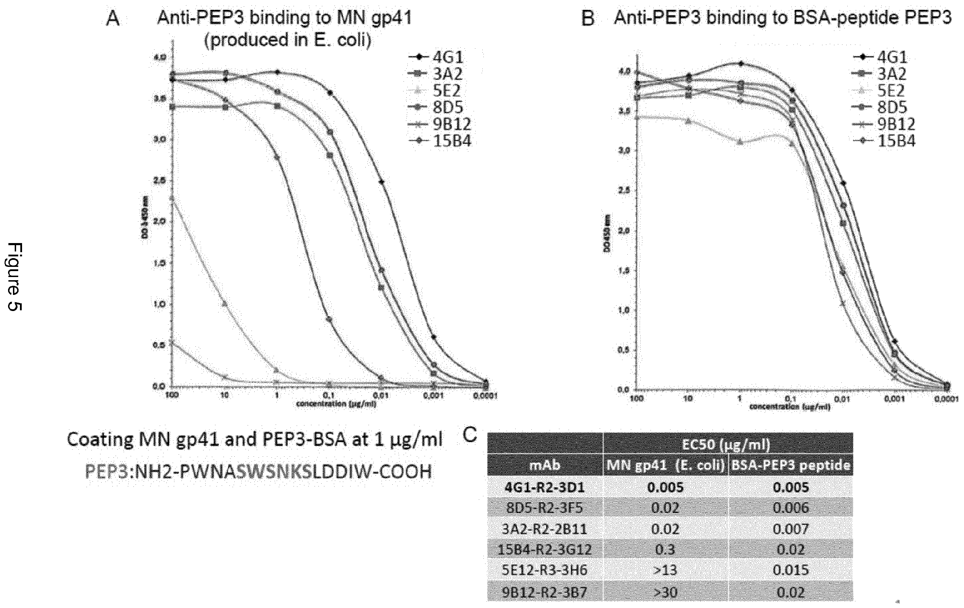

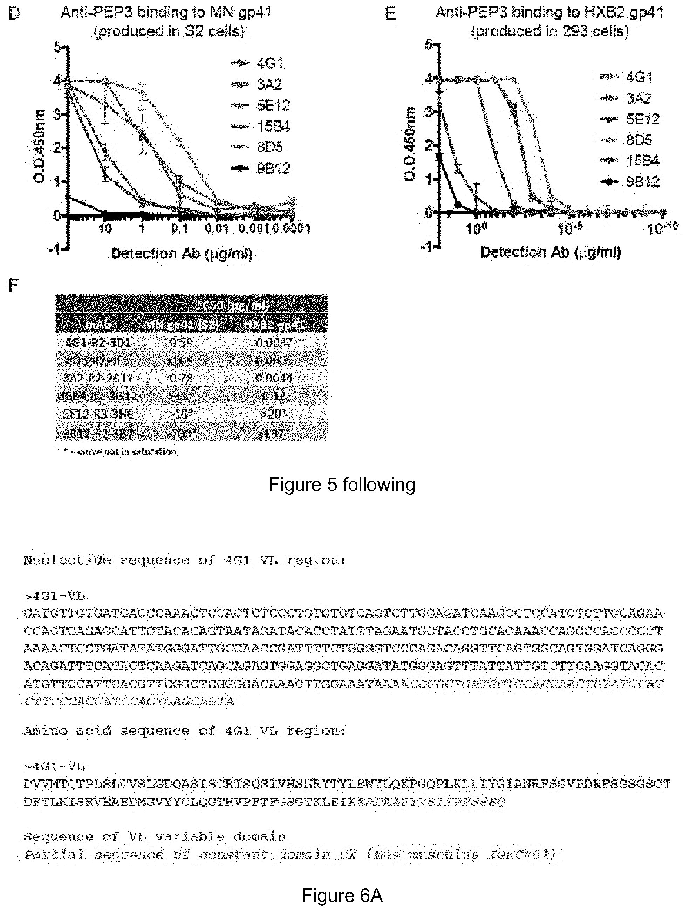

**Figure 5****. Characterization of anti-PEP3 gp41 mAbs by ELISA on PEP3 peptide and gp41 recombinant proteins.** ELISA microtiter plates were coated with 50µl of a solution at 1 µg/ml of **(A)** HIV MN gp41 or **(B)** BSA-PEP3 peptides. PEP3 sequence is detailed under the panel A. Then the binding of 50 µl of various dilutions of the 6 mAbs from 100-0.0001 µg/ml were tested and revealed with a HRP-conjugated F(ab)'2 fragment of goat anti-mouse IgG. **(C)** EC50 of the 6 anti-PEP3 mAbs binding to MN gp41 (produced in E. Coli) and BSA-PEP3 are presented in µg/ml of antibody. ELISA microtiter plates were coated with 100 µl of a solution at 1 µg/ml of **(D)** HIV MN gp41 (produced in S2 cells) or **(E)** HXB2 gp41 (produced in 293 Cells). Then the binding of 100 µl of various dilutions of the 6 mAbs from **(D)** 100-0.0001 µg/ml and **(E)** and 100-10⁻¹⁰ µg/ml were tested and revealed with a HRP-conjugated goat anti-mouse IgG. **(F)** EC50 of the 6 anti-PEP3 mAbs binding to MN gp41 (produced in S2 cells) and HXB2 are presented in µg/ml of antibody.

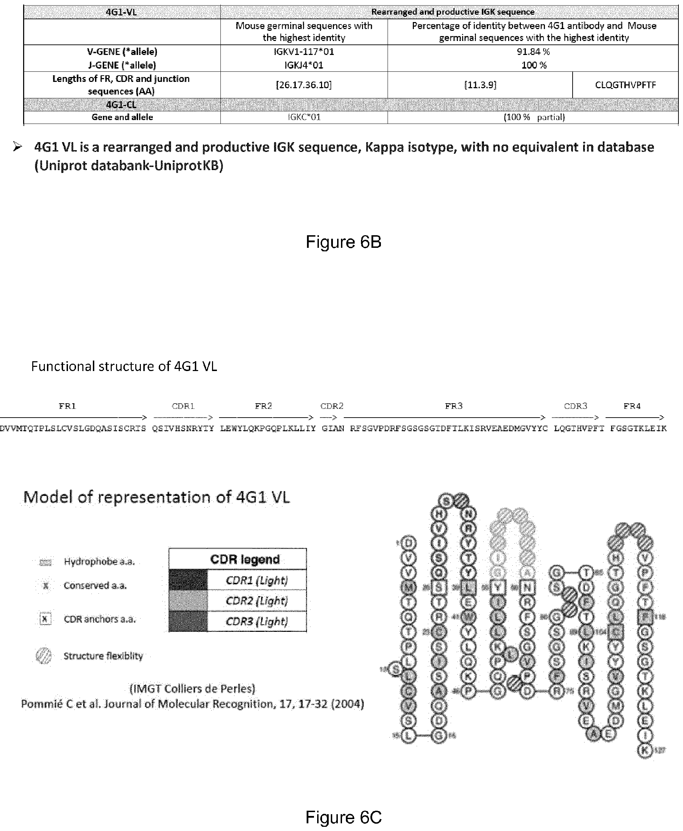

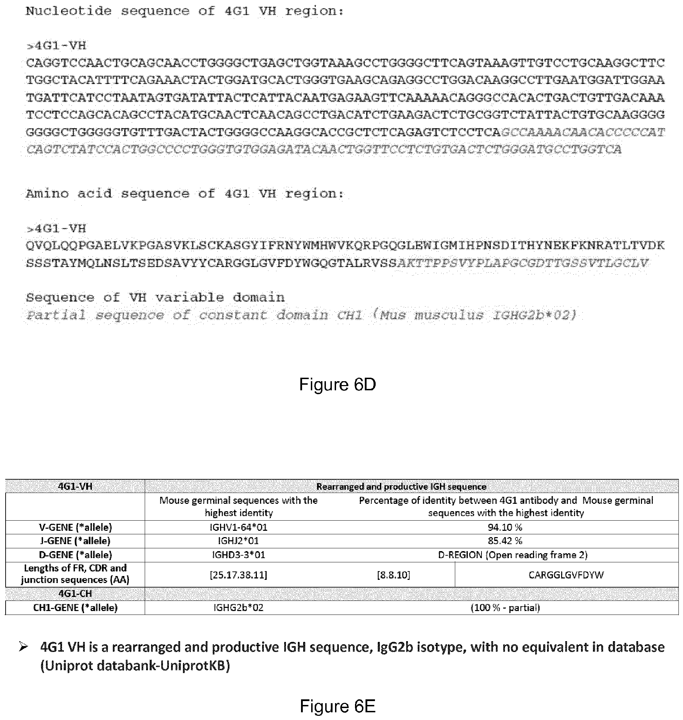

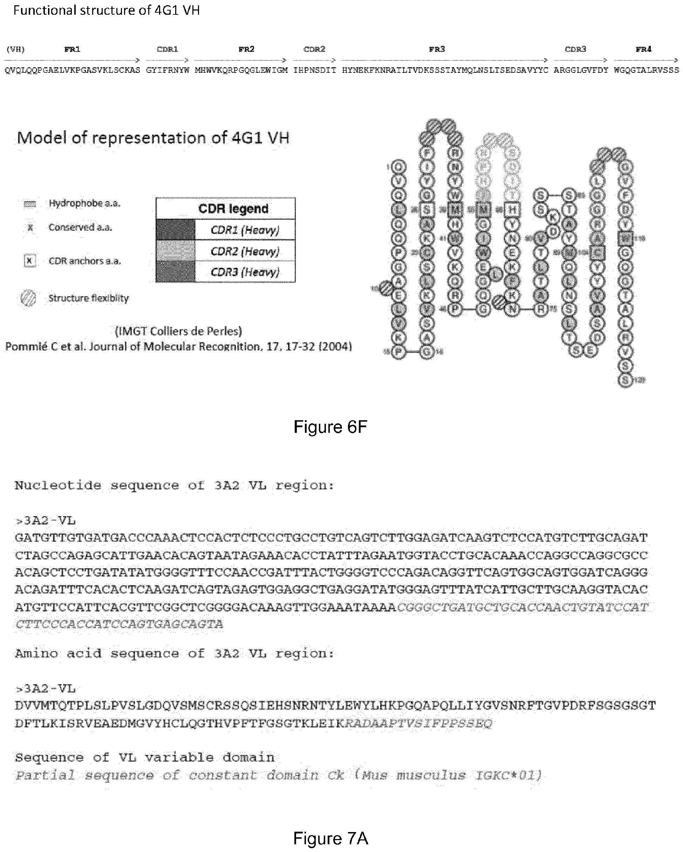

**Figure 6****. Sequences of 4G1 light (VL) and heavy (VH) chain variable regions. (A-C)** 4G1 VL is a rearranged and productive IGK sequence, Kappa isotype, with no equivalent in database (Uniprot databank-UniprotKB). **(A)** Nucleotide and amino acid sequence of 4G1 VL region. Sequence of 4G1 VL variable region is in black and partial sequence of constant Ck, Mus musculus IGKC^{∗}01 allele, is in red and italic. **(B)** Comparison of 4G1 VL with mouse germinal sequences (http://www.imgt.org/IMGT_vquest/share/textes/). **(C)** Functional structure and model of representation (IMGT Colliers de Perles) of 4G1 VL. **(D-F)** 4G1 VH is a rearranged and productive IGH sequence, IgG2b isotype, with no equivalent in database (Uniprot databank-UniprotKB). **(D)** Nucleotide and amino acid sequence of 4G1 VH region. Sequence of VH variable region is in black and partial sequence of constant domain CH1, Mus musculus IGHG2b^{∗}02 allele, is in red and italic. **(E)** Comparison of 4G1 VH with mouse germinal sequences (http://www.imgt.org/IMGT_vquest/share/textes/). **(F)** Functional structure and model of representation (IMGT Colliers de Perles) of 4G1 VH.

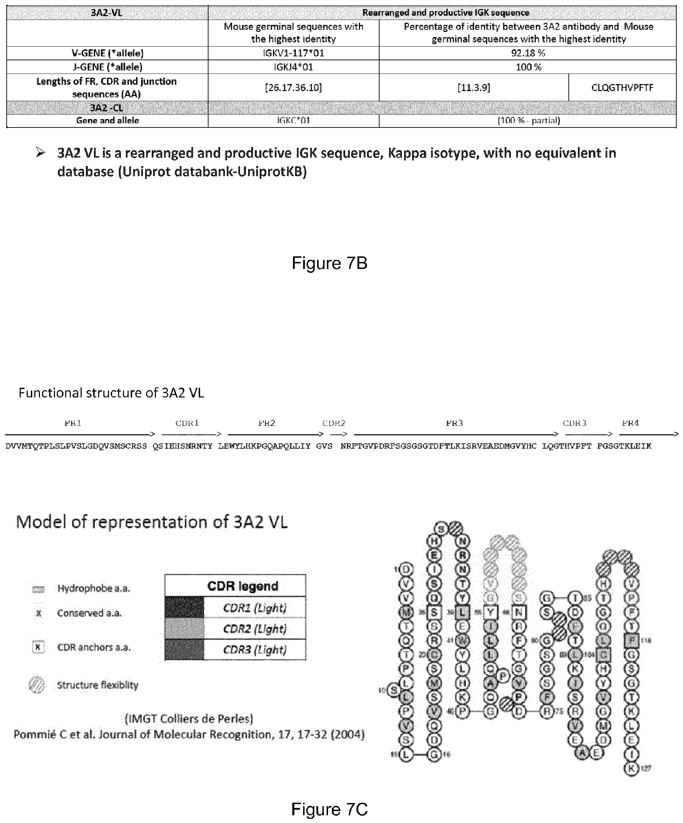

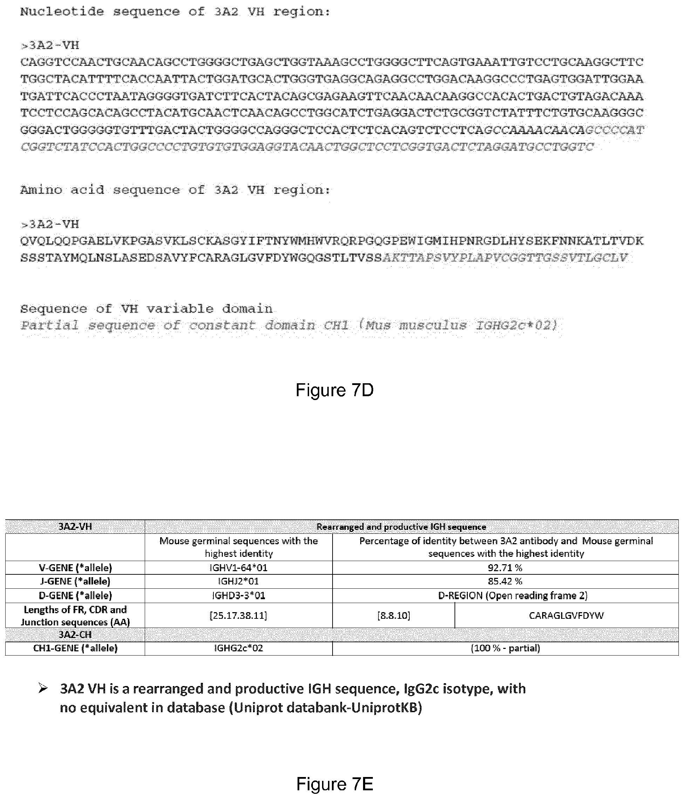

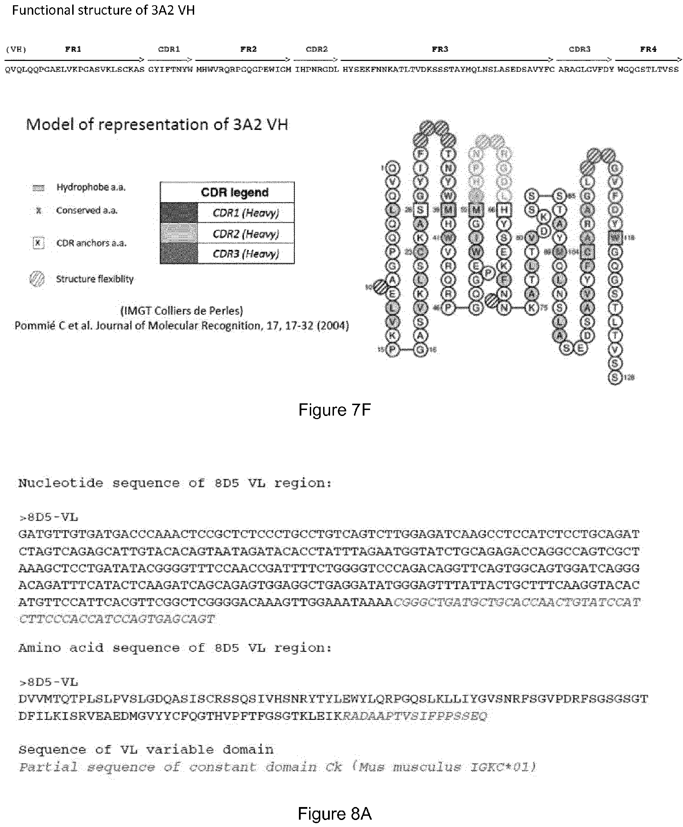

**Figure 7****. Sequences of 3A2 light (VL) and heavy (VH) chain variable regions. (A-C)** 3A2 VL is a rearranged and productive IGK sequence, Kappa isotype, with no equivalent in database (Uniprot databank-UniprotKB). **(A)** Nucleotide and amino acid sequence of 3A2 VL region. Sequence of 3A2 VL variable region is in black and partial sequence of constant Ck, Mus musculus IGKC*01 allele, is in red and italic. **(B)** Comparison of 3A2 VL with mouse germinal sequences (http://www.imgt.org/IMGT_vquest/share/textes/). **(C)** Functional structure and model of representation (IMGT Colliers de Perles) of 3A2 VL. **(D-F)** 3A2 VH is a rearranged and productive IGH sequence, IgG2c isotype, with no equivalent in database (Uniprot databank-UniprotKB). **(D)** Nucleotide and amino acid sequence of 3A2 VH region. Sequence of VH variable region is in black and partial sequence of constant domain CH1, Mus musculus IGHG2c^{∗}02 allele, is in red and italic. **(E)** Comparison of 3A2 VH with mouse germinal sequences (http://www.imgt.org/IMGT_vquest/share/textes/). **(F)** Functional structure and model of representation (IMGT Colliers de Perles) of 3A2 VH.

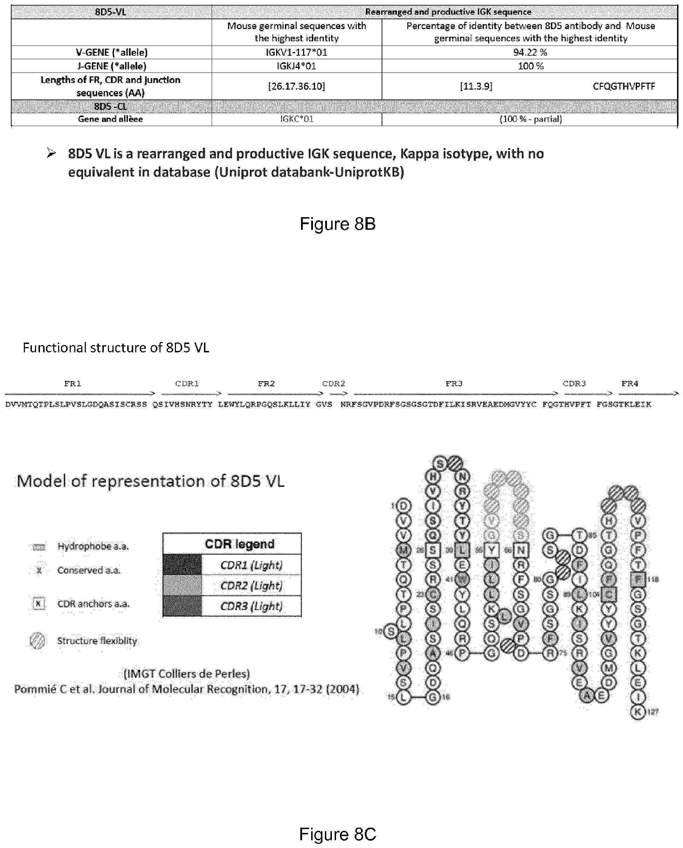

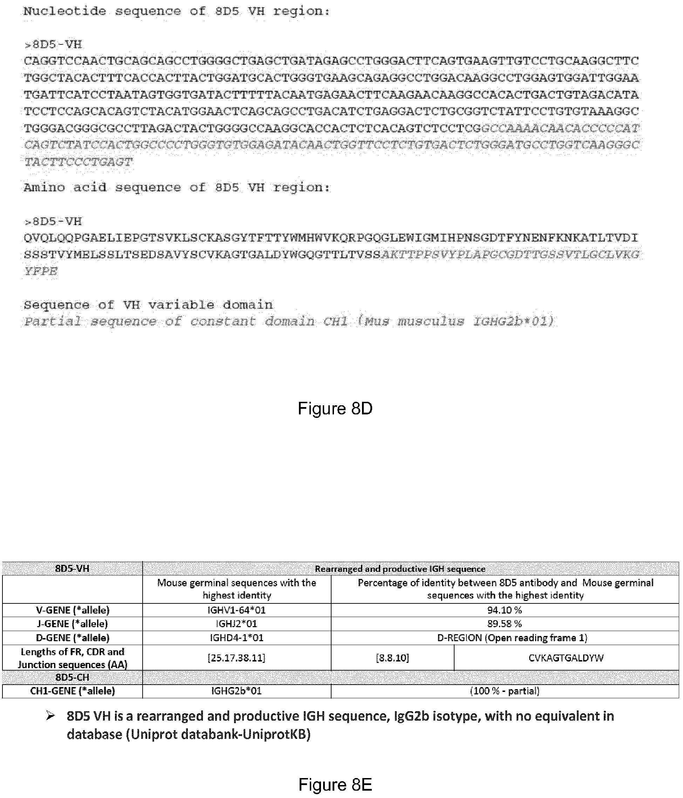

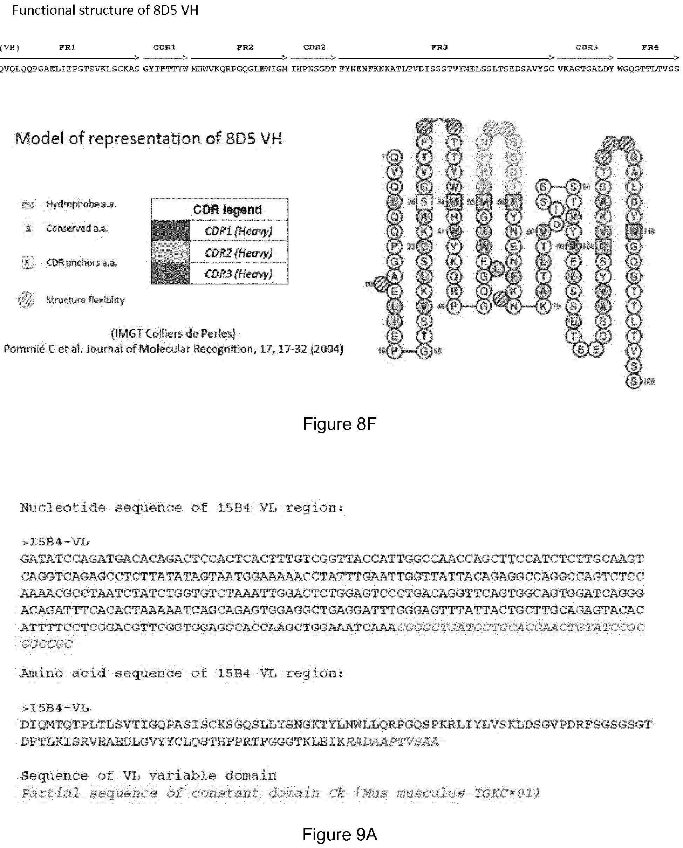

**Figure 8****. Sequences of 8D5 light (VL) and heavy (VH) chain variable regions. (A-C)** 8D5 VL is a rearranged and productive IGK sequence, Kappa isotype, with no equivalent in database (Uniprot databank-UniprotKB). **(A)** Nucleotide and amino acid sequence of 8D5 VL region. Sequence of 8D5 VL variable region is in black and partial sequence of constant Ck, Mus musculus IGKC^{∗}01 allele, is in red and italic. **(B)** Comparison of 8D5 VL with mouse germinal sequences (http://www.imgt.org/IMGT_vquest/share/textes/). **(C)** Functional structure and model of representation (IMGT Colliers de Perles) of 8D5 VL. **(D-F)** 8D5 VH is a rearranged and productive IGH sequence, IgG2b isotype, with no equivalent in database (Uniprot databank-UniprotKB). **(D)** Nucleotide and amino acid sequence of 8D5 VH region. Sequence of VH variable region is in black and partial sequence of constant domain CH1, Mus musculus IGHG2b^{∗}01 allele, is in red and italic. **(E)** Comparison of 8D5 VH with mouse germinal sequences (http://www.imgt.org/IMGT_vquest/share/textes/). **(F)** Functional structure and model of representation (IMGT Colliers de Perles) of 8D5 VH.

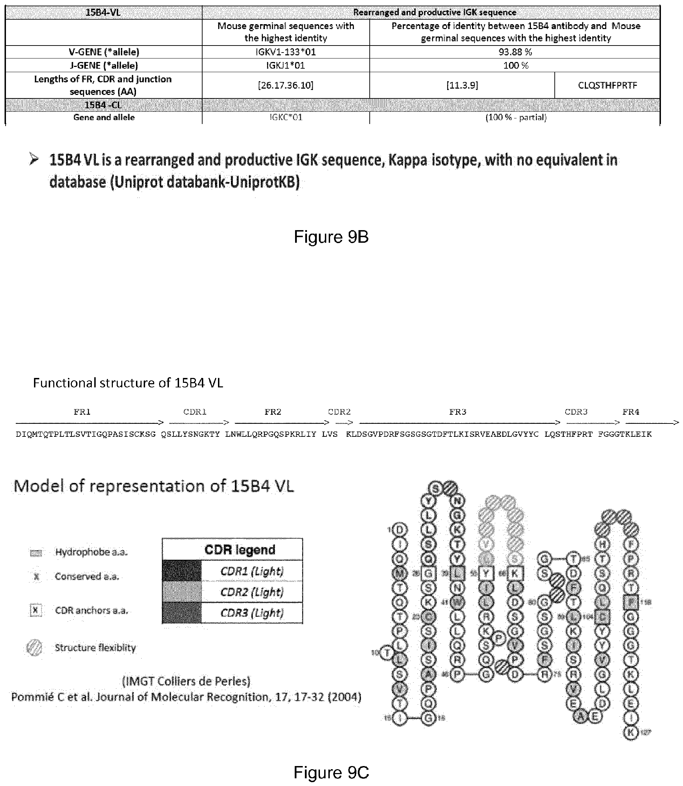

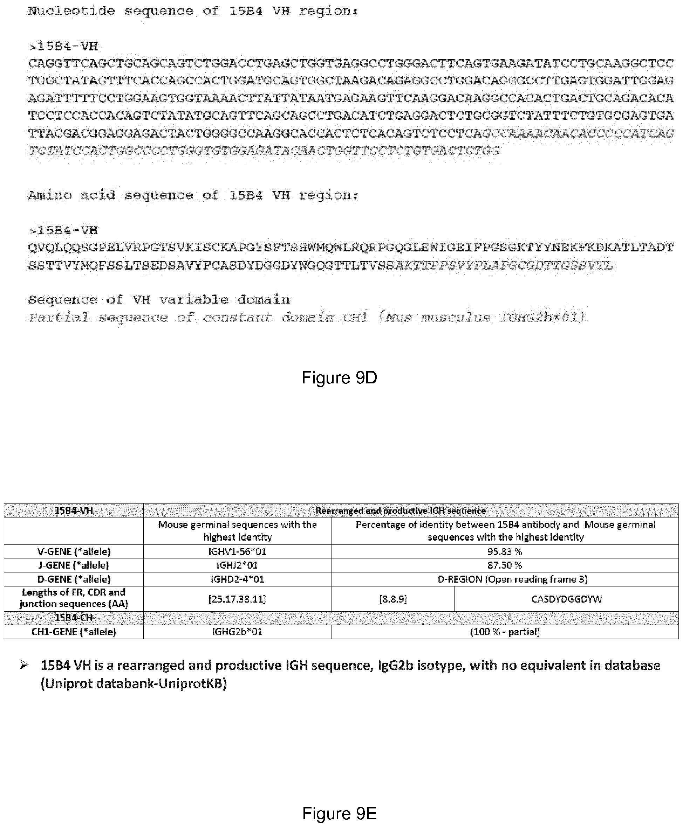

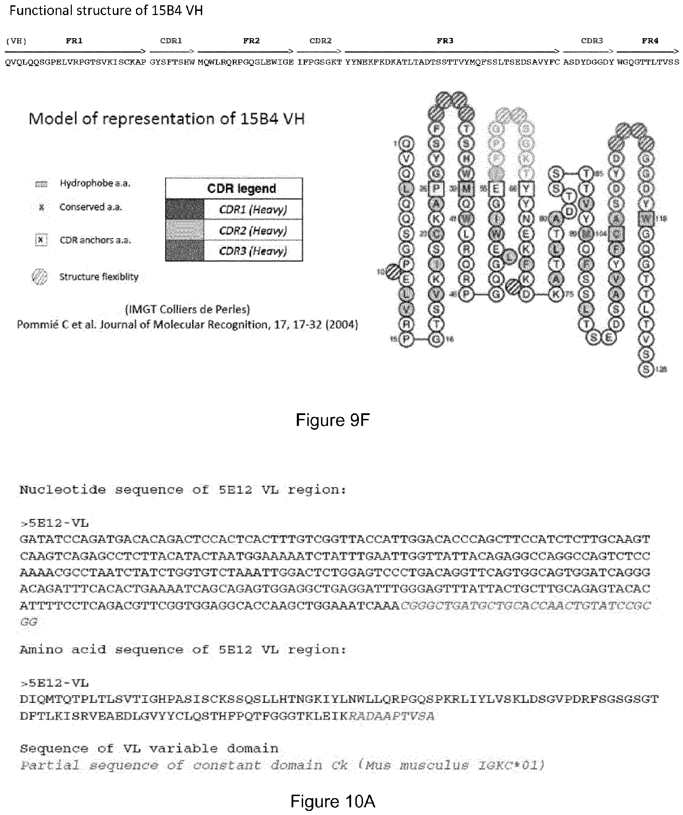

**Figure 9****. Sequences of 15B4 light (VL) and heavy (VH) chain variable regions. (A-C)** 15B4 VL is a rearranged and productive IGK sequence, Kappa isotype, with no equivalent in database (Uniprot databank-UniprotKB). **(A)** Nucleotide and amino acid sequence of 15B4 VL region. Sequence of 15B4 VL variable region is in black and partial sequence of constant Ck, Mus musculus IGKC^{∗}01 allele, is in red and italic. **(B)** Comparison of 15B4 VL with mouse germinal sequences (http://www.imgt.org/IMGT_vquest/share/textes/). **(C)** Functional structure and model of representation (IMGT Colliers de Perles) of 15B4 VL. **(D-F)** 15B4 VH is a rearranged and productive IGH sequence, IgG2b isotype, with no equivalent in database (Uniprot databank-UniprotKB). **(D)** Nucleotide and amino acid sequence of 15B4 VH region. Sequence of VH variable region is in black and partial sequence of constant domain CH1, Mus musculus IGHG2b^{∗}01 allele, is in red and italic. **(E)** Comparison of 15B4 VH with mouse germinal sequences (http://www.imgt.org/IMGT_vquest/share/textes/). **(F)** Functional structure and model of representation (IMGT Colliers de Perles) of 15B4 VH.

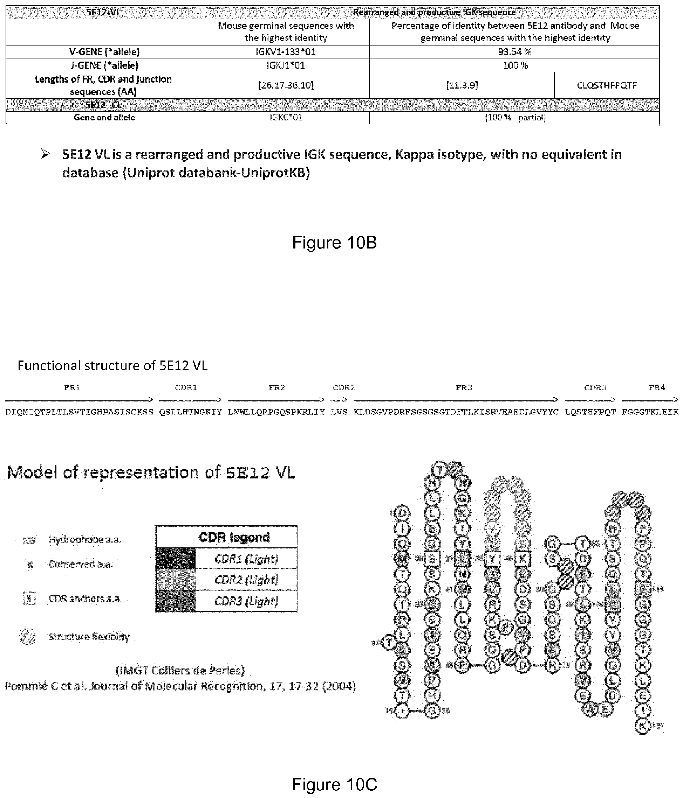

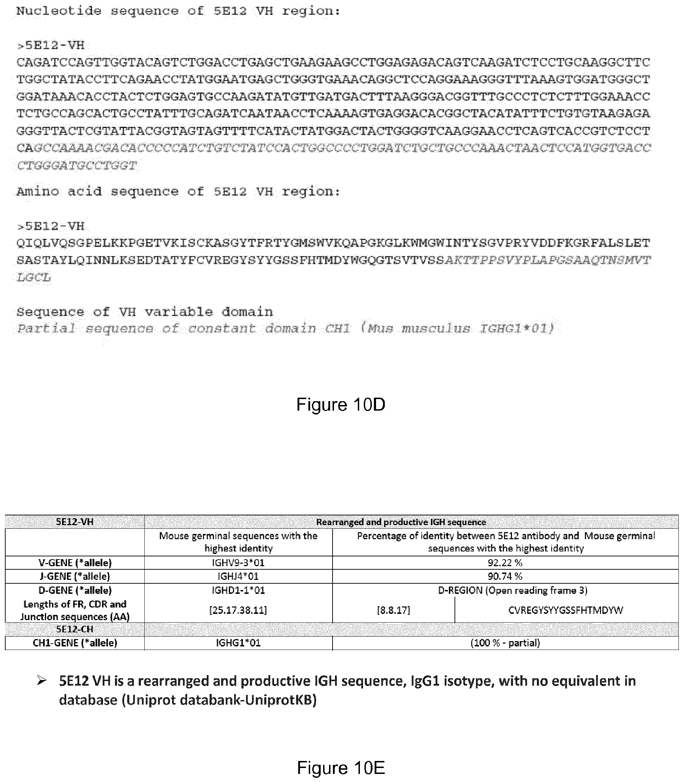

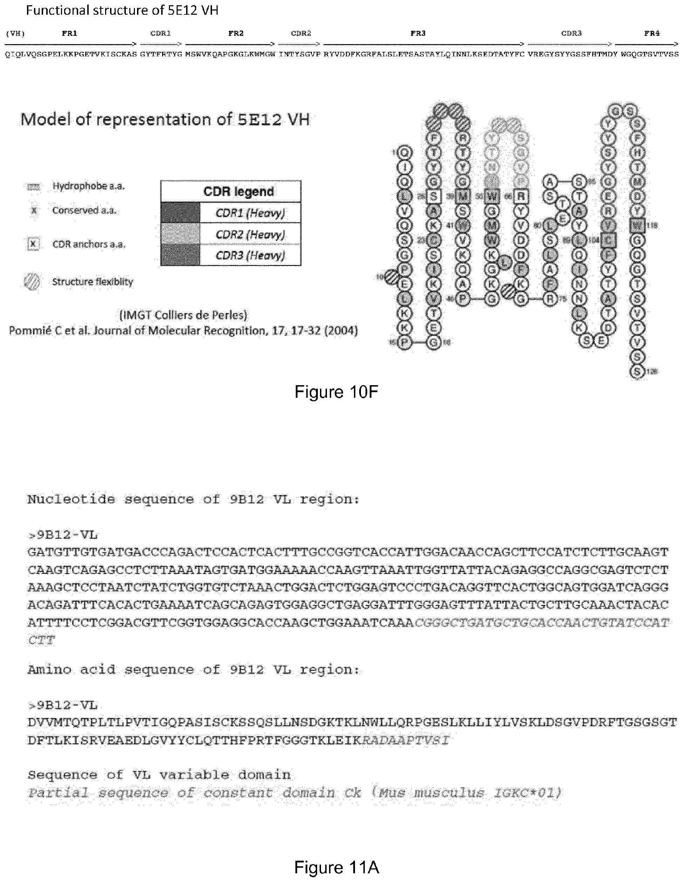

**Figure 10****. Sequences of 5E12 light (VL) and heavy (VH) chain variable regions. (A-C)** 5E12 VL is a rearranged and productive IGK sequence, Kappa isotype, with no equivalent in database (Uniprot databank-UniprotKB). **(A)** Nucleotide and amino acid sequence of 5E12 VL region. Sequence of 5E12 VL variable region is in black and partial sequence of constant Ck, Mus musculus IGKC^{∗}01 allele, is in red and italic. **(B)** Comparison of 5E12 VL with mouse germinal sequences (http://www.imgt.org/IMGT_vquest/share/textes/). **(C)** Functional structure and model of representation (IMGT Colliers de Perles) of 5E12 VL. **(D-F)** 5E12 VH is a rearranged and productive IGH sequence, IgG1 isotype, with no equivalent in database (Uniprot databank-UniprotKB). **(D)** Nucleotide and amino acid sequence of 5E12 VH region. Sequence of VH variable region is in black and partial sequence of constant domain CH1, Mus musculus IGHG1^{∗}01 allele, is in red and italic. **(E)** Comparison of 5E12 VH with mouse germinal sequences (http://www.imgt.org/IMGT _vquest/share/textes/). **(F)** Functional structure and model of representation (IMGT Colliers de Perles) of 5E12 VH.

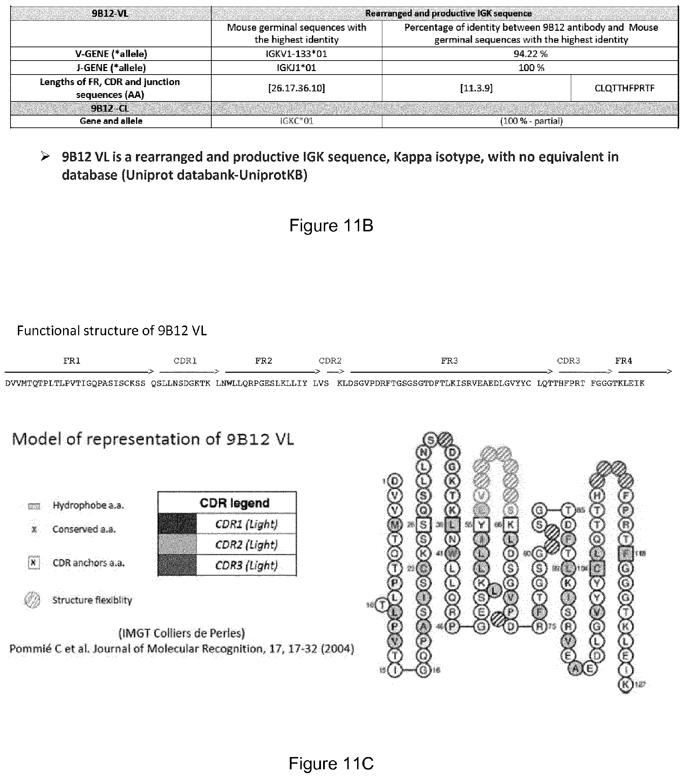

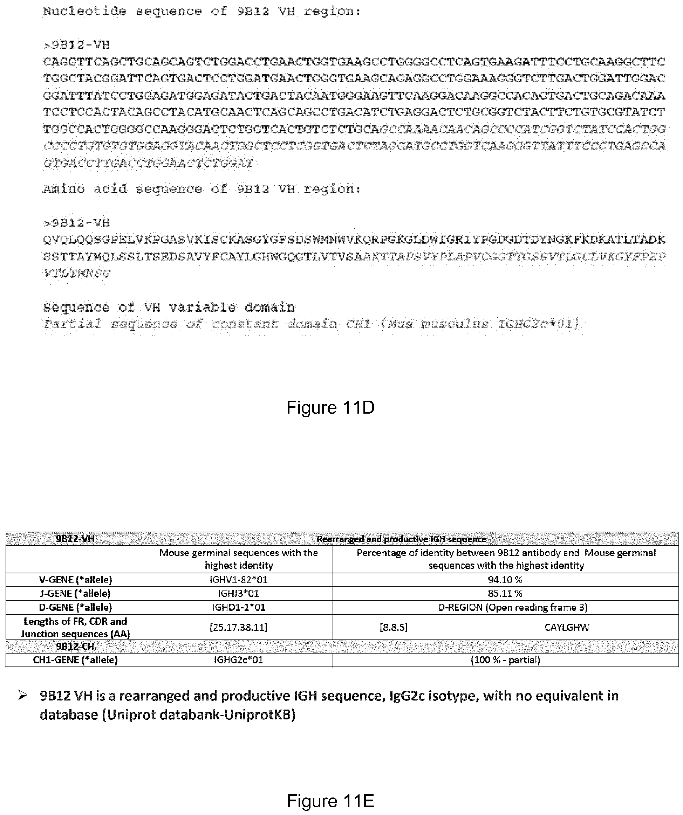

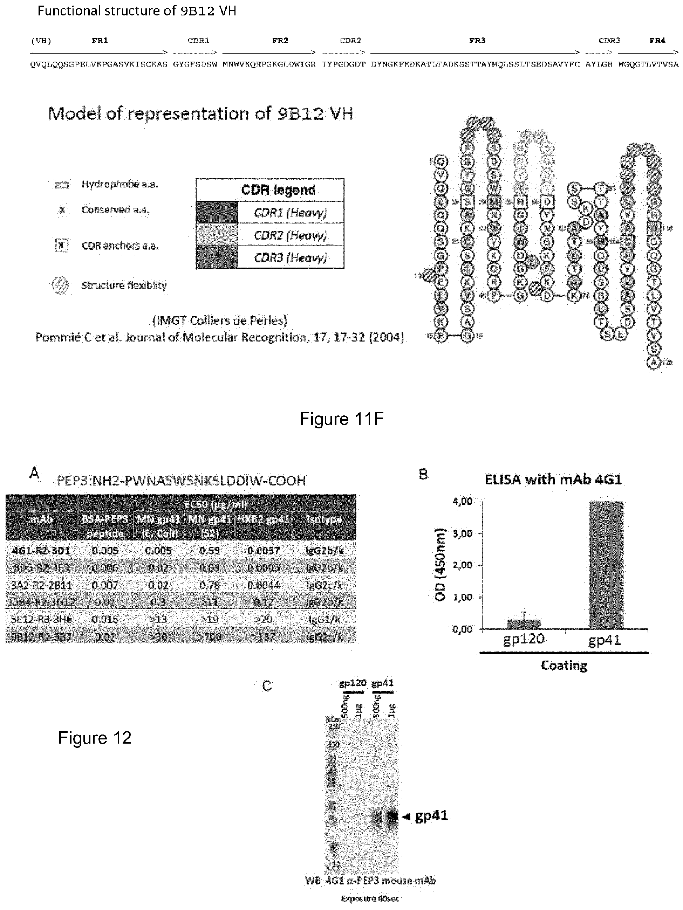

**Figure 11****. Sequences of 9B12 light (VL) and heavy (VH) chain variable regions. (A-C)** 9B12 VL is a rearranged and productive IGK sequence, Kappa isotype, with no equivalent in database (Uniprot databank-UniprotKB). **(A)** Nucleotide and amino acid sequence of 9B12 VL region. Sequence of 9B12 VL variable region is in black and partial sequence of constant Ck, Mus musculus IGKC^{∗}01 allele, is in red and italic. **(B)** Comparison of 9B12 VL with mouse germinal sequences (http://www.imgt.org/IMGT_vquest/share/textes/). **(C)** Functional structure and model of representation (IMGT Colliers de Perles) of 9B12 VL. **(D-F)** 9B12 VH is a rearranged and productive IGH sequence, IgG2c isotype, with no equivalent in database (Uniprot databank-UniprotKB). **(D)** Nucleotide and amino acid sequence of 9B12 VH region. Sequence of VH variable region is in black and partial sequence of constant domain CH1, Mus musculus IGHG2c^{∗}01 allele, is in red and italic. **(E)** Comparison of 9B12 VH with mouse germinal sequences (http://www.imgt.org/IMGT _vquest/share/textes/). **(F)** Functional structure and model of representation (IMGT Colliers de Perles) of 9B12 VH.
**Figure 12****. Summary of anti-PEP3 mAbs affinity and specificity of 4G1. (A)** Summary of the 6 mAbs EC50 of binding on coated BSA-PEP3, MN gp41 (produced in E Coli), MN gp41 (produced in S2) and HXB2 gp41 as well as antibody isotypes. 4 mAbs: 4G1, 8D5, 3A2 and 15B4 bind both to gp41 recombinant protein and PEP3 peptide. 4G1 was selected because it has the highest binding efficiency to gp41 protein and PEP3 peptide in the first ELISA performed (MN gp41 produced in E. Coli). 2 mAbs: 5E12 and 9B12 bind PEP3 peptide but not, or with very low efficiency, to gp41 protein. **(B, C)** The anti-PEP3 gp41 mAb 4G1 binds to gp41 but not to gp120. **(B)** ELISA: wells of microtiter plates were coated overnight with 10 µg of D117 III gp41 or D117 III gp120 recombinant proteins and bindings of 4G1 (1 µg/well) to the proteins were revealed with goat anti-mouse IgG-HRP. Results are shown as the mean ± SD of triplicates. **(B)** Immunoblot with 4G1 mAb: 500 ng or 1 µg of D117 III gp120 or MN gp41 recombinant proteins were separated on 4%-20% Tris-Bis SDS-PAGE in reducing conditions and transferred on PVDF membranes. Antibody binding was tested at 1 µg/ml and revealed with goat anti-mouse IgG-HRP (1:10000).

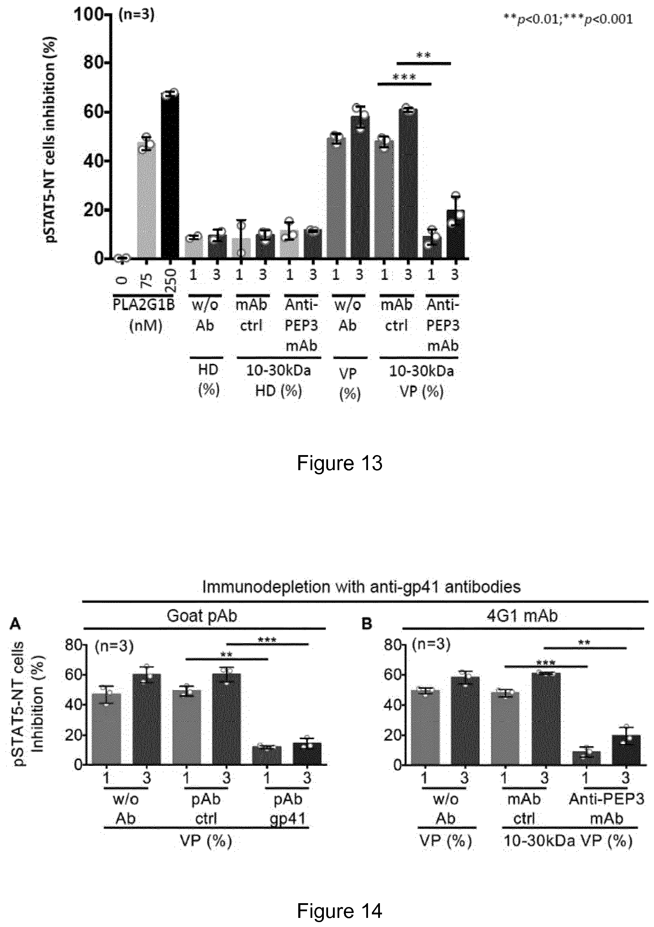

**Figure 13****. Immunodepletion of viremic plasma with 4G1 anti-PEP3 abrogates the inhibitory activity of PLA2-GIB on pSTAT5 NT in CD4 T cells.** Purified CD4 T cells from 3 independent healthy donors were treated in 3 independent experiments for 30 min with PLA2-GIB alone, as positive control of sensitivity to PLA2-GIB, healthy donor (HD) plasma or viremic patient (VP) plasma alone (w/o Ab), or 10-30 kDa HD or VP fractions previously treated with 4G1 anti-PEP3 mAb or control mAb (mAb ctrl) and stimulated with IL-7 for 15 min. Results are presented as the mean ± SD of the percentage of inhibition of pSTAT5 NT normalized with the pSTAT5 NT in response to IL-7 in buffer. ** means *p*<0.01 and *** means that *p*<0.001 with two tailed unpaired t-test for the difference of pSTAT5 NT inhibition with mAb ctrl-treated relatively to 4G1-treated 10-30 kDa VP plasma.
Figure 14. Immunodepletion of viremic plasma with goat anti-gp41 antibody or 4G1 anti-PEP3 similarly abrogates the inhibitory activity of PLA2-GIB on pSTAT5 NT in CD4 T cells. Summary of results presented in Figures 3 and 13 to compare 4G1 and goat anti-gp41 antibody effects. **(A, B)** Inhibitory activity of 1 or 3% of VP plasma depleted with anti-gp41 (gp41) polyclonal antibody (pAb) **(A)** or with 4G1 anti-PEP3 mAb (Anti-PEP3) **(B)**, control (ctrl) or not depleted (w/o Ab) on CD4 T cells. **(A, B)** Results are shown as the mean ± SD of the percentage of pSTAT5 NT cells inhibition on 3 donors. ***p*<0.01, ****p*<0.001 by two tailed unpaired *t*-test.

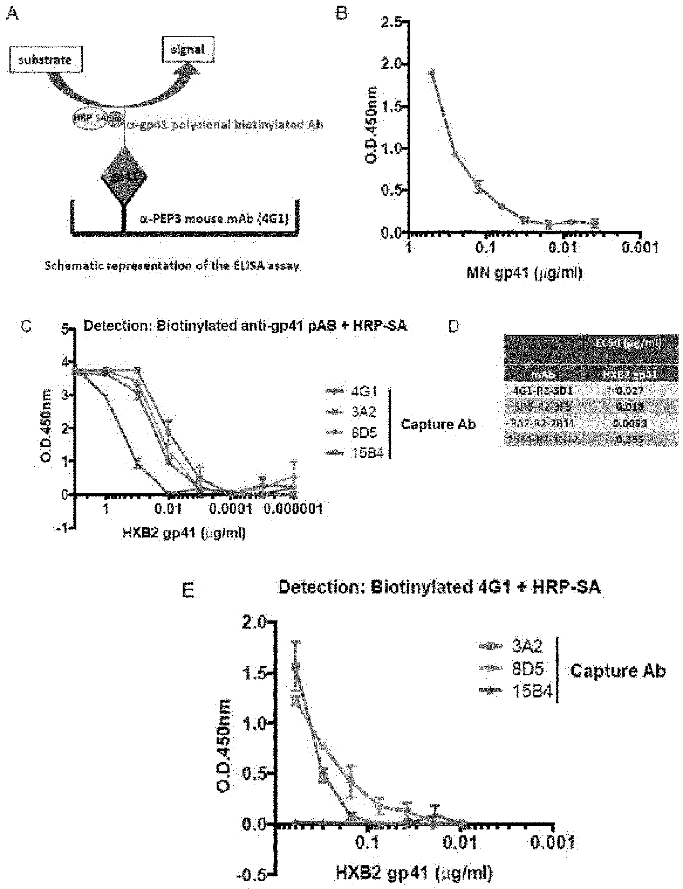

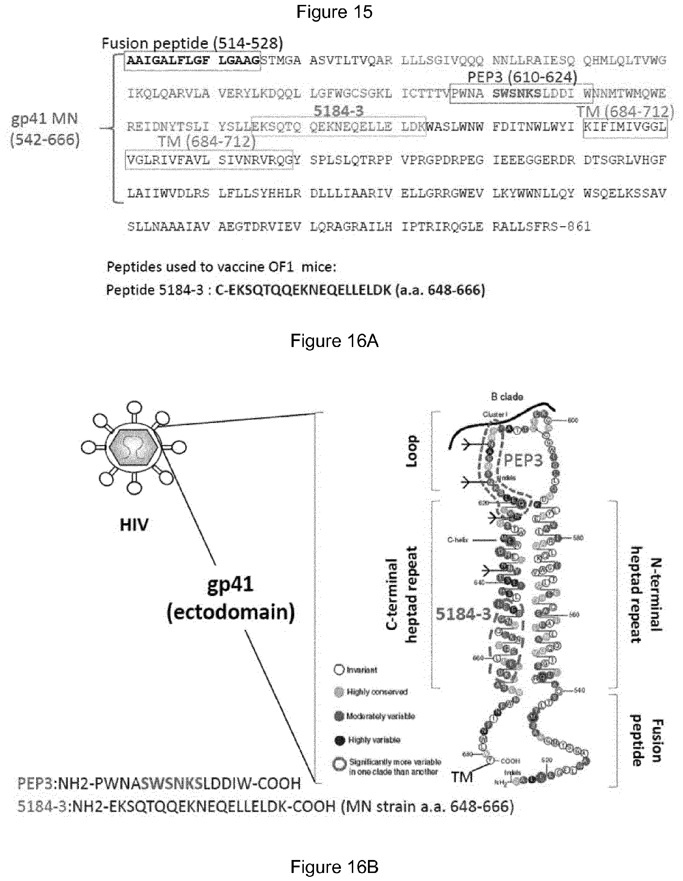

**Figure 15****. Sandwich ELISA with 4G1 anti-PEP3 mAb and goat anti-gp41 pAb. (A)** Schematic representation of the ELISA. **(B)** 4G1 can be used together with goat anti-gp41 pAb to quantify MN gp41 by ELISA. Test of pairing of 4G1 with goat anti-gp41 pAb using MN gp41 antigen. Microtiter plates were coated with 100 µl/well of 4G1 at 10 µg/ml, after blocking, various amount of MN gp41 recombinant (produced in S2 cells) were added. Detection of gp41 bound to coated 4G1 was performed using 100 µl/well of biotinylated goat anti-gp41 pAb at 5 µg/ml and revealed with HRP-streptavidin at 1:20000. **(C, D)** 4G1, 8D5, 3A2 and 15B4 can be used together with goat anti-gp41 pAb to quantify HXB2 gp41. **(C)** Test of pairing of 4G1, 3A2, 8D5 and 15B4 with anti-gp41 pAb using HXB2 gp41 antigen. Microtiter plates were coated with 100 µl/well of 4G1, 3A2, 8D5 and 15B4 mAb at 10 µg/ml, after blocking, various amount of HXB2 gp41 recombinant were added. Detection of gp41 bound to coated anti-PEP3 mAb was performed using 100 µl/well of biotinylated goat anti-gp41 pAb at 5 µg/ml and revealed with HRP-streptavidin at 1:20000. **(D)** EC50 of HXB2 gp41 detection in µg/ml of gp41 presented in C. **(E)** 4G1 can be used together with other anti-PEP3 mAb to quantify HXB2 gp41. Test of pairing of 4G1 with 3A2, 8D5 and 15B4 using HXB2 gp41 antigen. Microtiter plates were coated with 100 µl/well of **(E)** 3A2, 8D5 and 15B4 mAb at 10 µg/ml, after blocking, various amount of HXB2 gp41 recombinant were added. Detection of gp41 bound to coated anti-PEP3 mAb was performed using 100 µl/well of biotinylated 4G1 at 20 µg/ml and revealed with HRP-streptavidin at 1:20000.
**Figure 16****: Development of additional anti-gp41 mAb to detect gp41 in HIV viremic patient plasma by ELISA. (A)** Position of PEP3 and 5184-3 peptides in gp41 sequence. Both peptides are located in the ectodomain of MN gp41. **(B)** Molecular localization of PEP3 and 5184-3 peptides. PEP3 is located in the C-terminal portion of the gp41 loop and 5184-3 peptide in the C-terminal part of the HR2 (=C-terminal heptad repeat).

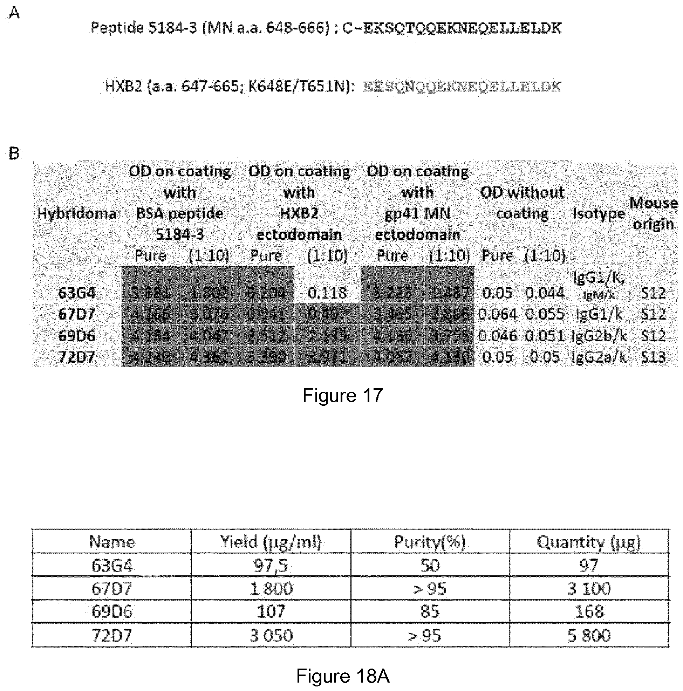

**Figure 17****: Characterization of anti-HR2 gp41 hybridoma. (A) Sequence of 5184-3** peptide used for mice immunization and the corresponding sequence in HXB2 recombinant protein. Amino acid substitution in HXB2 are indicated in red **(B)** ELISA with hybridoma supernatant on MN and HXB2 gp41 recombinant proteins. Microtiter plates were coated with 50 µl of a solution at 1 µg/ml of BSA-5184-3 peptide, MN and HXB2 gp41 recombinant proteins. Hybridoma supernatant were tested pure or at 1:10. Antibody binding was revealed with HRP-anti-mouse IgG. Isotypes of antibodies produced in hybridoma supernatant are indicated. 63G4 hybridoma is polyclonal with primarily IgG1 and some IgM antibodies. 63G4, 67D7 and 69D9 are all derived from mouse S12. 72D7 is derived from mouse S13.

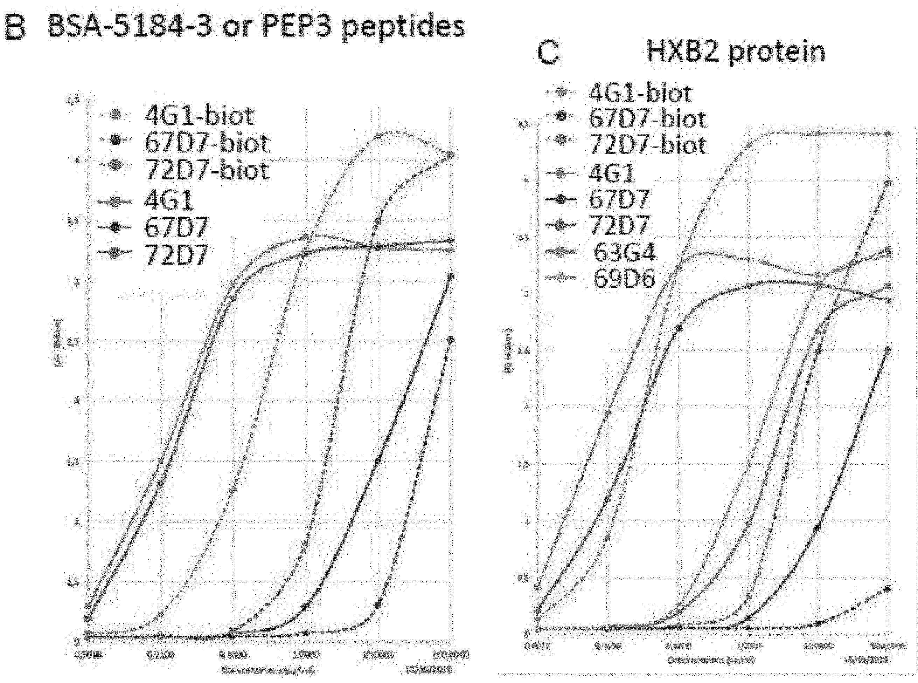

**Figure 18****: Purified anti-HR2 hybridoma Ab produced in vivo in mice from Ascite and 4G1 were biotinylated or not and validated on gp41 peptide and HXB2 gp41 by ELISA. (A)** Purity and amount of purified antibodies for the 4 anti-HR2 antibodies. Good yield and purity>95% for 67D7 and 72D7 allow biotinylation of these antibodies. **(B, C)** Test of antibodies binding to gp41 peptides and HXB2 gp41 protein. **(B)** Microtiter plates were coated with 50 µl of PEP3 (2 µg/ml) or BSA-5184-3 (1 µg/ml). 4G1 and biotinylated-4G1 (4G1-biot) were tested on PEP3 coated wells. 72D7 and 67D7 biotinylated (72D7-biot, 67D7-biot) or not were tested on BSA-5184-3 peptide coated wells. **(C)** Microtiter plates were coated with 50 µl of HXB2 gp41 (1 µg/ml). **(B, C)** The binding of 50 µl of various dilutions of antibodies from 100-0.0001 1 µg/ml were revealed with HRP-streptavidin (1:1000) for biotinylated antibodies or HRP-anti-mouse IgG (1:5000) for unbiotinylated antibodies.

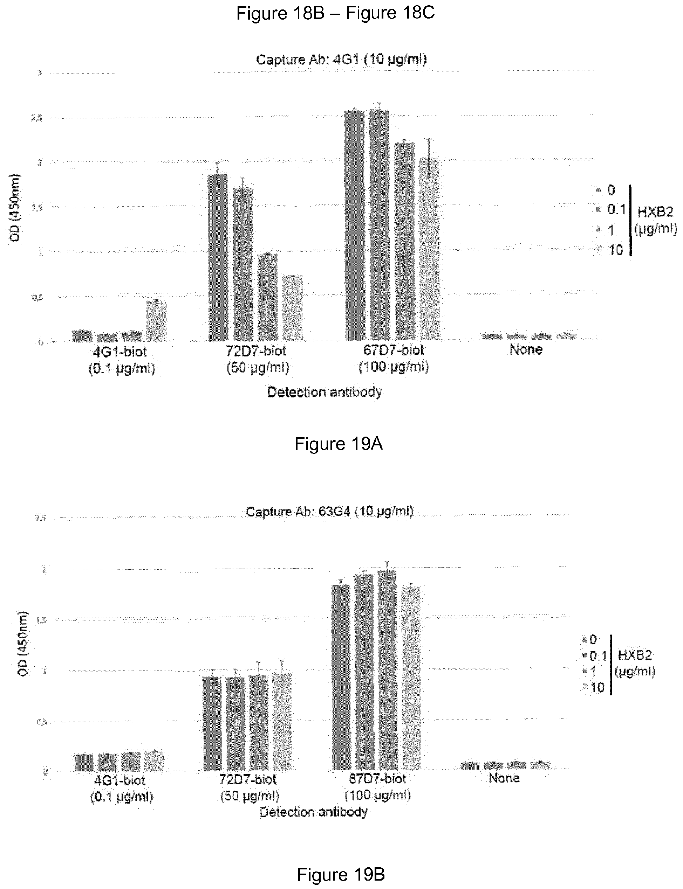

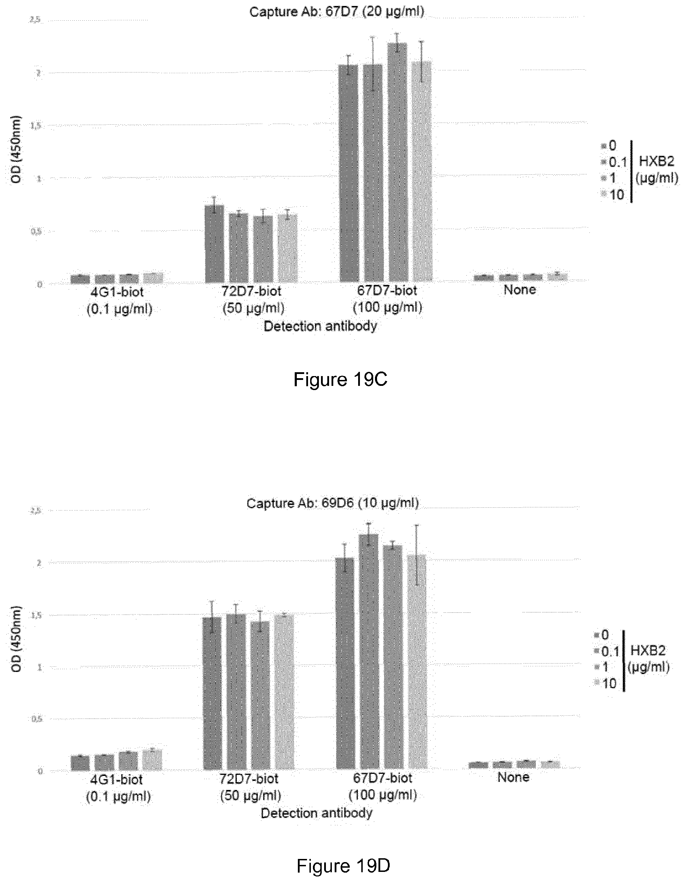

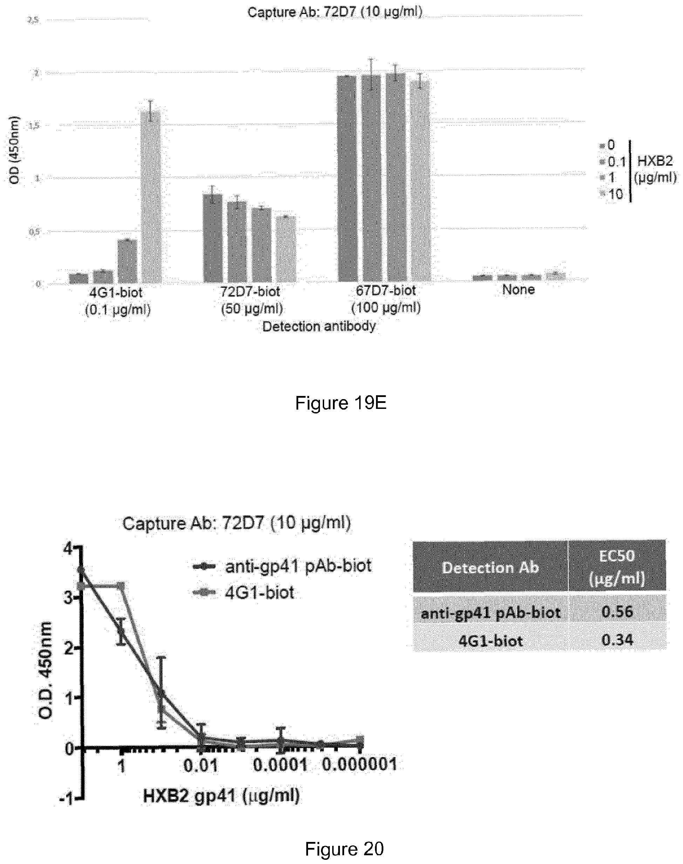

**Figure 19****: Test of pairing of anti-HR2 and 4G1 antibodies by sandwich ELISA.** Uniquely 72D7 as capture antibody and biotinylated 4G1 as detection antibody allow the quantification of HXB2 gp41. Test of pairing of biotinylated 4G1 (0.1 µg/ml), 72D7 (50 µg/ml) and 67D7 (100 µg/ml) on capture antibodies **(A)** anti-PEP3 4G1 (10 µg/ml), **(B)** anti-HR2 63G4 (10 µg/ml), **(C)** anti-HR2 67D7 (20 µg/ml), **(D)** anti-HR2 69D6 (10 µg/ml) or **(E)** anti-HR2 72D7 (10 µg/ml). **(A-E)** Microtiter plates were coated with 50 µl of capture Ab solution. 50 µl of various dilutions of HXB2 gp41 (0, 0.1, 1 and 10 µg/ml) were added. The pairing was tested by sandwich ELISA by adding biotinylated detection Ab and was revealed with HRP-streptavidin (1:1000).
**Figure 20****. Sandwich ELISA with anti-HR2 72D7 and 4G1 mAb or goat anti-gp41** pAb. 72D7 can be used together with 4G1 mAb or goat anti-gp41 pAb to quantify HXB2 gp41. Test of pairing of anti-HR2 72D7 mAb with 4G1 mAb or goat anti-gp41 pAb using HXB2 gp41 antigen. Microtiter plates were coated with 100 µl/well of 72D7 at 10 µg/ml, after blocking, various amount of HXB2 gp41 recombinant were added. Detection of gp41 bound to coated 72D7 was performed using 100 µl/well of biotinylated 4G1 mAb at 20 µg/ml or of biotinylated goat anti-gp41 pAb at 5 µg/ml and revealed with HRP-streptavidin at 1:20000.

### Detailed description of the invention

The invention generally relates to novel antibodies and the uses thereof. The invention particularly discloses anti-PEP3 antibodies that can be used to inhibit gp41 cofactor activity in HIV viremic patients. In addition, these antibodies can be used to quantify gp41 in the plasma of HIV viremic patients and to follow the outcome of gp41 concentration during antiretroviral treatment.

### Definitions

As used herein, the term "gp41" designates the glycoprotein of essentially 41kDa encoded by a HIV virus. The sequence of gp41 is available on Genbank, e.g., No. AAC31817.1. The amino acid sequence of an exemplary gp41 protein is shown as SEQ ID NO: 1. Amino acids 97 to 111 of SEQ ID NO: 1 (SEQ ID NO: 2) represent a domain of gp41 designated PEP3 sequence or domain or peptide.

The term gp41 designates any form of the protein, including any natural variants thereof, such as variants resulting from polymorphism or splicing. The term "gp41" designates typically a protein comprising the sequence of SEQ ID NO: 1 or any natural variants thereof, such as variants resulting from polymorphism or splicing.

Generally, the term "antibody" designates any immunoglobulin-type molecule comprising heavy and light chains. Typical antibodies possess the basic monomeric "H2L2" structure consisting of 2 Heavy and 2 Light chains. Each heavy chain is paired with a light chain. Heavy and light chains comprise a "variable region" or "variable domain". The variable domain of the heavy chain may be referred to as "VH." The variable domain of the light chain may be referred to as "VL." These domains are generally the most variable parts of an antibody and contain the antigen-binding sites. A light or heavy chain variable region (VL or VH) consists of a framework region interrupted by three hypervariable regions referred to as "complementarity determining regions" or "CDRs".

Antibody "fragments" designate any portion of an antibody that confers antigen binding. Fragments typically designate Fab, Fab'2, ScFv, nanobodies, or CDR.

Antibody "variant", as used herein, refers to an antibody which retains the antigenic specificity of a reference antibody but wherein one or more amino acid residues are (e.g., chemically, or biologically) modified, typically to improve its properties. Examples of such chemical modifications include, e.g. by alkylation, PEGylation, acylation, ester or amide formation, and the like. In particular, a variant is an antibody as disclosed herein that is modified to contain one or more additional non-proteinaceous moieties such as water-soluble polymers. Examples of water-soluble polymers include, but are not limited to, PEG, copolymers of ethylene glycol/propylene glycol, carboxymethylcellulose, dextran and polyvinyl alcohol.

Variants may also be generated to increase or decrease the extent to which the antibody is glycosylated. Addition or deletion of glycosylation sites to an antibody may be conveniently accomplished by altering the amino acid sequence such that one or more glycosylation sites is created or removed. Where the antibody comprises an Fc region, the carbohydrate attached thereto may be altered. Native antibodies produced by mammalian cells typically comprise a branched, biantennary oligosaccharide that is generally attached by an N-linkage to Asn297 of the CH2 domain of the Fc region (see e.g., Wright et al. TIBTECH, 1997, 15:26-32). The oligosaccharide may include various carbohydrates, e.g., mannose, N-acetyl glucosamine (GlcNAc), galactose, and sialic acid, as well as a fucose attached to a GlcNAc in the "stem" of the biantennary oligosaccharide structure. In some embodiments, modifications of the oligosaccharide in an antibody of the invention may be made in order to create antibody variants with certain improved properties.

In one embodiment, the variant has a modified Fc region in order to alter ADCC (through a modulation of FcgammaRIII binding).

In one embodiment, antibody variants are provided having a carbohydrate structure that lacks fucose attached (directly or indirectly) to an Fc region. For example, the amount of fucose in such antibody may be from 1% to 80%, from 1% to 65%, from 5% to 65% or from 20% to 40%). The amount of fucose is determined by calculating the average amount of fucose within the sugar chain at Asn297, relative to the sum of all glycostructures attached to Asn 297 (e. g. complex, hybrid and high mannose structures) as measured by MALDI-TOF mass spectrometry. Asn297 refers to the asparagine residue located at about position 297 in the Fc region (Eu numbering of Fc region residues); however, Asn297 may also be located about ± 3 amino acids upstream or downstream of position 297, i.e., between positions 294 and 300, due to minor sequence variations in antibodies. Examples of publications related to "defucosylated" or "fucose-deficient" antibody variants include, but are not limited to, Okazaki et al. J. Mol. Biol. 336: 1239-1249 (2004) and Yamane-Ohnuki N, Satoh M. mAbs. 2009;1:230-236. Examples of cell lines capable of producing defucosylated antibodies include Led 3 CHO cells deficient in protein fucosylation (Ripka et al. Arch. Biochem. Biophys. 249:533-545 (1986)), and knockout cell lines, such as alpha- 1 ,6-fucosyltransferase gene, FUT8, knockout CHO cells (see, e.g., Yamane-Ohnuki et al. Biotech. Bioeng. 87: 614 (2004); Kanda, Y. et al., Biotechnol. Bioeng., 94(4):680-688 (2006)).

In certain embodiments, it may be desirable to create cysteine engineered antibodies, e.g., "thioMAbs," in which one or more residues of an antibody are substituted with cysteine residues. In particular embodiments, the substituted residues occur at accessible sites of the antibody. By substituting those residues with cysteine, reactive thiol groups are thereby positioned at accessible sites of the antibody and may be used to conjugate the antibody to other moieties, such as drug moieties or linker-drug moieties, to create an immunoconjugate, as described further herein. In certain embodiments, any one or more of the following residues may be substituted with cysteine: V205 (Kabat numbering) of the light chain; A118 (EU numbering) of the heavy chain; and S400 (EU numbering) of the heavy chain Fc region. Cysteine engineered antibodies may be generated as described, e.g., in U.S. Patent No. 7,521,541.

The term "variants" also includes immunoconjugates comprising an antibody as defined above conjugated to one or more heterologous molecule(s), including but not limited to a cytotoxic agent, a detectable moiety such as a fluorescent moiety, a diagnostic radioisotope or an imaging agent; or to a solid support, such as agarose beads or the like. Examples of cytotoxic agents include, but are not limited to chemotherapeutic agents or drugs, growth inhibitory agents, toxins (e.g., protein toxins, enzymatically active toxins of bacterial, fungal, plant, or animal origin, or fragments thereof), or radioactive isotopes. Conjugates of an antibody and cytotoxic agent may be made using a variety of bifunctional protein coupling agents well known by the skilled person. The linker may be a "cleavable linker" facilitating release of a cytotoxic drug in the cell. For example, an acid-labile linker, peptidase-sensitive linker, photolabile linker, dimethyl linker or disulfide-containing linker (Chari et al., Cancer Res. 52: 127-131 (1992)) may be used. Derivatives may also be bispecific antibodies. Particular variants are labelled antibodies, carrying a label (such as a marker, tracer, tag) allowing detection or capture of an antibody. Examples of such variants include fusion molecules formed by covalent or noncovalent association of the antibody with one or more proteins or peptides, such as a reporter peptide allowing the detection, or the quantification of the fusion molecule. In some embodiments, antibody variants of the invention can be engineered, such as biotinylated, to be detectable and/or quantifiable. Variants also include humanized antibodies.

In preferred embodiments, antibodies and fragments and variants of the invention are isolated. An "isolated" antibody is one which has been separated from a component of its natural environment. In particular, the antibody may be purified to greater than 95% or 99% purity as determined by, for example, electrophoretic (e.g., SDS-PAGE, isoelectric focusing (IEF), capillary electrophoresis) or chromatographic (e.g., ion exchange or reverse phase HPLC). For review of methods for assessment of antibody purity, see, e.g. Flatman et al., J. Chromatogr. B 848:79-87 (2007).

An antibody is "selective" for an antigen, or "selectively binds" an antigen when such antibody exhibits preferential binding to said antigen as compared to other molecules. Selectivity can be determined by competitive ELISA or Biacore assays. The difference in affinity/avidity that marks selectivity can be any detectable preference, such as e.g., a ratio of more than 1:1.1, or more than about 1:5, 1:10, 1:100, 1:1000 or more.

"gC1qR" or "gC1q receptor" designates the receptor for complement C1q at the surface of cells, particularly of CD4 T cells, especially the human form of said receptor. gClqR is also known as C1q binding protein (C1QBP), ASF/SF2-associated protein p32 (SF2P32); Glycoprotein gClqBP; Hyaluronan-binding protein 1 (HABP1); Mitochondrial matrix protein p32; gC1q-R protein; p33; ClqBP and GC1QBP. The amino acid sequence of the receptor was disclosed in the art. An exemplary amino acid sequence of human gClqR is depicted as SEQ ID NO: 65.

The term gClqR designates any receptor of SEQ ID NO: 65 (accession number UniProtKB/Swiss-Prot: Q07021.1) above, as well as processed forms and variants thereof. Variants include naturally-occurring variants having e.g., at least 90% amino acid sequence identity to SEQ ID NO: 65.

Applicant has previously documented that gp41 binds GclqR, which triggers a signaling pathway that results in exocytosis of intracellular vesicles. Inhibiting binding of gp41 to gClqR designates any level of inhibition, preferably at least 10%, at least 20%, at least 30%, at least 40%, or at least 50% reduction of such binding. Such activity can be verified in vitro using methods known per se in the art.

### Anti-PEP3 antibodies

In a particular aspect, the invention relates to anti-gp41 antibodies, variants or fragments thereof. Such antibodies were generated by immunization with a PEP3 peptide and subsequently selected based on their capacity to bind gp41 and/or the PEP3 domain. The antibodies were further selected for their capacity to block gp41 binding to ClqR, and/or to effectively quantify reactive gp41 in biological samples. The amino acid sequences of the heavy and light chains of antibodies of the invention were determined, allowing recombinant production thereof and generation of variants thereof Upon testing, it was confirmed these antibodies are able to bind gp41 and/or PEP3. It was also found antibodies of the invention effectively inhibit the inhibitory effect of HIV viremic plasma on CD4 T cells. These antibodies thus exhibit remarkable properties for therapeutic and diagnostic uses.

The amino acid sequences of the light and heavy chains variable regions of these antibodies are provided in SEQ ID NOs: 3-14 (see also the FIG). The CDR domains of each variable region are also identified and provided as SEQ ID Nos: 15-50.

In a particular aspect, the invention relates to a gp41-binding antibody comprising a CDR region consisting, or consisting essentially, of a sequence selected from anyone of SEQ ID Nos: 15-50.

In a further particular embodiment, the invention relates to a monoclonal antibody comprising a light chain variable region, wherein the light chain variable region comprises (i) a CDR-L1 consisting, or consisting essentially, of a selected from SEQ ID NOs: 15, 21, 27, 33, 39, or 45, and/or (ii) a CDR-L2 consisting, or consisting essentially, of a selected from SEQ ID NOs: 16, 22, 28, 34, 40, or 46, and/or (iii) a CDR-L3 consisting, or consisting essentially, of a selected from SEQ ID NOs: 17, 23, 29, 35, 41, or 47.

In a more specific embodiment, the light chain variable region comprises a CDR-L1, a CDR-L2 and a CDR-L3 as follows:
• SEQ ID NOs: 15, 16 and 17; or
• SEQ ID NOs: 21, 22 and 23, or
• SEQ ID NOs: 27, 28, and 29, or
• SEQ ID NOs: 33, 34 and 35, or
• SEQ ID NOs: 39, 40 and 41, or
• SEQ ID NOs: 45,46 and 47.

In a further particular embodiment, the invention relates to a monoclonal antibody comprising a heavy chain variable region, wherein the heavy chain variable region comprises (i) a CDR-H1 consisting, or consisting essentially, of a selected from SEQ ID NOs: 18, 24, 30, 36, 42, or 48, and/or (ii) a CDR-H2 consisting, or consisting essentially, of a selected from SEQ ID NOs: 19, 25, 31, 37, 43, or 49, and/or (iii) a CDR-H3 consisting, or consisting essentially, of a selected from SEQ ID NOs: 20, 26, 32, 38, 44, or 50.

In a more specific embodiment, the heavy chain variable region comprises a CDR-H1, a CDR-H2 and a CDR-H3 as follows:
• SEQ ID NOs: 18, 19 and 20; or
• SEQ ID NOs: 24, 25 and 26, or
• SEQ ID NOs: 30,31, and 32, or
• SEQ ID NOs: 36, 37 and 38, or
• SEQ ID NOs: 42, 43 and 44, or
• SEQ ID NOs: 48, 49 and 50.

In a further particular embodiment, the invention relates to a monoclonal antibody comprising a light and a heavy chain variable region as defined above. More specifically, particular monoclonal antibodies of the invention comprise:
• a light chain variable region, wherein the light chain variable region comprises
   (i) a CDR-L1 selected from SEQ ID NOs: 15, 21, 27, 33, 39, or 45, and/or (ii) a CDR-L2 selected from SEQ ID NOs: 16, 22, 28, 34, 40, or 46, and/or (iii) a CDR-L3 selected from SEQ ID NOs: 17, 23, 29, 35, 41, or 47, and
• a heavy chain variable region, wherein the heavy chain variable region comprises
   (i) a CDR-H1 selected from SEQ ID NOs: 18,24, 30, 36,42, or 48, and/or (ii) a CDR-H2 selected from SEQ ID NOs: 19, 25, 31, 37, 43, or 49, and/or (iii) a CDR-H3 selected from SEQ ID NOs: 20, 26, 32, 38, 44, or 50.

In a more particular embodiment, the invention relates to a monoclonal antibody comprising the light chain variable region consisting, or consisting essentially, of a sequence selected from SEQ ID NOs: 3, 5, 7, 9, 11 and 13 and/or a heavy chain variable region consisting, or consisting essentially, of a sequence selected from SEQ ID NOs: 4, 6, 8, 10, 12, and 14.

In a more particular embodiment, the invention relates to a monoclonal antibody csomprising a light chain variable region and a heavy chain variable region as follows:
• SEQ ID NOs: 3 and 4; or
• SEQ ID NOs: 5 and 6; or
• SEQ ID NOs: 7 and 8; or
• SEQ ID NOs: 9 and 10; or
• SEQ ID NOs: 11 and 12; or
• SEQ ID NOs: 13 and 14.

Specific examples of antibodies of the invention are antibodies 4G1, 8D5, 3A2, 15B4, 5E12, and 9B12.

As discussed above and illustrated in the examples, antibodies of the invention bind gp41 and/or PEP3, and/or inhibit binding of gp41 to gClqR, and/or inhibit the inhibitory effect of HIV viremic plasma on CD4 T cells.

In another embodiment, the invention relates to an anti-gp41 antibody that binds an epitope of gp41 comprising one or more amino acid residues of SEQ ID NO: 63 or 64.

### Nucleic acids, vectors and host cells

The invention also relates to nucleic acids encoding an antibody or fragment as defined above, as well as cloning or expression vectors containing such nucleic acids, and recombinant host cells.

In another aspect, the present invention concerns nucleic acid molecules encoding an antibody of the invention, or a nucleic acid complementary to said encoding sequence. Preferably, the nucleic acid is an isolated or purified nucleic acid.

The nucleic acid can be DNA (cDNA or gDNA), RNA, or a mixture thereof. It can be in single stranded form or in duplex form or a mixture of the two. It can comprise modified nucleotides, comprising for example a modified bond, a modified purine or pyrimidine base, or a modified sugar. It can be prepared by any method known to one skilled in the art, including chemical synthesis, recombination, and mutagenesis. The nucleic acid according to the invention may be deduced from the sequence of the antibody according to the invention and codon usage may be adapted according to the host cell in which the nucleic acid shall be transcribed. These steps may be carried out according to methods well known to one of skill in the art and some of which are described in the reference manual Sambrook et al. (Sambrook J, Russell D (2001) Molecular cloning: a laboratory manual, Third Edition Cold Spring Harbor).

The nucleic acid of the invention may encode an amino acid sequence comprising the light chain and/or an amino acid sequence comprising the heavy chain of the antibody, or may be complementary to such encoding sequence.

Specific examples of such nucleic acid sequences include the sequences comprising anyone of SEQ ID NOs: 51-62, and the complementary sequence thereto.
The present invention further provides a vector comprising a nucleic acid of the invention. Optionally, the vector may comprise several nucleic acids of the invention. In particular, the vector may comprise a nucleic acid of the invention operably linked to a regulatory region, i.e. a region comprising one or more control sequences. Optionally, the vector may comprise several nucleic acids of the invention operably linked to several regulatory regions.

The term "control sequences" means nucleic acid sequences necessary for expression of a coding region. Control sequences may be endogenous or heterologous. Well-known control sequences and currently used by the person skilled in the art will be preferred. Such control sequences include, but are not limited to, promoter, signal peptide sequence and transcription terminator.

The term "operably linked" means a configuration in which a control sequence is placed at an appropriate position relative to a coding sequence, in such a way that the control sequence directs expression of the coding region.

The present invention further relates to the use of a nucleic acid or vector according to the invention to transform, transfect or transduce a host cell.

The present invention also provides a host cell comprising one or several nucleic acids of the invention and/or one or several vectors of the invention.

The term "host cell" also encompasses any progeny of a parent host cell that is not identical to the parent host cell due to mutations that occur during replication.
Suitable host cells for cloning or expression of antibody-encoding vectors include prokaryotic or eukaryotic cells described herein.

For example, antibodies may be produced in bacteria, in particular when glycosylation and Fc effector function are not needed. For expression of antibody fragments and polypeptides in bacteria, see, e.g., U.S. Patent Nos. 5,648,237, 5,789,199, and 5,840,523. (See also Charlton, Methods in Molecular Biology, Vol. 248 (B.K.C. Lo, ed., Humana Press, Totowa, NJ, 2003), pp. 245-254, describing expression of antibody fragments in E. coli.) After expression, the antibody may be isolated from the bacterial cell lysate in a soluble fraction and can be further purified.
In addition to prokaryotes, eukaryotic microbes such as filamentous fungi or yeast are suitable cloning or expression hosts for antibody-encoding vectors, including fungi and yeast strains whose glycosylation pathways have been "humanized," resulting in the production of an antibody with a partially or fully human glycosylation pattern. See Gerngross, Nat. Biotech. 22: 1409-1414 (2004), and Li et al., Nat. Biotech. 24:210-215 (2006).
Suitable host cells for the expression of glycosylated antibody are also derived from multicellular organisms (invertebrates and vertebrates). Examples of invertebrate cells include plant and insect cells. Numerous baculoviral strains have been identified which may be used in conjunction with insect cells, particularly for transfection of Spodoptera frugiperda cells. Plant cell cultures can also be utilized as hosts. See, e.g., US Patent Nos. 5,959,177, 6,040,498, 6,420,548, 7,125,978, and 6,417,429.
Vertebrate cells may also be used as hosts. For example, mammalian cell lines that are adapted to grow in suspension may be useful. Other examples of useful mammalian host cell lines are monkey kidney CV1 line transformed by SV40 (COS-7); human embryonic kidney line (293 or 293 cells as described, e.g., in Graham et al., J. Gen Virol. 36:59 (1977)); baby hamster kidney cells (BHK); mouse Sertoli cells (TM4 cells as described, e.g., in Mather, Biol. Reprod. 23:243-251 (1980)); monkey kidney cells (CV1); African green monkey kidney cells (VERO- 76); human cervical carcinoma cells (HELA); canine kidney cells (MDCK; buffalo rat liver cells (BRL 3A); human lung cells (W138); human liver cells (Hep G2); mouse mammary tumor (MMT 060562); TRI cells, as described, e.g., in Mather et al., Annals N. Y. Acad. Sci. 383:44-68 (1982); MRC 5 cells; and FS4 cells. Other useful mammalian host cell lines include Chinese hamster ovary (CHO) cells, including DHFR⁻ CHO cells (Urlaub et al., Proc. Natl. Acad. Sci. USA 77:4216 (1980)); and myeloma cell lines such as Y0, NS0 and Sp2/0. For a review of certain mammalian host cell lines suitable for antibody production, see, e.g., Yazaki and Wu, Methods in Molecular Biology, Vol. 248 (B.K.C. Lo, ed., Humana Press, Totowa, NJ), pp. 255- 268 (2003).
The present invention also concerns a method for producing an antibody of the invention, comprising culturing a host cell comprising a nucleic acid of the invention or a vector of the invention, as provided above, under conditions suitable for expression of the antibody, and optionally recovering the antibody from the host cell or from the host cell culture medium. Optionally, the recovered antibody may be further purified or isolated. Suitable media, culture conditions and production method are well-known by the skilled person and can be easily chosen according to the host cell and the antibody to be produced.

### Pharmaceutical and diagnostic compositions

The present invention further relates to pharmaceutical or diagnostic compositions comprising an antibody, a nucleic acid, a vector or a host cell of the invention. The composition may comprise one or several antibodies of the invention, one or several nucleic acids of the invention and/or one or several vectors of the invention and/or one or several host cells of the invention. Preferably, the composition comprises one or several antibodies of the invention.

Pharmaceutical compositions comprise an antibody of the invention and any suitable excipient. Typically, the antibody has the desired degree of purity and is mixed with optional physiologically acceptable carriers, vehicle or stabilizers (Remington: The Science and Practice of Pharmacy 20th edition (2000)), in the form of e.g., aqueous solutions, lyophilized or other dried formulations.

Acceptable carriers, excipients, or stabilizers are nontoxic to recipients at the dosages and concentrations employed, and include, but are not limited to, buffering agents, stabilizing agents, preservatives, isotonifiers, non-ionic detergents, antioxidants and other miscellaneous additives.

Buffering agents help to maintain the pH in the range which approximates physiological conditions. They are preferably present at concentration ranging from about 1 mM to about 50 mM. Suitable buffering agents for use with the present invention include, but are not limited to, both organic and inorganic acids and salts thereof such as citrate, succinate, tartrate, fumarate, gluconate, oxalate, lactate and acetate buffers, as well as phosphate buffers, histidine buffers and trimethylamine salts such as Tris. Preservatives may be added to retard microbial growth, and may be added in amounts ranging from 0.2%-1% (w/v). Suitable preservatives for use with the present invention include, but are not limited to, phenol, butyl or benzyl alcohol; meta-cresol; alkyl parabens such as methyl or propyl paraben; octadecyldimethylbenzyl ammonium chloride, benzalkonium halides (e.g., chloride, bromide, iodide); hexamethonium or benzethonium chloride; catechol; resorcinol; cyclohexanol; and 3-pentanol.

Isotonifiers may be added to ensure isotonicity of liquid compositions of the present invention and include polhydric sugar alcohols, preferably trihydric or higher sugar alcohols, such as glycerin, erythritol, arabitol, xylitol, sorbitol and mannitol.

Stabilizing agents refer to a broad category of excipients which can range in function from a bulking agent to an additive which solubilizes the therapeutic agent or helps to prevent denaturation or adherence to the container wall. Typical stabilizers can be polyhydric sugar alcohols (enumerated above); amino acids such as arginine, lysine, glycine, glutamine, asparagine, histidine, alanine, ornithine, L-leucine, 2-phenylalanine, glutamic acid, threonine, etc., organic sugars or sugar alcohols, such as lactose, trehalose, stachyose, mannitol, sorbitol, xylitol, ribitol, myoinisitol, galactitol, glycerol and the like, including cyclitols such as inositol; polyethylene glycol; amino acid polymers; sulfur containing reducing agents, such as urea, glutathione, thioctic acid, sodium thioglycolate, thioglycerol, .alpha.-monothioglycerol and sodium thio sulfate; low molecular weight polypeptides (i.e. <10 residues); proteins such as human serum albumin, bovine serum albumin, gelatin or immunoglobulins; hydrophylic polymers, such as polyvinylpyrrolidone monosaccharides, such as xylose, mannose, fructose, glucose; disaccharides such as lactose, maltose, sucrose and trisaccacharides such as raffinose; polysaccharides such as dextran. Stabilizers may be present in the range from 0.1 to 10,000 weights per part of weight therapeutic agent.

Non-ionic surfactants or detergents (also known as "wetting agents") may be added to help solubilize the therapeutic agent as well as to protect the therapeutic protein against agitation-induced aggregation. Suitable non-ionic surfactants include, but are not limited to, polysorbates (20, 80, etc.), polyoxamers (184, 188 etc.), PLURONICS™, polyols, polyoxyethylene sorbitan monoethers (TWEEN™-20, TWEEN™-80, etc.). Non-ionic surfactants may be present in a range of about 0.05 mg/ml to about 1.0 mg/ml, preferably about 0.07 mg/ml to about 0.2 mg/ml.

Additional miscellaneous excipients include, but are not limited to, bulking agents, (e.g. starch), chelating agents (e.g. EDTA), antioxidants (e.g., ascorbic acid, methionine, vitamin E), and cosolvents.

The active ingredients may also be entrapped in microcapsule prepared, for example, by coacervation techniques or by interfacial polymerization, for example, hydroxymethylcellulose or gelatin-microcapsule and poly-(methylmethacylate) microcapsule, respectively, in colloidal drug delivery systems (for example, liposomes, albumin micropheres, microemulsions, nano-particles and nanocapsules) or in macroemulsions. Such techniques are disclosed in Remington: The Science and Practice of Pharmacy 20th edition (2000).
The pharmaceutical compositions of the invention can be formulated for a topical, oral, parenteral, intranasal, intravenous, intramuscular, subcutaneous or intraocular administration and the like.

Preferably, the pharmaceutical formulation is a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions.

Sterile injectable solutions may be prepared by incorporating the active compounds in the required amount in the appropriate solvent with various of the other ingredients enumerated above, as required, followed by filtered sterilization. In the case of sterile powders for the preparation of sterile injectable solutions, the preferred methods of preparation are vacuum-drying and freeze-drying techniques which yield a powder of the active ingredient plus any additional desired ingredient from a previously sterile- filtered solution thereof.

In addition to the compositions formulated for parenteral administration, such as intravenous or intramuscular injection, other pharmaceutically acceptable forms include, e.g. tablets or other solids for oral administration; time release capsules; and any other form currently used.

The pharmaceutical composition of the invention may comprise one or several antibodies of the invention.

The pharmaceutical composition may further comprise one or several additional active compounds. Examples of additional active compounds include, but are not limited to, chemotherapeutic drug, antibiotics, antiparasitic agents, antifungal agents or antiviral agents.

The amount of antibody of the invention which will be effective in the treatment of a particular disorder or condition will depend on the nature of the disorder or condition, and can be determined by standard clinical techniques. In a preferred embodiment, each dose may range from about 0.1 mg to about 25 mg per kilogram of body weight of antibody, or more preferably, from about 1 mg to about 10 mg per kilogram body weight. The dosing schedule for administration may vary form once a month to daily depending on a number of clinical factors, including the type of disease, severity of disease, and the subject's sensitivity to the therapeutic agent.

### Methods of detection, dosage, diagnosis

In a further aspect, the present invention relates to a method for detecting or quantifying gp41 using an antibody or fragment or variant as defined above. The method can be used or implemented for monitoring the severity or progression of HIV infection in a subject, as well as for predicting the outcome of such infection or determining the optimal treatment.

In a particular embodiment, the method comprises a) providing a sample, and b) detecting the presence or amount of gp41protein in the sample using an antibody or fragment as defined above.

Typically, the detecting step b) comprises the steps of (i) contacting the sample, or biological replicates of the sample, with anyone of the monoclonal antibodies or fragments or variants thereof herein described, and (ii) detecting the presence of an antibody : antigen complex formed in step (i).

Detecting the presence of an antibody: antigen complex can be performed by any technique well known per se by the skilled person and extensively described in the literature. Typically, detection can be performed with a second antibody against gp41, preferably that binds an epitope distinct (and remote) from that of the first capture antibody. Alternatively, the capture antibody may be labelled and the detection step may comprise measuring the amount of label in the reaction, typically after removing unbound antibodies.

The antibody may be immobilized on a surface or particle, or not. The reaction can be performed in any appropriate support or container, such as microplate, on a chip, in a bottle, etc. Also, in a particular embodiment, the detecting step (ii) utilizes a labelled antibody, such as an antibody conjugated to a reporting group or engineered to be detectable, more preferentially said antibody is biotinylated.

The method may be used with any sample such as any biological sample, such as a serum sample, a plasma sample, whole blood, biological fluids, etc. In more particular embodiments, the sample used is obtained from a subject. The sample may be treated prior to detection, such as by dilution, concentration, enrichment, etc. It may be lyophilized, sterilized, frozen, etc., before use in a method of the invention.

In a particular embodiment, the invention thus relates to a method for detecting a HIV gp41 protein in a sample, comprising:
(i) contacting the sample with a monoclonal antibody, variant or fragment of the invention, and
(ii) detecting the presence of an antibody: antigen complex formed in step (i).

The invention is particularly relevant since the antibodies of the invention can detect or quantify the relevant (or reactive) form of gp41. Indeed, by targeting the PEP3 domain, essentially reactive gp41 protein is measured, which thus provides a relevant information about the disease/patient status.

The invention thus relates to a method for monitoring progression of HIV disease in a subject in need thereof, comprising in vitro quantifying gp41 at different time intervals in a plasma sample from the subject using a monoclonal antibody, variant or fragment of the invention.

The method of the invention may be used e.g., for monitoring anti-HIV treatment efficacy.

The method of the invention may also be used for determining if an HIV-infected subject can benefit from anti-PLA2G1B immunotherapy, preferably anti-PLA2G1B antibody therapy or trans CD4 immunotherapy.

The method of the invention can also be used for determining the severity of HIV disease in a subject, wherein an increased level of binding of the monoclonal antibody, variant or fragment of the invention in said sample as compared to a reference value is indicative of severity. For use in the invention, the reference value may be a value determined at an earlier stage in a sample from the same subject, or a mean value.

The invention mays also be used to monitor/follow the amount of gp41 (fragment) in the plasma of HIV-infected patients treated with antiretroviral molecules and correlate the gp41 levels with PLA2GIB activity and outcome of the disease.

In a further aspect, the present invention relates to kits comprising a least one antibody, or a variant or fragment thereof, as herein described, in a container (such as a plate, microplate, tube, bottle, flask, etc. The kit of the invention preferably further comprise one or more reagent(s) to perform, detect or quantify an immune reaction, or an antibody-antigen complex. The one or more reagents may be selected from e.g., diluents, substrates, labels, marker antibodies, etc., adapted to any immune reaction such as ELISA, RIA, etc.

### Method of treating HIV

The antibodies and compositions of the invention may further be used for treating HIV disease in a subject in need thereof.

In a further aspect, the invention relates to methods for treating HIV comprising administering to a subject in need thereof a composition or antibody, fragment or variant thereof as defined above.
In a further aspect, the invention relates to a composition or antibody, fragment or variant thereof as defined above for use for treating HIV disease.

As used herein, "treatment" or "treat" refers to clinical intervention in an attempt to alter the natural course of the individual being treated, and can be performed either for preventive or curative purpose. Desirable effects of treatment include, but are not limited to, preventing occurrence or recurrence of disease, alleviation of symptoms, diminishment of any direct or indirect pathological consequences of the disease, reducing viral load or replication or virulence, decreasing the rate of disease progression, amelioration or palliation of the disease state, and remission or improved prognosis. In some embodiments, compositions and methods of the invention are used to delay development of a disease or disorder or to slow the progression of a disease or disorder.

The duration, dosages and frequency of administering compounds or compositions of the invention may be adapted according to the subject and disease. The treatment may be used alone or in combination with other active ingredients, either simultaneously or separately or sequentially.

Further aspects and advantages of the invention are disclosed in the following experimental section.

### Examples

### MATERIALS AND METHODS

*Recombinant proteins and peptides-* Human PLA2-GIB was produced in E. coli (gift Gerard Lambeau, purity >98%) or in CHO-S (purity >98%). HIV-1 MN gp41 (Genbank accession #AAC31817.1) recombinant protein was produced in E. Coli and was obtained from Antibodies onlines (MN gp41 (565-771Delta642-725), ABIN2129703, lot 93-482, purity >95%,). HXB2 gp41 recombinant protein (GenBank accession #AAB50262.1, a.a. 534-681, purity >95%) was purified from 293 cell culture and purchased from antibodies online (ABIN1605865). HIV-1 MN gp41 (GenBank accession #AAC31817.1, a.a. 542-666), D117 III gp41 (GenBank accession #AAM08364.1, a.a. 532-656) (see alignment of recombinant proteins used, slide Methods), and D117 III gp120 (Genbank accession #AAM08364.1, a.a. 31-482) were produced in S2 cells and purified at DIACCURATE (purity > 98%). PEP3 peptide NH2-PWNASWSNKSLDDIW-COOH and control peptide (CTL) NH2-PWNATWTQRTLDDIW-COOH were ordered from Covalab (purity >98%).

*Development of anti-gp4l antibodies-* Antibodies specific for the PEP3 sequence (PWNASWSNKSLDDIW) in HIV-1 MN strain gp41 were generated at BIOTEM (France) by immunization of OF1 mice with the peptide NH2-C-PWNASWSNKSLDDIW-COOH conjugated to KLH (KLH-PEP3). Mice were immunized 5 times every 3 weeks. First immunization was subcutaneous and intraperitoneal with 50 µg of peptides and complete freund's adjuvant. Then mice were intraperitoneally injected three times with 50 µg of peptides and incomplete freund's adjuvant. The antibody titer in serum was evaluated by ELISA on PEP3 and MN gp41 protein (ABIN2129703). The mouse with the best titer on protein and peptide was selected and injected intravenously with 5 µg of KLH-PEP3 alone. Three days later the spleen was collected and cells were fused with myeloma (Sp2/0-Ag14) cells to generate hybridoma. Among 1093 supernatants from fusion 1072 were positive on BSA-PEP3 peptide. The best 123 were selected and tested another time on BSA-PEP3 peptide and MN gp41. The isotypes of 27 clones were determined and they were frozen including 6 clones that were maintained in cell culture to obtain stable clone (Table 1). These 6 clones, 4G1, 8D5, 3A2, 15B4, 5E12 and 9B12, were seeded in High Yielding Performance Flask (hyperflask) for cell culture to produce 560 ml of supernatant with 10% Low IgG fetal bovine serum (FBS) in DMEM medium. IgG were purified from supernatant by affinity chromatography on protein A in low endotoxin condition (<10 EU / mg, LAL test). Antibodies binding to BSA-PEP3, MN gp41 and HXB2 gp41 was studied as indicated in the patent and the cDNA sequence coding the variable regions of these antibodies was determined (see below).
Anti-HR2 antibodies specific for the sequence 5184-3 (EKSQTQQEKNEQELLELDK, a.a. 648-666, into HR2 region) in HIV-1 MN strain gp41 were generated at BIOTEM (France) by immunization of OF1 mice with the peptide NH2-C-EKSQTQQEKNEQELLELDK-COOH conjugated to KLH (KLH-5184-3). 3 mice were immunized 5 times with 3 weeks between the first 3 immunizations and immunizations #4 and #5 but 4 months between immunizations #3 and #4. First immunization was subcutaneous and intraperitoneal with 20 µg (S11 and S12 mice) or 50 µg (S13 mouse) of peptides and complete freund's adjuvant. The second immunization was intraperitoneal with 20 µg (S11 and S12 mice) or 50 µg (S13 mouse) of peptides and incomplete freund's adjuvant. Third and fourth injection were intraperitoneal with 10 µg of peptides and incomplete freund's adjuvant. The antibody titer in serum was evaluated by ELISA on 5184-3 peptide, MN gp41 and HXB2 gp41. Mice S12 and S13 had the best titers. Thus they were both intravenously injected with 2.5 µg of both MN gp41 and HXB2 gp41. Three days later the spleen was collected and cells were fused with myeloma (Sp2/0-Ag14) cells to generate hybridoma. Among 515 supernatants from fusion of S12 mouse cells, 3 were positive on BSA-5184-3 peptide by ELISA and confirmed by ELISA on BSA-5184-3, MN gp41 (produced in S2 cells) and HXB2 gp41 proteins (63G4, 69D6 and 67D7). Among 301 supernatants from fusion of S13 mouse cells, 1 was positive on BSA-5184-3 peptide by ELISA and confirmed by ELISA on BSA-5184-3, MN gp41 and HXB2 gp41 proteins (72D7). We obtained 4 clones that produced anti-HR2 Ab (anti-5184-3). They were frozen and the isotype was determined.

*ELISA-* ELISA on peptides or proteins were performed on microtiter plates coated overnight at room temperature (RT) with 50 µl of a solution in PBS (pH7.4) of BSA-PEP3, MN gp41 protein (screening of anti-PEP3 Ab, Figures 5A-C) or BSA-5184-3, MN gp41 and HXB2 gp41 proteins (screening of anti-HR2 Ab, Figures 17, 18B, 18C). After washing, wells were then blocked 1h at RT with 150 µl of PBS/2.5% skimmed milk (screening of anti-PEP3 Ab) or PBS/1%BSA (screening of anti-HR2 Ab). After washing, various dilutions in PBS/0.05%Tween 20/0.5%BSA of Ab from 100-0.0001 µg/ml were incubated for 2h at RT in 50 µl. Wells were washed and antibody binding was revealed after 1h of incubation at RT in 50 µl of PBS/0.05%Tween 20/0.5%BSA with HRP-conjugated affiniPure F(ab)'2 fragment Goat anti-Mouse IgG (1:5000, Jackson 115-036-008) for unbiotinylated Ab or HRP-Streptavidin HRP (1:1000) for biotinylated Ab followed by TMB addition (10 min at RT, SureBlue™ TMB Microwell Peroxidase Substrate Kit, KPL 52-00-01, VWR).

ELISA with anti-PEP3 mAb on coated MN gp41 (produced in S2 cells) or HXB2 gp41 (Figures 5D-F) were performed on microtiter plates coated overnight at 4°C with 100 µl of a solution at 1 µg/ml of protein recombinant in carbonate buffer pH 9.6. Nonspecific binding sites were blocked with 300 µl of Pierce blocking buffer (37572, Pierce). After washing, various dilutions in Pierce blocking buffer of Ab from (100-0.0001 µg/ml on MN gp41; 100-10⁻¹⁰ µg/ml on HXB2 gp41) were incubated in quadruplicate for 2h at RT in 100 µ1. Bound antibody were then detected by interaction with a goat-anti mouse HRP-conjugated antibody, followed by the addition of 100 µl/well TMB substrate (UP664781, Interchim).

To further study anti-PEP3 mAbs their pairing with biotinylated 4G1 or biotinylated goat anti-gp41 pAb (PA1-73100, Thermo fisher Scientific) were tested by sandwich ELISA (Figures 15A-E). Microtiter plates were coated with 100 µl of dilution in carbonate buffer pH 9.6 of capture Ab (4G1, 3A2, 8D5 or 15B4) at 10 µg/ml. Nonspecific binding sites were blocked with 300 µl of Pierce blocking buffer (37572, Pierce). After washing, various dilutions of untagged MN gp41 (0.5-0.00391 µg/ml, produced in S2 cells) or HXB2 gp41 (10-10⁻⁶ µg/ml) in Pierce blocking buffer of Ab were incubated in quadruplicate for 2h at RT in 100 µl. Untagged MN gp41 protein was obtained upon digestion by Enterokinase (P8070, NEB). MN gp41 at 100µg/ml in PBS containing 2µM of CaC12 was digested by adding Enterokinase at 1:300 for 16h at 37°C. Detection Ab were added in 100 µl of Pierce blocking buffer at 5 µg/ml for biotinylated goat anti-gp41 pAb or 20 µg/ml for biotinylated 4G1 Ab. Bound antibody were then detected by interaction with HRP-streptavidin (1:20,000, UP395888, Interchim Uptima) followed by the addition of 100 µl/well TMB substrate (UP664781, Interchim). To test the feasibility of sandwich ELISA between 4G1 and anti-HR2 Ab (anti-5184-3 Ab), the 4 anti-HR2 Hybridoma were injected in nude mice to produce Ab in ascites fluid. 2 ml of ascites fluid were collected and antibodies were purified by affinity chromatography on protein A. When the amount of antibody was sufficient they were biotinylated (67D7 and 72D7). 4G1 was also biotinylated. Antibodies biotinylated or not were first tested on microtiter plates coated with BSA-5184-3 peptide at 1µg/ml (all anti-HR2 Ab), PEP3 at 2 µg/ml (4G1 and 4G1-biot) or HXB2 at 1 µg/ml (All antibodies) in 50 µl of PBS (pH7.4) to validate their reactivity on HXB2 and 5184-3-peptide. Then the pairing between the different anti-HR2 or 4G1 as capture antibodies and the biotinylated Ab (67D7-biot, 72D7-biot and 4G1-biot) as detection Ab was tested by coating 50 µl of dilution in PBS of the capture Ab 4G1, 63G4, 69D6 and 72D7 at 10 µg/ml or 67D7 at 20 µg/ml (Figures 19A-E). After blocking with PBS/1%BSA for 1h at RT, various concentrations of HXB2 gp41 (0,0.1, 1 and 10 µg/ml, in 50 µl of PBS) were incubated for 2h at RT. 50 µl of detection Ab diluted in PBS/0.05%Tween 20/0.5%BSA were then incubated for 1h at RT and sandwich were revealed with HRP-streptavidin (1:1000, 1h at RT) and TMB (10 min at RT, SureBlue™ TMB Microwell Peroxidase Substrate Kit, KPL 52-00-01, VWR).

To further study the pairing of 72D7 with 4G1 or goat anti-g41 pAb, 72D7 was tested as capture Ab and the biotinylated 4G1 or anti-gp41 pAb were tested as detection Ab (Figure 20). Microtiter plates were coated with 100 µl of dilution in carbonate buffer pH 9.6 of 72D7 at 10 µg/ml. Nonspecific binding sites were blocked with 300 µl of Pierce blocking buffer (37572, Pierce). After washing, various dilutions of HXB2 gp41 (10-10⁻⁶ µg/ml) in Pierce blocking buffer of Ab were incubated in quadruplicate for 2h at RT in 100 µ1. Detection Ab were added in 100 µl of Pierce blocking buffer at 5 µg/ml for biotinylated goat anti-gp41 pAb or 20 µg/ml for biotinylated 4G1 Ab. Bound antibody were then detected by interaction with HRP-streptavidin (1:20,000, UP395888, Interchim Uptima) followed by the addition of 100 µl/well TMB substrate (UP664781, Interchim).

*Sequencing and analysis of cDNA coding variable region of the 6 anti-PEP3 mAbs-* RNA was extracted from cloned hybridoma and reverse transcribed into cDNA at BIOTEM (France) with high fidelity reverse transcriptase. The cDNA was amplified by PCR with high fidelity Taq polymerase and degenerated primers flanking variable regions of light and heavy chains. Both strands of the bulk of PCR products were directly sequenced. Sequences were aligned to obtain sequences coding the variable domains of light (VL) and heavy (VH) chains with partial sequences of the constant domains. Based on these nucleotide sequences, protein sequences were deduced and isotypes of light and heavy chains were confirmed. Using the IMGT web site, (the international ImMunoGeneTics information system® http://www.imgt.org) the percentage of identity between anti-PEP3 mAb VL and VH sequences and mouse germinal sequences was determined, the functional structure subdomains FR1-FR4 and CDR1-3 and a model of representation of 3D structures in colliers de Perles (IMGT Colliers de Perles) were predicted.

*Characterization of monoclonal 4G1 anti-gp41 specificity on gp41 and gp120 recombinant proteins-* Recombinant MN gp41, D117 III gp41 and D117 III gp120 were produced in S2 cells. Microtiter plates were coated in triplicate with 10 µg/well of either recombinant D117 III gp120 or recombinant D117 III gp41 in carbonate buffer pH 9.6. After overnight incubation at 4°C, nonspecific binding sites were blocked with Pierce blocking buffer (37572, Pierce). Anti-PEP3 mAb 4G1 was added to a final dilution of 1 µg/well in blocking buffer. Bound antibody was detected by interaction with a goat-anti mouse HRP-conjugated antibody, followed by the addition of 100 µ1/well TMB substrate (UP664781, Interchim). The reaction was stopped by adding 50 µl 0.1 M H₂SO₄ and the O.D. ₄₅₀ₙₘ was measured using a Tecan Infinite M1000 Pro.
For western blot analysis, 500 ng or 1 µg of recombinant MN gp41 or D117 III gp120 proteins were run on 4%-20% Tris-Bis SDS-PAGE (BIO-RAD) gels under reducing conditions. Antigens were transferred to PVDF membranes (BIO-RAD) using a Trans-Blot Turbo (BIO-RAD). After blocking nonspecific binding sites with 5% skimmed milk/0.05%tween 20 in PBS, antibody binding was tested at 1µg/ml for 4G1 and 5 µg/ml for the polyclonal anti-gp41 (PA1-7219, Thermofisher). Goat anti-mouse (31430, Pierce) and donkey anti-goat (705-035-003, Jackson Immunoresearch) HRP-conjugated antibodies were used at a 1:10,000 dilution. Detection occurred directly on the membrane using SuperSignal West Pico Plus Substrate (34580, Thermo Scientific).

*Purification of Human CD4 T-lymphocytes-* Venous blood was obtained from healthy volunteers through the EFS (Etablissement Français du Sang, Centre Necker-Cabanel, Paris). CD4 T-cells were purified from whole blood using RosetteSep Human CD4+ T cell Enrichment Cocktail (Stem Cell, 15062). This cocktail contains mouse and rat monoclonal antibodies purified from mouse ascites fluid or hybridoma culture supernatant, by affinity chromatography using protein A or Protein G sepharose. These antibodies are bound in bispecific tetrameric antibody complexes which are directed against cell surface antigens on human hematopoietic cells (CD8, CD16, CD19, CD36, CD56 CD66b, TCRγ/δ) and glycophorin A on red blood cells. The rosetteSep antibody cocktail crosslinks unwanted cells in human whole blood to multiple red blood cells, forming immunorosettes. This increases the density of unwanted cells, such that they pellet along with the free red blood cells when centrifuged over a buoyant density medium such as lymphocytes separation medium (Eurobio, CMSMSL01-01).

Whole blood was incubated with RosetteSep Human CD4+ T cell Enrichment Cocktail at 50µl/ml for 20 minutes at room temperature under gentle shaking (100 rpm), diluted with an equal volume of PBS/2%FBS and mixed gently. The diluted samples were centrifuged 20 minutes at 1200 X g on top of lymphocytes separation medium. The enriched cells were then collected from the density medium at plasma interface and washed twice with PBS/2%FBS. Cells were subsequently resuspended in RPMI 1640 medium (Lonza) supplemented with 5% FBS, 50 mM HEPES pH 7.4, glutamine, penicillin, streptomycin and fungizone (complete medium), counted with a Moxi Z mini automated cell counter (ORFLO, MXZ000). Cells suspension was adjusted at 7x10⁶ cells/ml and equilibrated at least 2 h at 37°C in a 5% CO2 humidified atmosphere.

The enriched CD4-T cell population was controlled by flow cytometry on a cytoflex (Beckman coulter). The quiescence of recovered CD4 T-cells was controlled by the low level of IL-2Rα (CD25). CD4 T cells were labeled with anti-Human CD3 eFluor780 (eBioscience, clone UCHT1, 47-0038-42), anti-Human CD25-PE (Biolegend, clone BC96, 302605) and anti-human CD4-PerCP (BD, clone SK3, 345770). The enriched CD4-T cell population contains >95% CD3+CD4+ and less than 8% of CD25+.

*PLA2-GIB bioassay on CD4 T cells and labelling of specific proteins for optical microscopy-* Equilibrated purified CD4 T-cells were loaded (3.5x10⁵cells/50µl in complete medium) on poly-L-Lysine-coated (Sigma, P8920) round coverslips (14mm-diameter, Marienfeld) in 24-well polystyrene plates at 37°C in a thermo-regulated water and mixed with 50 µl of a suspension in PBS/1% BSA containing peptides, recombinant proteins together with recombinant PLA2-GIB or not or containing viremic patient plasma (1 or 3%) or healthy donor plasma. The cells suspension was either pretreated with 40 µl of peptides, recombinant protein or HIV-1 NDK particles or mock dilutions in PBS/1% BSA for 15 minutes with subsequent addition of 10 µl PLA2-GIB (5 nM at the end) for 30 minutes or directly treated with 50 µl of dilution in PBS BSA 1% with peptides or recombinant protein together with PLA2-GIB (5nM at the end) for 45 minutes. Cells were activated for 15 minutes with 2 nM recombinant glycosylated human IL-7 (Accrobio System). Cells supernatant was removed and cells were fixed by addition of 500 µl of a 4% paraformaldehyde solution in PBS (Fisher, PFA 32% Electron Microscopy Science, 15714) for 15 minutes at 37 °C and then permeabilized overnight in 500 µl of ice-cold 90% methanol/water solution.

Cells were then rehydrated for 15 min in PBS plus 5% FBS and then labeled. Thus, slides were washed twice after methanol treatment in PBS and rehydrated for 15 min in PBS supplemented with 5% FBS at room temperature. Slides were labelled with primary antibodies (1/120) in 60 µl of PBS 5% FBS for 1h, washed in PBS buffer 15 times, 5 times in PBS/FBS buffer and then stained with secondary antibodies (1/300) for 1h. Slides were washed 5 times in PBS 5% FBS buffer, rinsed 15 times in PBS and then mounted in fresh Prolong Gold Antifade (ThermoFisher Scientific, P36930) mounting medium for confocal microscopy. The primary antibodies used consisted of rabbit anti-pSTAT5 (pY694, 9359, Cell Signalling), mouse anti-CD4 (BD Pharmingen, 555344) and secondary antibodies were Donkey anti-mouse IgG-AF488 (Invitrogen, A21202) and Donkey anti-rabbit IgG-AF555 (Invitrogen, A31572).

*Confocal Microscopy-* Images were acquired above the diffraction limit on an inverted laser scanning confocal microscope (LSM700, Zeiss), with an oil-immersion plan-apochromatic 63x/1.4 NA objective lens (Zeiss) for PFA-fixed cells. Images were acquired and analyzed with the ZEN software (Zeiss).

*Determination oƒ gp41 and PEP3 plasma cofactor peptide activity on healthy donor CD4 T cells-* The role of plasma gp41 as a regulator of PLA2-GIB activity was evaluated by immunodepleting endogenous gp41 with specific antibodies. Briefly, 1 ml VP or HD plasma was incubated with 100 µg goat anti-gp41 polyclonal antibody (PA1-7219, Thermofisher) or the control goat polyclonal antibody (preimmune, AB108-C, R&D Systems), to immunodeplete gp41, in 1.5-ml Eppendorf tubes overnight (Test tube-rotor, 34528, Snijders, Netherland) at 4°C. As the goat anti-gp41 pAb initially contained 0.1% sodium azide, it was washed five times with PBS on 10 kDa Amicon (051828, Millipore) spin filters by centrifugation at 16,100 x g for 2 min at 4°C to remove the sodium azide before proceeding to immunodepletion. The normal goat IgG control was treated similarly as the anti-gp41 pAb. Then, 200 µl Protein G Sepharose 4 Fast Flow beads (17-0618-01, GE healthcare), that had been washed three times in PBS/1%BSA, was added to each sample and the samples incubated for 3h at 4°C. Beads were removed by centrifugation of the samples at 400 x g for 2 min at 4°C, collection of the supernatant then centrifugation at 16,100 x g for 15 min at 4°C. The goat anti-gp41 pAb bind specifically to recombinant gp41 but not gp120 (Figure 3A).
VP or HD plasma (500 µl) from three VPs and three HDs were fractionated on Amicon spin filters with a 30 kDa cut-off (051850, Millipore) and then a 10k Da cut-off (051828, Millipore). The 10-30 kDa fraction was collected and treated with control isotype (Mouse IgG2b/k, 16-4732-85, Thermofisher) or gp41 was immunodepleted with 100 µg of 4G1 mAb as described above. The 4G1 mAb bind specifically to recombinant gp41 but not gp120 (Figures 12B and 12C).

The effect of gp41 on PLA2-GIB activity on CD4 T cells was assessed by incubating purified CD4 T cells in PBS/1%BSA containing peptides, recombinant proteins, together or not with recombinant PLA2-GIB, VP or HD plasma, or the 10-30 kDa fraction previously depleted, or not, of gp41.
The effect of the recombinant gp41 and PEP3 gp41 peptide on PLA2-GIB activity was tested by pretreating the cell suspension for 15 min with 40 µl of the recombinant gp41 protein or peptides with subsequent addition of 10 µl PLA2G1B for 30 min (Figure 2A-D). The regulation of PLA2-GIB by endogenous gp41 was tested by treating the cell suspension for 30 min with 50 µl of plasma dilutions or 10-30 kDa plasma fraction, previously depleted or not of gp41 (Figures 3B, 13 and 14). pSTAT5 NT inhibition was examined by microscopy as described above.

### RESULTS AND DISCUSSION

### 1.HIV-1 gp41 increases PLA2-GIB inhibitory activity on pSTAT5 NT in CD4 T cells stimulated with IL-7

We analyzed pSTAT5 NT response to IL-7 in cells pretreated with a dose of PLA2-GIB that cannot inhibit pSTAT5 NT in absence of cofactor (5nM), together or not with a recombinant gp41 protein or with gp41 protein alone, without PLA2-GIB (w/o GIB). As shown on Figure 2, gp41 protein alone has a minor inhibitory effect on pSTAT5 NT response to IL-7 at 34 nM of gp41 with only 10% of inhibition and less than 8% of inhibition with 17 to 3.4 nM of gp41 (Figures 2A and 2B). By a striking contrast, in presence of 5 nM of PLA2-GIB, 34 nM of gp41 protein resulted in more than 60% of inhibition of pSTAT5 NT (p<0.05, n=3 donors, Figure 2B) with a dose-dependent inhibition to 18% of inhibition with 0.34 nM of gp41 (Figure 2A).

### 2.The PEP3 motif in gp41 inhibits pSTAT5 NT in CD4 T cells stimulated with IL-7

CD4 T cells were exposed to a 15 amino acids peptide domain of gp41 containing a gClqR binding element and a highly conserved SWSNKS motif. The cells were also exposed to a control (CTL) peptide (Figure 2C), together with 5 nM of PLA2-GIB (5 nM) or not (w/o). While CTL peptide alone or with PLA2-GIB or PEP3 alone have no effect on pSTAT5 NT, treatments with PEP3 and 5nM of PLA2-GIB resulted in a PEP3 dose-dependent inhibition of pSTAT5 NT from 51% to 18% of inhibition with 1375 to 13.75 nM of PEP3 respectively (Figure 2C). As summarized on Figure 2D, treatment with 275 nM of PEP3 alone only resulted in 4% of inhibition of pSTAT5 NT in CD4 T cells while treatment with 275 nM of PEP3 together with 5nM PLA2-GIB resulted in 51% of inhibition (p<0.01, n=4 donors).

### 3.HIV-1 gp41 protein plays a critical role in the inhibitory activity of viremic patient plasma on pSTAT5 NT in CD4 T cells stimulated with IL-7

We first identified polyclonal antibodies that are specific of gp41 relatively to gp120, another HIV envelope subunit, in immunoblot (Figure 3A). Then to verify that gp41 protein could be a cofactor of PLA2-GIB in viremic patient plasma, we depleted viremic patient plasma with polyclonal antibodies against gp41 (pAb anti-gp41) or control polyclonal antibodies (pAb ctrl). Healthy donor plasma was similarly treated as negative control. As presented on Figure 3B, the inhibition of pSTAT5 NT was 49% with 75 nM and 80% with 250 nM of PLA2-GIB as expected and 47% with 1% and 60% with 3% of viremic patient plasma without antibody. Healthy donor plasma had no inhibitory effect on pSTAT5 NT in response to IL-7 without antibody, with control polyclonal antibody or anti-gp41 polyclonal antibody which demonstrates that antibodies have no toxicity on CD4 T cells (Figure 3B). Treatment of viremic patient plasma with control polyclonal antibody does not change the inhibitory activity with 49% and 60% of inhibition with 1% and 3% of plasma respectively (Figure 3B). Notably, immunodepletion with anti-gp41 polyclonal antibody almost abrogated the inhibitory activity of viremic patient plasma with 12% and 14% of residual inhibitory activity with 1% and 3% of immunodepleted plasma (*p*<0.01 and *p*<0.001 pAb anti-gp41- vs pAb ctrl-treated plasma respectively, Figure 3B). Altogether these results demonstrate that gp41 is a cofactor of PLA2-GIB in viremic patient plasma.

### 4.Development of anti-PEP3 monoclonal antibodies

The above demonstration that PEP3 peptide alone is able to increase the inhibitory activity of PLA2-GIB (Figures 2C and 2D) suggests that this sequence is present in the HIV viremic plasma gp41 protein and is very likely involved in the cofactor effect on plasma PLA2-GIB. We developed new anti-gp41 antibodies specific of the PEP3 sequence. OF1 mice were immunized with a peptide containing only the PEP3 sequence conjugated to KLH. From 1093 hybridoma, 1072 were positive on BSA-PEP3 peptide by ELISA. The best 123 were confirmed another time on BSA-PEP3 peptide and MN gp41 protein by ELISA. We selected 27 clones (Table 1) that were frozen and their isotypes were determined. Among them, 6 clones were maintained in cell culture to obtain stable clones: 4G1, 8D5, 3A2, 15B4, 5E12 and 9B12. High amount of antibodies were purified from these 6 clones. They were fully characterized by ELISA on BSA-PEP3 peptide, MN and HXB2 gp41 recombinant proteins with calculation of EC50 of antibody binding (Figure 5). Their variable light (VL) and heavy (VL) chain regions were sequenced (Figures 6-11). This confirmed the light (Figures 6-11A and B) and heavy chain isotypes (Figures 6-11D and E) and allow the prediction of their functional subdomains FR1-FR4 and CDR1-3 and to establish a model of representation of the 3D structure (IMGT colliers de Perles) of the VL (Figures 6-11C) and VH (Figures 6-11F). The analysis of VL (Figures 6-11B) and VH (Figures 6-11E) sequence identity with mouse germinal sequence demonstrate that no equivalent sequence have previously been described in database (Uniprot databank-UniportkB). Results of EC50 bindings on several MN and HXB2 gp41 recombinant proteins and BSA-PEP3 and isotypes of light and heavy chains of the 6 anti-PEP3 mAbs are summarized in Figure 12A. We first focused our interest on the 4G1 mAb because it has the highest EC50 on BSA-PEP3 and the first MN gp41 (produced in E. Coli) we tested (Figure 12A), then additional analyses confirmed that it has one of the highest EC50 on all gp41 recombinant proteins tested by ELISA (Figure 12A). We confirmed by ELISA and immunoblot on recombinant gp41 and gp120 proteins that this antibody is specific of the HIV envelope gp41 proteins (Figures 12B and C). Altogether these results show the specificity and the high affinity of the 4G1 mAb for PEP3 gp41 sequence.

### 5.The 4G1 anti-PEP3 mAb almost abrogates the inhibitory activity of viremic patient plasma on pSTAT5 NT in CD4 T cells stimulated with IL-7

We depleted viremic patient (VP) plasma with the 4G1 anti-PEP3 mAb (anti-PEP3 mAb) or control isotype (mAb ctrl). The plasma of healthy donor (HD) was similarly treated as negative control. The inhibition of pSTAT5 NT was 47% with 75 nM and 67% with 250 nM of PLA2-GIB and 49% with 1% and 58% with 3% of VP plasma without antibody (Figure 13). Healthy donor (HD) plasma had only slight inhibitory effect on pSTAT5 NT in response to IL-7 without antibody. Similarly, the fraction of HD plasma treated with both antibodies had no inhibitory activity on pSTAT5 NT which demonstrates that antibodies have no toxicity on CD4 T cells (Figure 13). Interestingly, treatment with control isotype of the fraction of VP plasma did not change the inhibitory activity with 48% with 1% and 61% with 3% of the fraction of VP plasma respectively. This level of inhibition was very similar as total VP plasma alone which further supported our previous findings that VP plasma activity is due to a 10-30kDa fraction (Figure 13). Notably, 4G1 immunodepletion highly decreases the inhibitory activity of VP plasma with 9% and 19% of residual inhibitory activity with 1% and 3% of immunodepleted plasma (p<0.001 and *p*<0.01 4G1 anti-PEP3-vs ctrl mAb-treated plasma respectively, Figure 13). To compare 4G1 and goat anti-gp41 pAb effect on VP inhibitory activity we combined data presented on Figures 3B and 13 on the Figure 14. Altogether these results show that goat anti-gp41 (Figure 14A) and 4G1 anti-PEP3 mAb (Figure 14B) both highly decrease VP inhibitory activity. This confirms our model of regulation of PLA2-GIB activity in plasma of viremic plasma by a gp41 fragment with the PEP3 sequence.

### 6.Detection of gp41 in HIV viremic plasma: development of anti-gp41 Ab and ELISA

Another challenge is to quantify gp41 products in the plasma of HIV viremic patient. The presence of anti-gp41 antibodies in the plasma of HIV viremic patients has been well described (Boyd and James, 1992; Butler et al., 2019; Mikell et al., 2011; Trama et al., 2014; Vaidya et al., 2018; Williams et al., 2015). The first anti-HIV protein monoclonal antibodies observed from blood plasma cells of acute HIV-1-infected individuals are predominantly targeted to gp41 (Butler et al., 2019; Trama et al., 2014). However to our knowledge the gp41 protein has never been detected in the plasma of HIV infected patients which suggests that the concentration of gp41 is very low and/or the anti-gp41 antibodies that have been developed have too low affinity to allow the detection of gp41 in plasma.
Among the 6 anti-PEP3 mAbs we developed, the 4 mAbs that bind with the highest affinity to gp41 proteins are 4G1, 8D5, 3A2 and 15B4 (Figure 12A). To evaluate the potential of the antibodies to be used in ELISA, we tested these anti-PEP3 mAbs and goat anti-gp41 pAb in sandwich ELISA on recombinant MN gp41 or HXB2 gp41 (Figures 15A-D). All antibodies allow the capture of gp41 and detection with biotinylated goat pAb (Figures 15A-D). The 4G1, 3A2 and 8D5 have lower EC50 calculated on HXB2 gp41 than 15B4 (Figures 15C and D). However the minimal amount of gp41 detected remain high, about 1 ng/ml for 4G1, 8D5 and 3A2 as capture Ab and more than 10 ng/ml for 15B4 (Figures 15B and C). We also tested 15B4, 8D5 and 3A2 as capture Ab and biotinylated 4G1 as detection Ab. The minimal amount of gp41 detected was 37.5 ng/ml with 8D5 as capture Ab and 150 ng/ml with 3A2 as capture Ab (Figure 15E). 15B4 did not form a sandwich with 4G1 under the experimental condition tested.

In order to further increase the efficiency of the antibodies to detect gp41 in ELISA, we generated additional antibodies against gp41. Our experiments have shown that the gp41 fragment in VP plasma contains the PEP3 sequence and is into the 10-30kDa fraction of VP plasma. So, we hypothesized that it could be a part of/or the ectodomain of the gp41 protein. We thus decided to immunize OF1 mice against the peptide that we named 5184-3 present at the C-terminal part of the second heptad repeat region (HR2) of gp41 (Figures 16A and B).
We selected two mice (S12 and S13) based on the titer of their serum on 5184-3 peptide and HXB2 gp41 protein. Among 816 supernatants 4 clones that produced anti-HR2 Ab (anti-5184-3) were positive on BSA-5184-3 peptide by ELISA and confirmed by ELISA on BSA-5184-3, MN gp41 (produced in S2 cells) and HXB2 gp41 protein (63G4, 69D6 and 67D7 from mouse S12 and 72D7 from mouse S13, Figure 17). Their isotypes were determined and they were frozen.
Then we tested the 4 anti-HR2 Ab capacity to be used in sandwich ELISA with 4G1 mAb. A mini production of these anti-HR2 antibodies was performed in vivo after injection of hybridoma in nude mice. Purity and quantity obtained were high for 67D7 and 72D7 but low for 63G4 and 69D6 (Figure 18A). Thus only 67D7 and 72D7 were biotinylated (67D7-biot and 72D7-biot). The binding of the purified antibodies biotinylated or not was checked on 5184-3 (Figure 18B) or HXB2 gp41 protein (Figure 18C). The binding of 4G1 and biotinylated 4G1 (4G1-biot) batches to PEP3 peptide and HXB2 gp41 protein were also checked (Figures 18B and 18C).
Then the pairing of 4G1, 63G4, 69D6, 67D7 and 72D7 as capture Ab and 4G1-biot, 72D7-biot and 67D7-biot as detection Ab were tested using HXB2-gp41 antigen (Figures 19A-E). The only pairing observed was 72D7 as capture Ab and 4G1-biot as detection Ab.
The pairing of 72D7 with 4G1-biot was confirmed on various dilutions of HXB2 gp41 protein and compared with goat anti-gp41 pAb (Figure 20). Notably, 72D7 can be used as capture Ab with both 4G1-biot and goat anti-gp41 pAb-biot as detection Ab and the EC50 were very similar: 0.56 µg/ml with anti-gp41 pAb-biot and 0.34 µg/ml with 4G1-biot (Figure 20).

**7. Test of detection and quantification of gp41 fragment in plasma of HIV viremic plasma using the single molecule array (SIMOA) technology.** The concentration of gp41 in the plasma of HIV viremic plasma is probably very low, that would explain that even if anti-gp41 antibodies have been well described in the plasma of viremic patient plasma, the gp41 protein itself has not been detected by classical ELISA or immunoblot assays. The anti-PEP3 and 72D7 mAb can be used to detect gp41 in VP plasma using the ultrasensitive technique SIMOA that in general increases the 1000-fold the sensitivity in comparison to ELISA.

### REFERENCES

Boyd, J.E., and James, K. (1992). B cell responses to HIV and the development of human monoclonal antibodies. Clin. Exp. Immunol. 88, 189-202.
Butler, A.L., Fischinger, S., and Alter, G. (2019). The Antibodiome-Mapping the Humoral Immune Response to HIV. Curr. HIV/AIDS Rep. 16, 169-179.
Mikell, I., Sather, D.N., Kalams, S.A., Altfeld, M., Alter, G., and Stamatatos, L. (2011). Characteristics of the Earliest Cross-Neutralizing Antibody Response to HIV-1. PLoS Pathog. 7, e1001251.
Trama, A.M., Moody, M.A., Alam, S.M., Jaeger, F.H., Lockwood, B., Parks, R., Lloyd, K.E., Stolarchuk, C., Scearce, R., Foulger, A., et al. (2014). HIV-1 Envelope gp41 Antibodies Can Originate from Terminal Ileum B Cells that Share Cross-Reactivity with Commensal Bacteria. Cell Host Microbe 16, 215-226.
Vaidya, N.K., Ribeiro, R.M., Liu, P., Haynes, B.F., Tomaras, G.D., and Perelson, A.S. (2018). Correlation Between Anti-gp41 Antibodies and Virus Infectivity Decay During Primary HIV-1 Infection. Front. Microbiol. 9, 1326.
Williams, W.B., Liao, H.-X., Moody, M.A., Kepler, T.B., Alam, S.M., Gao, F., Wiehe, K., Trama, A.M., Jones, K., Zhang, R., et al. (2015). Diversion of HIV-1 vaccine-induced immunity by gp41-microbiota cross-reactive antibodies. Science (80-.). 349, aab1253-aab1253.

**Table 1. Characterization of the 27 anti-PEP3 hybridoma selected : Isotypes and results of screening by ELISA.**

| | | **BSA-PEP3 peptide** | | **MN gp4 1 protein** | | **w/o Ag** | |
|---|---|---|---|---|---|---|---|
| **hybridomes** | **Isotypes** | Pur | 1/10 | Pur | 1/10 | Pur | 1/10 |
| **1C5** | IgG2b, IgG1/κ | 2,767 | 3,087 | 2,808 | 2,794 | 0,125 | 0,055 |
| **2G11** | IgG1/λ, κ | 2,605 | 2,377 | 0,189 | 0,103 | 0,121 | 0,075 |
| **3A2** | IgG1, IgG2b/κ | 2,933 | 3,059 | 2,634 | 2,746 | 0,071 | 0,061 |
| **5C1** | IgM, IgG2a, IgG1, IgG2b/κ, λ | 2,514 | 2,544 | 1,750 | 1,816 | 0,070 | 0,059 |
| **5E12** | IgG1, IgM/κ | 3,003 | 3,210 | 2,610 | 2,709 | 0,095 | 0,048 |
| **6G12** | IgG2b, IgG1, IgM/κ | 2,605 | 2,785 | 0,100 | 0,062 | 0,071 | 0,041 |
| **6H7** | IgG1/κ | 2,491 | 2,479 | 1,984 | 1,978 | 0,055 | 0,054 |
| **7B12** | IgG2b, IgG3/κ | 2,644 | 2,496 | 0,051 | 0,043 | 0,051 | 0,041 |
| **7C1** | IgG1, IgG2a/κ | 2,599 | 2,501 | 0,096 | 0,066 | 0,045 | 0,043 |
| **7E5** | IgG1, IgM/κ | 2,418 | 2,651 | 0,059 | 0,066 | 0,044 | 0,043 |
| **7H10** | IgG2b/κ | 2,607 | 2,700 | 0,078 | 0,051 | 0,053 | 0,043 |
| **8B4** | IgG1, IgG2b/κ | 2,240 | 2,347 | 1,894 | 1,911 | 0,047 | 0,042 |
| **8B5** | IgG1/κ | 2,193 | 2,167 | 1,355 | 1,323 | 0,046 | 0,041 |
| **8D5** | IgG2b, IgG1/κ | 2,509 | 2,686 | 2,579 | 2,552 | 0,048 | 0,043 |
| **8F6** | IgG1/κ | 2,138 | 2,018 | 1,465 | 1,508 | 0,043 | 0,051 |
| **9B12** | IgG2a/κ | 2,788 | 2,782 | 0,054 | 0,047 | 0,048 | 0,041 |
| **9C6** | IgG1/κ | 2,536 | 2,512 | 2,114 | 2,227 | 0,101 | 0,125 |
| **9D1** | IgG2b, IgG1/κ | 2,780 | 3,006 | 2,178 | 2,397 | 0,053 | 0,048 |
| **9D6** | IgG1/κ | 2,330 | 2,593 | 2,014 | 2,093 | 0,049 | 0,048 |
| **10A2** | IgG1/κ | 2,501 | 2,294 | 0,086 | 0,082 | 0,073 | 0,069 |
| **10B2** | IgG2b, IgG1/κ | 2,775 | 3,017 | 2,072 | 2,326 | 0,061 | 0,072 |
| **10D5** | IgG1/κ | 2,033 | 2,056 | 1,449 | 1,334 | 0,043 | 0,040 |
| **10D11** | IgG2a, IgG2b/κ | 2,849 | 2,971 | 0,048 | 0,042 | 0,045 | 0,042 |
| **10F6** | IgG1/κ | 2,473 | 2,617 | 2,077 | 2,330 | 0,048 | 0,061 |
| **14A3** | IgM,IgG2a,IgG2b/κ | 2,451 | 2,331 | 1,771 | 1,680 | 0,045 | 0,043 |
| **15B4** | IgG2b/κ | 2,726 | 2,587 | 1,815 | 1,445 | 0,048 | 0,040 |
| **17C4** | IgG1/κ | 2,737 | 2,835 | 1,866 | 1,827 | 0,055 | 0,051 |

### LIST OF SEQUENCES (see Seq Listing)

SEQ ID NO: 1 gp41
AAIGALFLGFLGAAGSTMGAASVTLTVQARLLLSGIVQQQNNLLRAIESQQHMLRLTVWGIKQLQARVLAVERYLKDQ
QLLGFWGCSGKLICTTTVPWNASWSNKSLDDIWNNMTWMQWEREIDNYTSLIYSLLEKSQTQQEKNEQELLELDKWAS
LWNWFDITNWLWYIKIFIMIVGGLVGLRIVFAVLSIVNRVRQGYSPLSLQTRPPVPRGPDRPEGIEEEGGERDRDTSG
RLVHGFLAIIWVDLRSLFLLSYHHLRDLLLIAARIVELLGRRGWEVLKYWWNLLQYWSQELKSSAVSLLNAAAIAVAE
GTDRVIEVLQRAGRAILHIPTRIRQGLERALL

SEQ ID NO : 2 PEP3
   PWNASWSNKSLDDIW 
SEQ ID NO :3 >4G1-VL
DVVMTQTPLSLCVSLGDQASISCRTSQSIVHSNRYTYLEWYLQKPGQPLKLLIYGIANRFSGVPDRFSGSGSGTDFTL
KISRVEAEDMGVYYCLQGTHVPFTFGSGTKLEIK
 
SEQ ID NO :4 >4G1-VH
QVQLQQPGAELVKPGASVKLSCKASGYIFRNYWMHWVKQRPGQGLEWIGMIHPNSDITHYNEKFKNRATLTVDKSSST
AYMQLNSLTSEDSAVYYCARGGLGVFDYWGQGTALRVSS
 
SEQ ID NO :5 >3A2-VL
DVVMTQTPLSLPVSLGDQVSMSCRSSQSIEHSNRNTYLEWYLHKPGQAPQLLIYGVSNRFTGVPDRFSGSGSGTDFTL
KISRVEAEDMGVYHCLQGTHVPFTFGSGTKLEIK
 
SEQ ID NO :6 >3A2-VH
QVQLQQPGAELVKPGASVKLSCKASGYIFTNYWMHWVRQRPGQGPEWIGMIHPNRGDLHYSEKFNNKATLTVDKSSST
AYMQLNSLASEDSAVYFCARAGLGVFDYWGQGSTLTVSS
 
SEQ ID NO :7 >8D5-VL
DVVMTQTPLSLPVSLGDQASISCRSSQSIVHSNRYTYLEWYLQRPGQSLKLLIYGVSNRFSGVPDRFSGSGSGTDFIL
KISRVEAEDMGVYYCFQGTHVPFTFGSGTKLEIK
 
SEQ ID NO :8 >8D5-VH
QVQLQQPGAELIEPGTSVKLSCKASGYTFTTYWMHWVKQRPGQGLEWIGMIHPNSGDTFYNENFKNKATLTVDISSST
VYMELSSLTSEDSAVYSCVKAGTGALDYWGQGTTLTVSS
 
SEQ ID NO :9 >15B4-VL
DIQMTQTPLTLSVTIGQPASISCKSGQSLLYSNGKTYLNWLLQRPGQSPKRLIYLVSKLDSGVPDRFSGSGSGTDFTL
KISRVEAEDLGVYYCLQSTHFPRTFGGGTKLEIK
 
SEQ ID NO :10 >15B4-VH
QVQLQQSGPELVRPGTSVKISCKAPGYSFTSHWMQWLRQRPGQGLEWIGEIFPGSGKTYYNEKFKDKATLTADTSSTT
VYMQFSSLTSEDSAVYFCASDYDGGDYWGQGTTLTVSS
 
SEQ ID NO :11 >5E12-VL
DIQMTQTPLTLSVTIGHPASISCKSSQSLLHTNGKIYLNWLLQRPGQSPKRLIYLVSKLDSGVPDRFSGSGSGTDFTL
KISRVEAEDLGVYYCLQSTHFPQTFGGGTKLEIK
 
SEQ ID NO :12 >5E12-VH
QIQLVQSGPELKKPGETVKISCKASGYTFRTYGMSWVKQAPGKGLKWMGWINTYSGVPRYVDDFKGRFALSLETSAST
AYLQINNLKSEDTATYFCVREGYSYYGSSFHTMDYWGQGTSVTVSS
 
SEQ ID NO :13 >9B12-VL
DVVMTQTPLTLPVTIGQPASISCKSSQSLLNSDGKTKLNWLLQRPGESLKLLIYLVSKLDSGVPDRFTGSGSGTDFTL
KISRVEAEDLGVYYCLQTTHFPRTFGGGTKLEIK
 
SEQ ID NO :14 >9B12-VH
QVQLQQSGPELVKPGASVKISCKASGYGFSDSWMNWVKQRPGKGLDWIGRIYPGDGDTDYNGKFKDKATLTADKSSTT
AYMQLSSLTSEDSAVYFCAYLGHWGQGTLVTVSA
SEQ ID NO: 15
   QSIVHSNRYTY
SEQ ID NO: 16
   GIAN
SEQ ID NO: 17
   LQGTHVPFT
SEQ ID NO: 18
   GYIFRNYW
SEQ ID NO: 19
   IHPNSDIT
SEQ ID NO: 20
   ARGGLGVFDY
SEQ ID NO: 21
   QSIEHSNRNTY
SEQ ID NO: 22
   GVS
SEQ ID NO: 23
   LQGTHVPFT
SEQ ID NO: 24
   GYIFTNYW
SEQ ID NO: 25
   IHPNRGDL
SEQ ID NO: 26
   ARAGLGVFDY
SEQ ID NO: 27
   QSIVHSNRYTY
SEQ ID NO: 28
   GVS
SEQ ID NO: 29
   FQGTHVPFT
SEQ ID NO: 30
   GYTFTTYW
SEQ ID NO: 31
   IHPNSGDT
SEQ ID NO: 32
   VKAGTGALDY
SEQ ID NO: 33
   QSLLYSNGKTY
SEQ ID NO: 34
   LVS
SEQ ID NO: 35
   LQSTHFPRT
SEQ ID NO: 36
   GYSFTSHW
SEQ ID NO: 37
   IFPGSGKT
SEQ ID NO: 38
   ASDYDGGDY
SEQ ID NO: 39
   QSLLHTNGKIY
SEQ ID NO: 40
   LVS
SEQ ID NO: 41
   LQSTHFPQT
SEQ ID NO: 42
   GYTFRTYG
SEQ ID NO: 43
   INTYSGVP
SEQ ID NO: 44
   VREGYSYYGSSFHTMDY
SEQ ID NO: 45
   QSLLNSDGKTK
SEQ ID NO: 46
   LVS
SEQ ID NO: 47
   LQTTHFPRT
SEQ ID NO: 48
   GYGFSDSW
SEQ ID NO: 49
   IYPGDGDT
SEQ ID NO: 50
   AYLGH 
SEQ ID NO :51 >4G1-VL
GATGTTGTGATGACCCAAACTCCACTCTCCCTGTGTGTCAGTCTTGGAGATCAAGCCTCCATCTCTTGCAGAACCAGT
CAGAGCATTGTACACAGTAATAGATACACCTATTTAGAATGGTACCTGCAGAAACCAGGCCAGCCGCTAAAACTCCTG
ATATATGGGATTGCCAACCGATTTTCTGGGGTCCCAGACAGGTTCAGTGGCAGTGGATCAGGGACAGATTTCACACTC
AAGATCAGCAGAGTGGAGGCTGAGGATATGGGAGTTTATTATTGTCTTCAAGGTACACATGTTCCATTCACGTTCGGC
TCGGGGACAAAGTTGGAAATAAAA
 
SEQ ID NO :52 >4G1-VH
CAGGTCCAACTGCAGCAACCTGGGGCTGAGCTGGTAAAGCCTGGGGCTTCAGTAAAGTTGTCCTGCAAGGCTTCTGGC
TACATTTTCAGAAACTACTGGATGCACTGGGTGAAGCAGAGGCCTGGACAAGGCCTTGAATGGATTGGAATGATTCAT
CCTAATAGTGATATTACTCATTACAATGAGAAGTTCAAAAACAGGGCCACACTGACTGTTGACAAATCCTCCAGCACA
GCCTACATGCAACTCAACAGCCTGACATCTGAAGACTCTGCGGTCTATTACTGTGCAAGGGGGGGGCTGGGGGTGTTT
GACTACTGGGGCCAAGGCACCGCTCTCAGAGTCTCCTCA
 
SEQ ID NO :53 >3A2-VL
GATGTTGTGATGACCCAAACTCCACTCTCCCTGCCTGTCAGTCTTGGAGATCAAGTCTCCATGTCTTGCAGATCTAGC
CAGAGCATTGAACACAGTAATAGAAACACCTATTTAGAATGGTACCTGCACAAACCAGGCCAGGCGCCACAGCTCCTG
ATATATGGGGTTTCCAACCGATTTACTGGGGTCCCAGACAGGTTCAGTGGCAGTGGATCAGGGACAGATTTCACACTC
AAGATCAGTAGAGTGGAGGCTGAGGATATGGGAGTTTATCATTGCTTGCAAGGTACACATGTTCCATTCACGTTCGGC
TCGGGGACAAAGTTGGAAATAAAA
 
SEQ ID NO :54 >3A2-VH
CAGGTCCAACTGCAACAGCCTGGGGCTGAGCTGGTAAAGCCTGGGGCTTCAGTGAAATTGTCCTGCAAGGCTTCTGGC
TACATTTTCACCAATTACTGGATGCACTGGGTGAGGCAGAGGCCTGGACAAGGCCCTGAGTGGATTGGAATGATTCAC
CCTAATAGGGGTGATCTTCACTACAGCGAGAAGTTCAACAACAAGGCCACACTGACTGTAGACAAATCCTCCAGCACA
GCCTACATGCAACTCAACAGCCTGGCATCTGAGGACTCTGCGGTCTATTTCTGTGCAAGGGCGGACTGGGGGTGTTT
GACTACTGGGGCCAGGGCTCCACTCTCACAGTCTCCTCAGCCAAAACAACA
 
SEQ ID NO :55 >8D5-VL
GATGTTGTGATGACCCAAACTCCGCTCTCCCTGCCTGTCAGTCTTGGAGATCAAGCCTCCATCTCCTGCAGATCTAGT
CAGAGCATTGTACACAGTAATAGATACACCTATTTAGAATGGTATCTGCAGAGACCAGGCCAGTCGCTAAAGCTCCTG
ATATACGGGGTTTCCAACCGATTTTCTGGGGTCCCAGACAGGTTCAGTGGCAGTGGATCAGGGACAGATTTCATACTC
AAGATCAGCAGAGTGGAGGCTGAGGATATGGGAGTTTATTACTGCTTTCAAGGTACACATGTTCCATTCACGTTCGGC
TCGGGGACAAAGTTGGAAATAAAA
 
SEQ ID NO :56 >8D5-VH
CAGGTCCAACTGCAGCAGCCTGGGGCTGAGCTGATAGAGCCTGGGACTTCAGTGAAGTTGTCCTGCAAGGCTTCTGGC
TACACTTTCACCACTTACTGGATGCACTGGGTGAAGCAGAGGCCTGGACAAGGCCTGGAGTGGATTGGAATGATTCAT
CCTAATAGTGGTGATACTTTTTACAATGAGAACTTCAAGAACAAGGCCACACTGACTGTAGACATATCCTCCAGCACA
GTCTACATGGAACTCAGCAGCCTGACATCTGAGGACTCTGCGGTCTATTCCTGTGTAAAGGCTGGGACGGGCGCCTTA
GACTACTGGGGCCAAGGCACCACTCTCACAGTCTCCTCG
 
SEQ ID NO :57 >15B4-VL
GATATCCAGATGACACAGACTCCACTCACTTTGTCGGTTACCATTGGCCAACCAGCTTCCATCTCTTGCAAGTCAGGT
CAGAGCCTCTTATATAGTAATGGAAAAACCTATTTGAATTGGTTATTACAGAGGCCAGGCCAGTCTCCAAAACGCCTA
ATCTATCTGGTGTCTAAATTGGACTCTGGAGTCCCTGACAGGTTCAGTGGCAGTGGATCAGGGACAGATTTCACACTA
AAAATCAGCAGAGTGGAGGCTGAGGATTTGGGAGTTTATTACTGCTTGCAGAGTACACATTTTCCTCGGACGTTCGGT
GGAGGCACCAAGCTGGAAATCAAA
 
SEQ ID NO :58 >15B4-VH
CAGGTTCAGCTGCAGCAGTCTGGACCTGAGCTGGTGAGGCCTGGGACTTCAGTGAAGATATCCTGCAAGGCTCCTGGC
TATAGTTTCACCAGCCACTGGATGCAGTGGCTAAGACAGAGGCCTGGACAGGGCCTTGAGTGGATTGGAGAGATTTTT
CCTGGAAGTGGTAAAACTTATTATAATGAGAAGTTCAAGGACAAGGCCACACTGACTGCAGACACATCCTCCACCACA
GTCTATATGCAGTTCAGCAGCCTGACATCTGAGGACTCTGCGGTCTATTTCTGTGCGAGTGATTACGACGGAGGAGAC
TACTGGGGCCAAGGCACCACTCTCACAGTCTCCTCA
 
SEQ ID NO :59 >5E12-VL
GATATCCAGATGACACAGACTCCACTCACTTTGTCGGTTACCATTGGACACCCAGCTTCCATCTCTTGCAAGTCAAGT
CAGAGCCTCTTACATACTAATGGAAAAATCTATTTGAATTGGTTATTACAGAGGCCAGGCCAGTCTCCAAAACGCCTA
ATCTATCTGGTGTCTAAATTGGACTCTGGAGTCCCTGACAGGTTCAGTGGCAGTGGATCAGGGACAGATTTCACACTG
AAAATCAGCAGAGTGGAGGCTGAGGATTTGGGAGTTTATTACTGCTTGCAGAGTACACATTTTCCTCAGACGTTCGGT
GGAGGCACCAAGCTGGAAATCAAA
 
SEQ ID NO :60 >5E12-VH
CAGATCCAGTTGGTACAGTCTGGACCTGAGCTGAAGAAGCCTGGAGAGACAGTCAAGATCTCCTGCAAGGCTTCTGGC
TATACCTTCAGAACCTATGGAATGAGCTGGGTGAAACAGGCTCCAGGAAAGGGTTTAAAGTGGATGGGCTGGATAAAC
ACCTACTCTGGAGTGCCAAGATATGTTGATGACTTTAAGGGACGGTTTGCCCTCTCTTTGGAAACCTCTGCCAGCACT
GCCTATTTGCAGATCAATAACCTCAAAAGTGAGGACACGGCTACATATTTCTGTGTAAGAGAGGGTTACTCGTATTAC
GGTAGTAGTTTTCATACTATGGACTACTGGGGTCAAGGAACCTCAGTCACCGTCTCCTCA
 
SEQ ID NO :61 >9B12-VL
GATGTTGTGATGACCCAGACTCCACTCACTTTGCCGGTCACCATTGGACAACCAGCTTCCATCTCTTGCAAGTCAAGT
CAGAGCCTCTTAAATAGTGATGGAAAAACCAAGTTAAATTGGTTATTACAGAGGCCAGGCGAGTCTCTAAAGCTCCTA
ATCTATCTGGTGTCTAAACTGGACTCTGGAGTCCCTGACAGGTTCACTGGCAGTGGATCAGGGACAGATTTCACACTG
AAAATCAGCAGAGTGGAGGCTGAGGATTTGGGAGTTTATTACTGCTTGCAAACTACACATTTTCCTCGGACGTTCGGT
GGAGGCACCAAGCTGGAAATCAAA
 
SEQ ID NO :62 >9B12-VH
CAGGTTCAGCTGCAGCAGTCTGGACCTGAACTGGTGAAGCCTGGGGCCTCAGTGAAGATTTCCTGCAAGGCTTCTGGC
TACGGATTCAGTGACTCCTGGATGAACTGGGTGAAGCAGAGGCCTGGAAAGGGTCTTGACTGGATTGGACGGATTTAT
CCTGGAGATGGAGATACTGACTACAATGGGAAGTTCAAGGACAAGGCCACACTGACTGCAGACAAATCCTCCACTACA
GCCTACATGCAACTCAGCAGCCTGACATCTGAGGACTCTGCGGTCTACTTCTGTGCGTATCTTGGCCACTGGGGCCAA
GGGACTCTGGTCACTGTCTCTGCA
SEQ ID NO: 63 (5184-3 peptide)
   EKSQTQQEKNEQELLELDK
SEQ ID NO: 64 (HXB2 domain)
   EESQNQQEKNEQELLELDK

SEQ ID NO: 65: gC1qR
MLPLLRCVPRVLGSSVAGLRAAAPASPFRQLLQPAPRLCTRPFGLLSVRAGSER

RPGLLRPRGPCACGCGCGSLHTDGDKAFVDFLSDEIKEERKIQKHKTLPKMSGG
WELELNGTEAKLVRKVAGEKITVTFNINNSIPPTFDGEEEPSQGQKVEEQEPELT
STPNFVVEVIKNDDGKKALVLDCHYPEDEVGQEDEAESDIFSIREVSFQSTGESE
WKDTNYTLNTDSLDWALYDHLMDFLADRGVDNTFADELVELSTALEHQEYIT
FLEDLKSFVKSQ

## Claims

1. An isolated monoclonal antibody, or a variant or fragment thereof, that binds to an epitope of a HIV gp41 protein, wherein the epitope includes at least one amino acid residues of PEP3 domain of gp41 protein, and wherein the monoclonal antibody or variant or fragment thereof inhibits the binding of gp41 to ClqR.

2. An isolated monoclonal antibody, or a variant or fragment thereof, that binds HIV gp41 protein at one or more amino acid residues of PEP3 domain of gp41 protein, and wherein the monoclonal antibody or variant or fragment thereof inhibits the binding of gp41 to ClqR.

3. A monoclonal antibody comprising a light chain variable region, wherein the light chain variable region comprises (i) a CDR-L1 consisting, or consisting essentially, of a selected from SEQ ID NOs: 15, 21, 27, 33, 39, or 45, and/or (ii) a CDR-L2 consisting, or consisting essentially, of a selected from SEQ ID NOs: 16, 22, 28, 34, 40, or 46, and/or (iii) a CDR-L3 consisting, or consisting essentially, of a selected from SEQ ID NOs: 17, 23, 29, 35, 41, or 47.

4. The monoclonal antibody of claim 3, wherein the light chain variable region comprises a CDR-L1, a CDR-L2 and a CDR-L3 as follows:
• SEQ ID NOs: 15, 16 and 17; or
• SEQ ID NOs: 21, 22 and 23, or
•SEQ ID NOs: 27, 28, and 29, or
• SEQ ID NOs: 33, 34 and 35, or
• SEQ ID NOs: 39, 40 and 41, or
• SEQ ID NOs: 45, 46 and 47.

5. A monoclonal antibody of anyone of the preceding claims, comprising a heavy chain variable region, wherein the heavy chain variable region comprises (i) a CDR-H1 consisting, or consisting essentially, of a selected from SEQ ID NOs: 18, 24, 30, 36, 42, or 48, and/or (ii) a CDR-H2 consisting, or consisting essentially, of a selected from SEQ ID NOs: 19, 25, 31, 37, 43, or 49, and/or (iii) a CDR-H3 consisting, or consisting essentially, of a selected from SEQ ID NOs: 20, 26, 32, 38, 44, or 50.

6. The monoclonal antibody of claim 5, wherein the heavy chain variable region comprises a CDR-H1, a CDR-H2 and a CDR-H3 as follows:
• SEQ ID NOs: 18, 19 and 20; or
• SEQ ID NOs: 24, 25 and 26, or
• SEQ ID NOs: 30, 31, and 32, or
• SEQ ID NOs: 36, 37 and 38, or
• SEQ ID NOs: 42, 43 and 44, or
• SEQ ID NOs: 48, 49 and 50.

7. A monoclonal antibody of anyone of the preceding claims, comprising a light chain variable region consisting, or consisting essentially, of a sequence selected from SEQ ID NOs: 3, 5, 7, 9, 11 and 13 and/or a heavy chain variable region consisting, or consisting essentially, of a sequence selected from SEQ ID NOs: 4, 6, 8, 10, 12, and 14.

8. The monoclonal antibody of claim 7, comprising a light chain variable region and a heavy chain variable region as follows:
• SEQ ID NOs: 3 and 4; or
• SEQ ID NOs: 5 and 6; or
• SEQ ID NOs: 7 and 8; or
• SEQ ID NOs: 9 and 10; or
• SEQ ID NOs: 11 and 12; or
• SEQ ID NOs: 13 and 14.

9. The monoclonal antibody of claim 1, which essentially does not bind HIV gp120 protein.

10. The monoclonal antibody of claim 8, which is selected from 4G1, 8D5, 3A2, 15B4, 5E12, and 9B12, preferably from 4G1, 8D5, 3A2, and 15B4.

11. The monoclonal antibody of anyone of claims 1 to 10, which is humanized.

12. The fragment of a monoclonal antibody of anyone of claims 1 to 11, which is a Fab, Fab'2, ScFv, nanobody, or CDR.

13. A nucleic acid encoding an antibody of anyone of claims 1-11, or a light or heavy chain thereof, or a variable domain thereof.

14. A composition comprising an antibody, variant or fragment of anyone of claims 1-12 or a nucleic acid of claim 13.

15. The composition of claim 14, which further comprises a pharmaceutically acceptable excipient.

16. The composition of claim 14 or 15, which further comprises (i) a further antiviral agent or (ii) an anti-PLA2sGIB antibody.

17. A kit comprising an antibody, variant or fragment of anyone of claims 1-12 in a container.

18. The kit of claim 17, further comprising one or more reagent(s) for performing an immune reaction, preferably a substrate or label and/or a marker antibody.

19. The kit of claim 17 or 18, which comprises reagents for an ELISA reaction.

20. A composition of anyone of claims 14 to 16, for use for treating HIV in a subject in need thereof.

21. A method for detecting a HIV gp41 protein in a sample, comprising:
(i) contacting the sample with a monoclonal antibody, variant or fragment of anyone of claims 1 to 12, and
(ii) detecting the presence of an antibody:antigen complex formed in step (i).

22. A method for monitoring progression of HIV disease in a subject in need thereof, comprising in vitro quantifying gp41 at different time intervals in a plasma sample from the subject using a monoclonal antibody, variant or fragment of anyone of claims 1 to 12.

23. The method of claim 22, for monitoring anti-HIV treatment efficacy.

24. The method of claim 22, for determining if an HIV-infected subject can benefit from anti-PLA2G1B immunotherapy, preferably anti-PLA2G1B antibody therapy or trans CD4 immunotherapy.

25. The method of claim 22, for determining the severity of HIV disease in a subject, wherein an increased level of binding of the monoclonal antibody, variant or fragment of anyone of claims 1 to 12 in said sample as compared to a reference value is indicative of severity.

26. The method of claim 25, wherein the reference value is a value determined at an earlier stage in a sample from the same subject, or a mean value.


SEQUENCE LISTING

<110>  DIACCURATE

<120>  ANTIBODIES AND THE USES THEREOF

<130>  B3078

<160>  65

<170>  PatentIn version 3.5

<210>  1
<211>  344
<212>  PRT
<213>  Artificial Sequence

<220>
<223>  HIV gp41

<400>  1

Ala Ala Ile Gly Ala Leu Phe Leu Gly Phe Leu Gly Ala Ala Gly Ser
1               5                   10                  15

Thr Met Gly Ala Ala Ser Val Thr Leu Thr Val Gln Ala Arg Leu Leu
            20                  25                  30

Leu Ser Gly Ile Val Gln Gln Gln Asn Asn Leu Leu Arg Ala Ile Glu
        35                  40                  45

Ser Gln Gln His Met Leu Arg Leu Thr Val Trp Gly Ile Lys Gln Leu
    50                  55                  60

Gln Ala Arg Val Leu Ala Val Glu Arg Tyr Leu Lys Asp Gln Gln Leu
65                  70                  75                  80

Leu Gly Phe Trp Gly Cys Ser Gly Lys Leu Ile Cys Thr Thr Thr Val
                85                  90                  95

Pro Trp Asn Ala Ser Trp Ser Asn Lys Ser Leu Asp Asp Ile Trp Asn
            100                 105                 110

Asn Met Thr Trp Met Gln Trp Glu Arg Glu Ile Asp Asn Tyr Thr Ser
        115                 120                 125

Leu Ile Tyr Ser Leu Leu Glu Lys Ser Gln Thr Gln Gln Glu Lys Asn
    130                 135                 140

Glu Gln Glu Leu Leu Glu Leu Asp Lys Trp Ala Ser Leu Trp Asn Trp
145                 150                 155                 160

Phe Asp Ile Thr Asn Trp Leu Trp Tyr Ile Lys Ile Phe Ile Met Ile

165                 170                 175

Val Gly Gly Leu Val Gly Leu Arg Ile Val Phe Ala Val Leu Ser Ile
            180                 185                 190

Val Asn Arg Val Arg Gln Gly Tyr Ser Pro Leu Ser Leu Gln Thr Arg
        195                 200                 205

Pro Pro Val Pro Arg Gly Pro Asp Arg Pro Glu Gly Ile Glu Glu Glu
    210                 215                 220

Gly Gly Glu Arg Asp Arg Asp Thr Ser Gly Arg Leu Val His Gly Phe
225                 230                 235                 240

Leu Ala Ile Ile Trp Val Asp Leu Arg Ser Leu Phe Leu Leu Ser Tyr
                245                 250                 255

His His Leu Arg Asp Leu Leu Leu Ile Ala Ala Arg Ile Val Glu Leu
            260                 265                 270

Leu Gly Arg Arg Gly Trp Glu Val Leu Lys Tyr Trp Trp Asn Leu Leu
        275                 280                 285

Gln Tyr Trp Ser Gln Glu Leu Lys Ser Ser Ala Val Ser Leu Leu Asn
    290                 295                 300

Ala Ala Ala Ile Ala Val Ala Glu Gly Thr Asp Arg Val Ile Glu Val
305                 310                 315                 320

Leu Gln Arg Ala Gly Arg Ala Ile Leu His Ile Pro Thr Arg Ile Arg
                325                 330                 335

Gln Gly Leu Glu Arg Ala Leu Leu
            340

<210>  2
<211>  15
<212>  PRT
<213>  Artificial Sequence

<220>
<223>  PEP3

<400>  2

Pro Trp Asn Ala Ser Trp Ser Asn Lys Ser Leu Asp Asp Ile Trp
1               5                   10                  15

<210>  3
<211>  112

<212>  PRT
<213>  Artificial Sequence

<220>
<223>  4G1 VL

<400>  3

Asp Val Val Met Thr Gln Thr Pro Leu Ser Leu Cys Val Ser Leu Gly
1               5                   10                  15

Asp Gln Ala Ser Ile Ser Cys Arg Thr Ser Gln Ser Ile Val His Ser
            20                  25                  30

Asn Arg Tyr Thr Tyr Leu Glu Trp Tyr Leu Gln Lys Pro Gly Gln Pro
        35                  40                  45

Leu Lys Leu Leu Ile Tyr Gly Ile Ala Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Met Gly Val Tyr Tyr Cys Leu Gln Gly
                85                  90                  95

Thr His Val Pro Phe Thr Phe Gly Ser Gly Thr Lys Leu Glu Ile Lys
            100                 105                 110

<210>  4
<211>  117
<212>  PRT
<213>  Artificial Sequence

<220>
<223>  4G1 VH

<400>  4

Gln Val Gln Leu Gln Gln Pro Gly Ala Glu Leu Val Lys Pro Gly Ala
1               5                   10                  15

Ser Val Lys Leu Ser Cys Lys Ala Ser Gly Tyr Ile Phe Arg Asn Tyr
            20                  25                  30

Trp Met His Trp Val Lys Gln Arg Pro Gly Gln Gly Leu Glu Trp Ile
        35                  40                  45

Gly Met Ile His Pro Asn Ser Asp Ile Thr His Tyr Asn Glu Lys Phe
    50                  55                  60

Lys Asn Arg Ala Thr Leu Thr Val Asp Lys Ser Ser Ser Thr Ala Tyr

65                  70                  75                  80

Met Gln Leu Asn Ser Leu Thr Ser Glu Asp Ser Ala Val Tyr Tyr Cys
                85                  90                  95

Ala Arg Gly Gly Leu Gly Val Phe Asp Tyr Trp Gly Gln Gly Thr Ala
            100                 105                 110

Leu Arg Val Ser Ser
        115

<210>  5
<211>  112
<212>  PRT
<213>  Artificial Sequence

<220>
<223>  3A2 VL

<400>  5

Asp Val Val Met Thr Gln Thr Pro Leu Ser Leu Pro Val Ser Leu Gly
1               5                   10                  15

Asp Gln Val Ser Met Ser Cys Arg Ser Ser Gln Ser Ile Glu His Ser
            20                  25                  30

Asn Arg Asn Thr Tyr Leu Glu Trp Tyr Leu His Lys Pro Gly Gln Ala
        35                  40                  45

Pro Gln Leu Leu Ile Tyr Gly Val Ser Asn Arg Phe Thr Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Met Gly Val Tyr His Cys Leu Gln Gly
                85                  90                  95

Thr His Val Pro Phe Thr Phe Gly Ser Gly Thr Lys Leu Glu Ile Lys
            100                 105                 110

<210>  6
<211>  117
<212>  PRT
<213>  Artificial Sequence

<220>
<223>  3A2 VH

<400>  6

Gln Val Gln Leu Gln Gln Pro Gly Ala Glu Leu Val Lys Pro Gly Ala
1               5                   10                  15

Ser Val Lys Leu Ser Cys Lys Ala Ser Gly Tyr Ile Phe Thr Asn Tyr
            20                  25                  30

Trp Met His Trp Val Arg Gln Arg Pro Gly Gln Gly Pro Glu Trp Ile
        35                  40                  45

Gly Met Ile His Pro Asn Arg Gly Asp Leu His Tyr Ser Glu Lys Phe
    50                  55                  60

Asn Asn Lys Ala Thr Leu Thr Val Asp Lys Ser Ser Ser Thr Ala Tyr
65                  70                  75                  80

Met Gln Leu Asn Ser Leu Ala Ser Glu Asp Ser Ala Val Tyr Phe Cys
                85                  90                  95

Ala Arg Ala Gly Leu Gly Val Phe Asp Tyr Trp Gly Gln Gly Ser Thr
            100                 105                 110

Leu Thr Val Ser Ser
        115

<210>  7
<211>  112
<212>  PRT
<213>  Artificial Sequence

<220>
<223>  8D5 VL

<400>  7

Asp Val Val Met Thr Gln Thr Pro Leu Ser Leu Pro Val Ser Leu Gly
1               5                   10                  15

Asp Gln Ala Ser Ile Ser Cys Arg Ser Ser Gln Ser Ile Val His Ser
            20                  25                  30

Asn Arg Tyr Thr Tyr Leu Glu Trp Tyr Leu Gln Arg Pro Gly Gln Ser
        35                  40                  45

Leu Lys Leu Leu Ile Tyr Gly Val Ser Asn Arg Phe Ser Gly Val Pro
    50                  55                  60

Asp Arg Phe Ser Gly Ser Gly Ser Gly Thr Asp Phe Ile Leu Lys Ile
65                  70                  75                  80

Ser Arg Val Glu Ala Glu Asp Met Gly Val Tyr Tyr Cys Phe Gln Gly

85                  90                  95

Thr His Val Pro Phe Thr Phe Gly Ser Gly Thr Lys Leu Glu Ile Lys
            100                 105                 110

<210>  8
<211>  117
<212>  PRT
<213>  Artificial Sequence

<220>
<223>  8D5 VH

<400>  8

Gln Val Gln Leu Gln Gln Pro Gly Ala Glu Leu Ile Glu Pro Gly Thr
1               5                   10                  15

Ser Val Lys Leu Ser Cys Lys Ala Ser Gly Tyr Thr Phe Thr Thr Tyr
            20                  25                  30

Trp Met His Trp Val Lys Gln Arg Pro Gly Gln Gly Leu Glu Trp Ile
        35                  40                  45

Gly Met Ile His Pro Asn Ser Gly Asp Thr Phe Tyr Asn Glu Asn Phe
    50                  55                  60

Lys Asn Lys Ala Thr Leu Thr Val Asp Ile Ser Ser Ser Thr Val Tyr
65                  70                  75                  80

Met Glu Leu Ser Ser Leu Thr Ser Glu Asp Ser Ala Val Tyr Ser Cys
                85                  90                  95

Val Lys Ala Gly Thr Gly Ala Leu Asp Tyr Trp Gly Gln Gly Thr Thr
            100                 105                 110

Leu Thr Val Ser Ser
        115

<210>  9
<211>  112
<212>  PRT
<213>  Artificial Sequence

<220>
<223>  15B4 VL

<400>  9

Asp Ile Gln Met Thr Gln Thr Pro Leu Thr Leu Ser Val Thr Ile Gly
1               5                   10                  15

Gln Pro Ala Ser Ile Ser Cys Lys Ser Gly Gln Ser Leu Leu Tyr Ser
            20                  25                  30

Asn Gly Lys Thr Tyr Leu Asn Trp Leu Leu Gln Arg Pro Gly Gln Ser
        35                  40                  45

Pro Lys Arg Leu Ile Tyr Leu Val Ser Lys Leu Asp Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Leu Gly Val Tyr Tyr Cys Leu Gln Ser
                85                  90                  95

Thr His Phe Pro Arg Thr Phe Gly Gly Gly Thr Lys Leu Glu Ile Lys
            100                 105                 110

<210>  10
<211>  116
<212>  PRT
<213>  Artificial Sequence

<220>
<223>  15B4 VH

<400>  10

Gln Val Gln Leu Gln Gln Ser Gly Pro Glu Leu Val Arg Pro Gly Thr
1               5                   10                  15

Ser Val Lys Ile Ser Cys Lys Ala Pro Gly Tyr Ser Phe Thr Ser His
            20                  25                  30

Trp Met Gln Trp Leu Arg Gln Arg Pro Gly Gln Gly Leu Glu Trp Ile
        35                  40                  45

Gly Glu Ile Phe Pro Gly Ser Gly Lys Thr Tyr Tyr Asn Glu Lys Phe
    50                  55                  60

Lys Asp Lys Ala Thr Leu Thr Ala Asp Thr Ser Ser Thr Thr Val Tyr
65                  70                  75                  80

Met Gln Phe Ser Ser Leu Thr Ser Glu Asp Ser Ala Val Tyr Phe Cys
                85                  90                  95

Ala Ser Asp Tyr Asp Gly Gly Asp Tyr Trp Gly Gln Gly Thr Thr Leu
            100                 105                 110

Thr Val Ser Ser

        115

<210>  11
<211>  112
<212>  PRT
<213>  Artificial Sequence

<220>
<223>  5E12 VL

<400>  11

Asp Ile Gln Met Thr Gln Thr Pro Leu Thr Leu Ser Val Thr Ile Gly
1               5                   10                  15

His Pro Ala Ser Ile Ser Cys Lys Ser Ser Gln Ser Leu Leu His Thr
            20                  25                  30

Asn Gly Lys Ile Tyr Leu Asn Trp Leu Leu Gln Arg Pro Gly Gln Ser
        35                  40                  45

Pro Lys Arg Leu Ile Tyr Leu Val Ser Lys Leu Asp Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Leu Gly Val Tyr Tyr Cys Leu Gln Ser
                85                  90                  95

Thr His Phe Pro Gln Thr Phe Gly Gly Gly Thr Lys Leu Glu Ile Lys
            100                 105                 110

<210>  12
<211>  124
<212>  PRT
<213>  Artificial Sequence

<220>
<223>  5E12 VH

<400>  12

Gln Ile Gln Leu Val Gln Ser Gly Pro Glu Leu Lys Lys Pro Gly Glu
1               5                   10                  15

Thr Val Lys Ile Ser Cys Lys Ala Ser Gly Tyr Thr Phe Arg Thr Tyr
            20                  25                  30

Gly Met Ser Trp Val Lys Gln Ala Pro Gly Lys Gly Leu Lys Trp Met
        35                  40                  45

Gly Trp Ile Asn Thr Tyr Ser Gly Val Pro Arg Tyr Val Asp Asp Phe
    50                  55                  60

Lys Gly Arg Phe Ala Leu Ser Leu Glu Thr Ser Ala Ser Thr Ala Tyr
65                  70                  75                  80

Leu Gln Ile Asn Asn Leu Lys Ser Glu Asp Thr Ala Thr Tyr Phe Cys
                85                  90                  95

Val Arg Glu Gly Tyr Ser Tyr Tyr Gly Ser Ser Phe His Thr Met Asp
            100                 105                 110

Tyr Trp Gly Gln Gly Thr Ser Val Thr Val Ser Ser
        115                 120

<210>  13
<211>  112
<212>  PRT
<213>  Artificial Sequence

<220>
<223>  9B12 VL

<400>  13

Asp Val Val Met Thr Gln Thr Pro Leu Thr Leu Pro Val Thr Ile Gly
1               5                   10                  15

Gln Pro Ala Ser Ile Ser Cys Lys Ser Ser Gln Ser Leu Leu Asn Ser
            20                  25                  30

Asp Gly Lys Thr Lys Leu Asn Trp Leu Leu Gln Arg Pro Gly Glu Ser
        35                  40                  45

Leu Lys Leu Leu Ile Tyr Leu Val Ser Lys Leu Asp Ser Gly Val Pro
    50                  55                  60

Asp Arg Phe Thr Gly Ser Gly Ser Gly Thr Asp Phe Thr Leu Lys Ile
65                  70                  75                  80

Ser Arg Val Glu Ala Glu Asp Leu Gly Val Tyr Tyr Cys Leu Gln Thr
                85                  90                  95

Thr His Phe Pro Arg Thr Phe Gly Gly Gly Thr Lys Leu Glu Ile Lys
            100                 105                 110

<210>  14
<211>  112
<212>  PRT
<213>  Artificial Sequence

<220>
<223>  9B12 VH

<400>  14

Gln Val Gln Leu Gln Gln Ser Gly Pro Glu Leu Val Lys Pro Gly Ala
1               5                   10                  15

Ser Val Lys Ile Ser Cys Lys Ala Ser Gly Tyr Gly Phe Ser Asp Ser
            20                  25                  30

Trp Met Asn Trp Val Lys Gln Arg Pro Gly Lys Gly Leu Asp Trp Ile
        35                  40                  45

Gly Arg Ile Tyr Pro Gly Asp Gly Asp Thr Asp Tyr Asn Gly Lys Phe
    50                  55                  60

Lys Asp Lys Ala Thr Leu Thr Ala Asp Lys Ser Ser Thr Thr Ala Tyr
65                  70                  75                  80

Met Gln Leu Ser Ser Leu Thr Ser Glu Asp Ser Ala Val Tyr Phe Cys
                85                  90                  95

Ala Tyr Leu Gly His Trp Gly Gln Gly Thr Leu Val Thr Val Ser Ala
            100                 105                 110


<210>  15
<211>  11
<212>  PRT
<213>  Artificial Sequence

<220>
<223>  CDR

<400>  15

Gln Ser Ile Val His Ser Asn Arg Tyr Thr Tyr
1               5                   10


<210>  16
<211>  4
<212>  PRT
<213>  Artificial Sequence

<220>
<223>  CDR

<400>  16

Gly Ile Ala Asn
1


<210>  17
<211>  9

<212>  PRT
<213>  Artificial Sequence

<220>
<223>  CDR

<400>  17

Leu Gln Gly Thr His Val Pro Phe Thr
1               5


<210>  18
<211>  8
<212>  PRT
<213>  Artificial Sequence

<220>
<223>  CDR

<400>  18

Gly Tyr Ile Phe Arg Asn Tyr Trp
1               5


<210>  19
<211>  8
<212>  PRT
<213>  Artificial Sequence

<220>
<223>  CDR

<400>  19

Ile His Pro Asn Ser Asp Ile Thr
1               5


<210>  20
<211>  10
<212>  PRT
<213>  Artificial Sequence

<220>
<223>  CDR

<400>  20

Ala Arg Gly Gly Leu Gly Val Phe Asp Tyr
1               5                   10


<210>  21
<211>  11
<212>  PRT
<213>  Artificial Sequence

<220>
<223>  CDR

<400>  21

Gln Ser Ile Glu His Ser Asn Arg Asn Thr Tyr
1               5                   10

<210>  22
<211>  3
<212>  PRT
<213>  Artificial Sequence

<220>
<223>  CDR

<400>  22

Gly Val Ser
1

<210>  23
<211>  9
<212>  PRT
<213>  Artificial Sequence

<220>
<223>  CDR

<400>  23

Leu Gln Gly Thr His Val Pro Phe Thr
1               5

<210>  24
<211>  8
<212>  PRT
<213>  Artificial Sequence

<220>
<223>  CDR

<400>  24

Gly Tyr Ile Phe Thr Asn Tyr Trp
1               5

<210>  25
<211>  8
<212>  PRT
<213>  Artificial Sequence

<220>
<223>  CDR

<400>  25

Ile His Pro Asn Arg Gly Asp Leu
1               5

<210>  26
<211>  10

<212>  PRT
<213>  Artificial Sequence

<220>
<223>  CDR

<400>  26

Ala Arg Ala Gly Leu Gly Val Phe Asp Tyr
1               5                   10


<210>  27
<211>  11
<212>  PRT
<213>  Artificial Sequence

<220>
<223>  CDR

<400>  27

Gln Ser Ile Val His Ser Asn Arg Tyr Thr Tyr
1               5                   10


<210>  28
<211>  3
<212>  PRT
<213>  Artificial Sequence

<220>
<223>  CDR

<400>  28

Gly Val Ser
1


<210>  29
<211>  9
<212>  PRT
<213>  Artificial Sequence

<220>
<223>  CDR

<400>  29

Phe Gln Gly Thr His Val Pro Phe Thr
1               5


<210>  30
<211>  8
<212>  PRT
<213>  Artificial Sequence

<220>
<223>  CDR

<400>  30

Gly Tyr Thr Phe Thr Thr Tyr Trp
1               5

<210>  31
<211>  8
<212>  PRT
<213>  Artificial Sequence

<220>
<223>  CDR

<400>  31

Ile His Pro Asn Ser Gly Asp Thr
1               5

<210>  32
<211>  10
<212>  PRT
<213>  Artificial Sequence

<220>
<223>  CDR

<400>  32

Val Lys Ala Gly Thr Gly Ala Leu Asp Tyr
1               5                   10

<210>  33
<211>  11
<212>  PRT
<213>  Artificial Sequence

<220>
<223>  CDR

<400>  33

Gln Ser Leu Leu Tyr Ser Asn Gly Lys Thr Tyr
1               5                   10

<210>  34
<211>  3
<212>  PRT
<213>  Artificial Sequence

<220>
<223>  CDR

<400>  34

Leu Val Ser
1

<210>  35
<211>  9

<212>  PRT
<213>  Artificial Sequence

<220>
<223>  CDR

<400>  35

Leu Gln Ser Thr His Phe Pro Arg Thr
1               5


<210>  36
<211>  8
<212>  PRT
<213>  Artificial Sequence

<220>
<223>  CDR

<400>  36

Gly Tyr Ser Phe Thr Ser His Trp
1               5


<210>  37
<211>  8
<212>  PRT
<213>  Artificial Sequence

<220>
<223>  CDR

<400>  37

Ile Phe Pro Gly Ser Gly Lys Thr
1               5


<210>  38
<211>  9
<212>  PRT
<213>  Artificial Sequence

<220>
<223>  CDR

<400>  38

Ala Ser Asp Tyr Asp Gly Gly Asp Tyr
1               5


<210>  39
<211>  11
<212>  PRT
<213>  Artificial Sequence

<220>
<223>  CDR

<400>  39

Gln Ser Leu Leu His Thr Asn Gly Lys Ile Tyr
1               5                   10

<210>  40
<211>  3
<212>  PRT
<213>  Artificial Sequence

<220>
<223>  CDR

<400>  40

Leu Val Ser
1

<210>  41
<211>  9
<212>  PRT
<213>  Artificial Sequence

<220>
<223>  CDR

<400>  41

Leu Gln Ser Thr His Phe Pro Gln Thr
1               5

<210>  42
<211>  8
<212>  PRT
<213>  Artificial Sequence

<220>
<223>  CDR

<400>  42

Gly Tyr Thr Phe Arg Thr Tyr Gly
1               5

<210>  43
<211>  8
<212>  PRT
<213>  Artificial Sequence

<220>
<223>  CDR

<400>  43

Ile Asn Thr Tyr Ser Gly Val Pro
1               5

<210>  44
<211>  17

<212>  PRT
<213>  Artificial Sequence

<220>
<223>  CDR

<400>  44

Val Arg Glu Gly Tyr Ser Tyr Tyr Gly Ser Ser Phe His Thr Met Asp
1               5                   10                  15

Tyr

<210>  45
<211>  11
<212>  PRT
<213>  Artificial Sequence

<220>
<223>  CDR

<400>  45

Gln Ser Leu Leu Asn Ser Asp Gly Lys Thr Lys
1               5                   10

<210>  46
<211>  3
<212>  PRT
<213>  Artificial Sequence

<220>
<223>  CDR

<400>  46

Leu Val Ser
1

<210>  47
<211>  9
<212>  PRT
<213>  Artificial Sequence

<220>
<223>  CDR

<400>  47

Leu Gln Thr Thr His Phe Pro Arg Thr
1               5

<210>  48
<211>  8
<212>  PRT
<213>  Artificial Sequence

<220>
<223>  CDR

<400>  48

Gly Tyr Gly Phe Ser Asp Ser Trp
1               5


<210>  49
<211>  8
<212>  PRT
<213>  Artificial Sequence

<220>
<223>  CDR

<400>  49

Ile Tyr Pro Gly Asp Gly Asp Thr
1               5


<210>  50
<211>  5
<212>  PRT
<213>  Artificial Sequence

<220>
<223>  CDR

<400>  50

Ala Tyr Leu Gly His
1               5


<210>  51
<211>  336
<212>  DNA
<213>  Artificial Sequence

<220>
<223>  4G1 VL

<400>  51
gatgttgtga tgacccaaac tccactctcc ctgtgtgtca gtcttggaga tcaagcctcc      60

atctcttgca gaaccagtca gagcattgta cacagtaata gatacaccta tttagaatgg     120

tacctgcaga aaccaggcca gccgctaaaa ctcctgatat atgggattgc caaccgattt     180

tctggggtcc cagacaggtt cagtggcagt ggatcaggga cagatttcac actcaagatc     240

agcagagtgg aggctgagga tatgggagtt tattattgtc ttcaaggtac acatgttcca     300

ttcacgttcg gctcggggac aaagttggaa ataaaa                               336


<210>  52
<211>  351
<212>  DNA
<213>  Artificial Sequence

<220>
<223>  4G1 VH

<400>  52
caggtccaac tgcagcaacc tggggctgag ctggtaaagc ctggggcttc agtaaagttg      60

tcctgcaagg cttctggcta cattttcaga aactactgga tgcactgggt gaagcagagg     120

cctggacaag gccttgaatg gattggaatg attcatccta atagtgatat tactcattac     180

aatgagaagt tcaaaaacag ggccacactg actgttgaca aatcctccag cacagcctac     240

atgcaactca acagcctgac atctgaagac tctgcggtct attactgtgc aagggggggg     300

ctgggggtgt ttgactactg gggccaaggc accgctctca gagtctcctc a              351


<210>  53
<211>  336
<212>  DNA
<213>  Artificial Sequence

<220>
<223>  3A2 VL

<400>  53
gatgttgtga tgacccaaac tccactctcc ctgcctgtca gtcttggaga tcaagtctcc      60

atgtcttgca gatctagcca gagcattgaa cacagtaata gaaacaccta tttagaatgg     120

tacctgcaca aaccaggcca ggcgccacag ctcctgatat atggggtttc caaccgattt     180

actggggtcc cagacaggtt cagtggcagt ggatcaggga cagatttcac actcaagatc     240

agtagagtgg aggctgagga tatgggagtt tatcattgct tgcaaggtac acatgttcca     300

ttcacgttcg gctcggggac aaagttggaa ataaaa                               336


<210>  54
<211>  363
<212>  DNA
<213>  Artificial Sequence

<220>
<223>  3A2 VH

<400>  54
caggtccaac tgcaacagcc tggggctgag ctggtaaagc ctggggcttc agtgaaattg      60

tcctgcaagg cttctggcta cattttcacc aattactgga tgcactgggt gaggcagagg     120

cctggacaag gccctgagtg gattggaatg attcacccta ataggggtga tcttcactac     180

agcgagaagt tcaacaacaa ggccacactg actgtagaca aatcctccag cacagcctac     240

atgcaactca acagcctggc atctgaggac tctgcggtct atttctgtgc aagggcggga     300

ctgggggtgt ttgactactg gggccagggc tccactctca cagtctcctc agccaaaaca     360

aca                                                                   363

<210>  55
<211>  336
<212>  DNA
<213>  Artificial Sequence

<220>
<223>  8D5 VL

<400>  55
gatgttgtga tgacccaaac tccgctctcc ctgcctgtca gtcttggaga tcaagcctcc      60

atctcctgca gatctagtca gagcattgta cacagtaata gatacaccta tttagaatgg     120

tatctgcaga gaccaggcca gtcgctaaag ctcctgatat acggggtttc caaccgattt     180

tctggggtcc cagacaggtt cagtggcagt ggatcaggga cagatttcat actcaagatc     240

agcagagtgg aggctgagga tatgggagtt tattactgct ttcaaggtac acatgttcca     300

ttcacgttcg gctcggggac aaagttggaa ataaaa                               336


<210>  56
<211>  351
<212>  DNA
<213>  Artificial Sequence

<220>
<223>  8D5 VH

<400>  56
caggtccaac tgcagcagcc tggggctgag ctgatagagc ctgggacttc agtgaagttg      60

tcctgcaagg cttctggcta cactttcacc acttactgga tgcactgggt gaagcagagg     120

cctggacaag gcctggagtg gattggaatg attcatccta atagtggtga tactttttac     180

aatgagaact tcaagaacaa ggccacactg actgtagaca tatcctccag cacagtctac     240

atggaactca gcagcctgac atctgaggac tctgcggtct attcctgtgt aaaggctggg     300

acgggcgcct tagactactg gggccaaggc accactctca cagtctcctc g              351


<210>  57
<211>  336
<212>  DNA
<213>  Artificial Sequence

<220>
<223>  15B4 VL

<400>  57
gatatccaga tgacacagac tccactcact ttgtcggtta ccattggcca accagcttcc      60

atctcttgca agtcaggtca gagcctctta tatagtaatg gaaaaaccta tttgaattgg     120

ttattacaga ggccaggcca gtctccaaaa cgcctaatct atctggtgtc taaattggac     180

tctggagtcc ctgacaggtt cagtggcagt ggatcaggga cagatttcac actaaaaatc     240

agcagagtgg aggctgagga tttgggagtt tattactgct tgcagagtac acattttcct     300

cggacgttcg gtggaggcac caagctggaa atcaaa 336

<210> 58
<211> 348
<212> DNA
<213> Artificial Sequence

<220>
<223> 15B4 VH

<400> 58
caggttcagc tgcagcagtc tggacctgag ctggtgaggc ctgggacttc agtgaagata 60

tcctgcaagg ctcctggcta tagtttcacc agccactgga tgcagtggct aagacagagg 120

cctggacagg gccttgagtg gattggagag atttttcctg gaagtggtaa aacttattat 180

aatgagaagt tcaaggacaa ggccacactg actgcagaca catcctccac cacagtctat 240

atgcagttca gcagcctgac atctgaggac tctgcggtct atttctgtgc gagtgattac 300

gacggaggag actactgggg ccaaggcacc actctcacag tctcctca 348

<210> 59
<211> 336
<212> DNA
<213> Artificial Sequence

<220>
<223> 5E12 VL

<400> 59
gatatccaga tgacacagac tccactcact ttgtcggtta ccattggaca cccagcttcc 60

atctcttgca agtcaagtca gagcctctta catactaatg gaaaaatcta tttgaattgg 120

ttattacaga ggccaggcca gtctccaaaa cgcctaatct atctggtgtc taaattggac 180

tctggagtcc ctgacaggtt cagtggcagt ggatcaggga cagatttcac actgaaaatc 240

agcagagtgg aggctgagga tttgggagtt tattactgct tgcagagtac acattttcct 300

cagacgttcg gtggaggcac caagctggaa atcaaa 336

<210> 60
<211> 372
<212> DNA
<213> Artificial Sequence

<220>
<223> 5E12 VH

<400> 60
cagatccagt tggtacagtc tggacctgag ctgaagaagc ctggagagac agtcaagatc 60

tcctgcaagg cttctggcta taccttcaga acctatggaa tgagctgggt gaaacaggct 120

ccaggaaagg gtttaaagtg gatgggctgg ataaacacct actctggagt gccaagatat 180

gttgatgact ttaagggacg gtttgccctc tctttggaaa cctctgccag cactgcctat 240

ttgcagatca ataacctcaa aagtgaggac acggctacat atttctgtgt aagagagggt 300

tactcgtatt acggtagtag ttttcatact atggactact ggggtcaagg aacctcagtc 360

accgtctcct ca 372

<210> 61
<211> 336
<212> DNA
<213> Artificial Sequence

<220>
<223> 9B12 VL

<400> 61
gatgttgtga tgacccagac tccactcact ttgccggtca ccattggaca accagcttcc 60

atctcttgca agtcaagtca gagcctctta aatagtgatg gaaaaaccaa gttaaattgg 120

ttattacaga ggccaggcga gtctctaaag ctcctaatct atctggtgtc taaactggac 180

tctggagtcc ctgacaggtt cactggcagt ggatcaggga cagatttcac actgaaaatc 240

agcagagtgg aggctgagga tttgggagtt tattactgct tgcaaactac acattttcct 300

cggacgttcg gtggaggcac caagctggaa atcaaa 336

<210> 62
<211> 336
<212> DNA
<213> Artificial Sequence

<220>
<223> 9B12 VH

<400> 62
caggttcagc tgcagcagtc tggacctgaa ctggtgaagc ctggggcctc agtgaagatt 60

tcctgcaagg cttctggcta cggattcagt gactcctgga tgaactgggt gaagcagagg 120

cctggaaagg gtcttgactg gattggacgg atttatcctg gagatggaga tactgactac 180

aatgggaagt tcaaggacaa ggccacactg actgcagaca aatcctccac tacagcctac 240

atgcaactca gcagcctgac atctgaggac tctgcggtct acttctgtgc gtatcttggc 300

cactggggcc aagggactct ggtcactgtc tctgca 336

<210> 63
<211> 19
<212> PRT
<213> Artificial Sequence

<220>
<223> 5184-3 PEPTIDE

<400> 63

Glu Lys Ser Gln Thr Gln Gln Glu Lys Asn Glu Gln Glu Leu Leu Glu
1               5                   10                  15

Leu Asp Lys

<210>  64
<211>  19
<212>  PRT
<213>  Artificial Sequence

<220>
<223>  HXB2 domain

<400>  64

Glu Glu Ser Gln Asn Gln Gln Glu Lys Asn Glu Gln Glu Leu Leu Glu
1               5                   10                  15

Leu Asp Lys

<210>  65
<211>  282
<212>  PRT
<213>  Artificial Sequence

<220>
<223>  gC1qR

<400>  65

Met Leu Pro Leu Leu Arg Cys Val Pro Arg Val Leu Gly Ser Ser Val
1               5                   10                  15

Ala Gly Leu Arg Ala Ala Ala Pro Ala Ser Pro Phe Arg Gln Leu Leu
            20                  25                  30

Gln Pro Ala Pro Arg Leu Cys Thr Arg Pro Phe Gly Leu Leu Ser Val
        35                  40                  45

Arg Ala Gly Ser Glu Arg Arg Pro Gly Leu Leu Arg Pro Arg Gly Pro
    50                  55                  60

Cys Ala Cys Gly Cys Gly Cys Gly Ser Leu His Thr Asp Gly Asp Lys
65                  70                  75                  80

Ala Phe Val Asp Phe Leu Ser Asp Glu Ile Lys Glu Glu Arg Lys Ile
                85                  90                  95

Gln Lys His Lys Thr Leu Pro Lys Met Ser Gly Gly Trp Glu Leu Glu
            100                 105                 110

Leu Asn Gly Thr Glu Ala Lys Leu Val Arg Lys Val Ala Gly Glu Lys
        115                 120                 125

Ile Thr Val Thr Phe Asn Ile Asn Asn Ser Ile Pro Pro Thr Phe Asp
    130                 135                 140

Gly Glu Glu Glu Pro Ser Gln Gly Gln Lys Val Glu Glu Gln Glu Pro
145                 150                 155                 160

Glu Leu Thr Ser Thr Pro Asn Phe Val Val Glu Val Ile Lys Asn Asp
                165                 170                 175

Asp Gly Lys Lys Ala Leu Val Leu Asp Cys His Tyr Pro Glu Asp Glu
            180                 185                 190

Val Gly Gln Glu Asp Glu Ala Glu Ser Asp Ile Phe Ser Ile Arg Glu
        195                 200                 205

Val Ser Phe Gln Ser Thr Gly Glu Ser Glu Trp Lys Asp Thr Asn Tyr
    210                 215                 220

Thr Leu Asn Thr Asp Ser Leu Asp Trp Ala Leu Tyr Asp His Leu Met
225                 230                 235                 240

Asp Phe Leu Ala Asp Arg Gly Val Asp Asn Thr Phe Ala Asp Glu Leu
                245                 250                 255

Val Glu Leu Ser Thr Ala Leu Glu His Gln Glu Tyr Ile Thr Phe Leu
            260                 265                 270

Glu Asp Leu Lys Ser Phe Val Lys Ser Gln
        275                 280